(19) Europäisches Patentamt
European Patent Office
Office européen des brevets

(11) Veröffentlichungsnummer: **0 173 321**
**A1**

(12) # EUROPÄISCHE PATENTANMELDUNG

(21) Anmeldenummer: **85110834.0**

(22) Anmeldetag: **19.08.85**

(51) Int. Cl.⁴: **C 07 D 239/42**, C 07 D 239/46, A 01 N 47/44

(30) Priorität: **30.08.84 DE 3431924**
**17.05.85 DE 3517842**

(43) Veröffentlichungstag der Anmeldung: **05.03.86**
**Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Köln 80 (DE)**
Erfinder: **Schmidt, Robert R. Dr., Im Waldwinkel 110, D-5060 Bergisch-Gladbach 2 (DE)**

(54) **Sulfonylguanidinopyrimidin-Derivate.**

(57) Die Erfindung betrifft neue Sulfonylguanidinopyrimidin-Derivate der allgemeinen Formel (I)

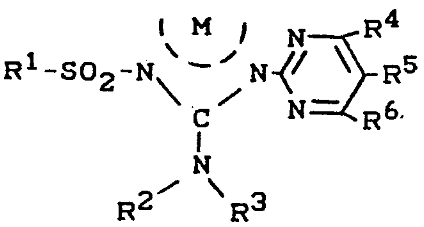

(I)

(worin die Reste M, $R^1$, $R^2$, $R^3$, $R^4$, $R^5$ und $R^6$ die in der Beschreibung angegebenen Bedeutungen haben), sowie 1:1-Addukte von Verbindungen der Formel (I) mit starken Säuren, Verfahren zu ihrer Herstellung und ihre Verwendung als Herbizide.

0 173 321

BAYER AKTIENGESELLSCHAFT 5090 Leverkusen, Bayerwerk

Konzernverwaltung RP
Patentabteilung Bi/mö
I b

Sulfonylguanidinopyrimidin-Derivate

Die Erfindung betrifft neue Sulfonylguanidinopyrimidin-Derivate, Verfahren zu ihrer Herstellung und ihre Verwendung als Herbizide.

Verschiedene Guanidin-Derivate sind aus Patentschriften (vergl. DE-AS 1 089 210, DD-PS 71 016 und 84 530) als potentielle Herbizide bekannt geworden, haben jedoch bisher als Mittel zur Unkrautbekämpfung und/oder Regulierung des Pflanzenwachstums keine größere Bedeutung erlangt.

Weitere Guanidin-Derivate sind Gegenstand älterer Schutzrechte (EP-OS 121 082).

Es wurden nun neue Sulfonylguanidinopyrimidin-Derivate der allgemeinen Formel (I)

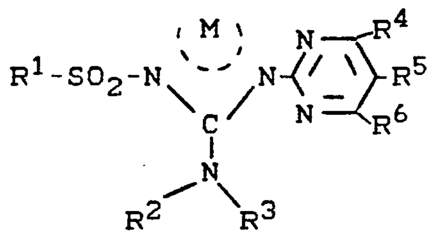

(I)

in welcher

M für Wasserstoff oder ein Metalläquivalent steht,

$R^1$ für einen gegebenenfalls substituierten Phenylrest

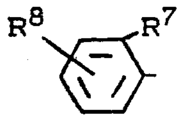

steht, worin

$R^7$ und $R^8$ gleich oder verschieden sind und für Wasserstoff, Halogen [insbesondere Fluor, Chlor und/oder Brom], Cyano, Nitro, $C_1$-$C_4$-Alkyl [welches gegebenenfalls durch Fluor, Chlor, Brom, Cyano, $C_1$-$C_4$-Alkoxycarbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkyl-sulfonyl substituiert ist], für $C_1$-$C_4$-Alkoxy [welches gegebenenfalls durch Fluor, Chlor, Brom, Cyano, $C_1$-$C_4$-Alkoxy-carbonyl, $C_1$-$C_4$-Alkoxy, $C_1$-$C_4$-Alkylthio, $C_1$-$C_4$-Alkylsulfinyl

oder $C_1$-$C_4$-Alkylsulfonyl substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], für $C_1$-$C_4$-Alkylamino-sulfonyl, Di-($C_1$-$C_4$-alkyl)-amino-sulfonyl, $C_1$-$C_4$-Alkoxyamino-sulfonyl, Benzyloxy-aminosulfonyl, $C_1$-$C_4$-Alkoxy-$C_1$-$C_4$-alkyl-aminosulfonyl, für Phenyl oder Phenoxy, für $C_1$-$C_4$-Alkoxysulfonyl, für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano oder $C_1$-$C_4$-Alkoxy-carbonyl substituiert ist], für $C_3$-$C_6$-Alkenoxy [welches gegebenenfalls durch Fluor, Chlor, Brom, Cyano oder $C_1$-$C_4$--Alkoxy-carbonyl substituiert ist], für $C_3$-$C_6$-Alkenylthio [welches gegebenenfalls durch Fluor, Chlor, Brom, Cyano oder $C_1$-$C_4$-Alkoxy-carbonyl substituiert ist], für $C_1$-$C_6$-Alkoxy-carbonyl [welches gegebenenfalls durch Fluor, Chlor, Brom, Cyano oder $C_1$-$C_4$--Alkoxy substituiert ist], für $C_3$-$C_6$--Cycloalkoxy-carbonyl, für $C_1$-$C_6$-Alkylthiocarbonyl [welches gegebenenfalls durch Fluor, Chlor, Brom, Cyano oder

$C_1$-$C_4$-Alkoxy-carbonyl substituiert ist], für Aminocarbonyl, $C_1$-$C_4$-Alkylamino-carbonyl, Di-($C_1$-$C_4$-alkyl)-amino-carbonyl, $C_1$-$C_4$-Alkoxy-amino-carbonyl, $C_3$-$C_4$-Alkenyloxy-amino-carbonyl, Benzyloxyaminocarbonyl, $C_1$-$C_4$--Alkoxy-$C_1$-$C_4$-alkyl-amino-carbonyl, Dimethylhydrazinocarbonyl, Dimethylhydrazinosulfonyl, für $C_3$-$C_6$-Alkinyloxy oder $C_3$-$C_6$-Alkinylthio stehen;

in welcher weiter

$R^1$ für eine gegebenenfalls substituierten Benzylrest

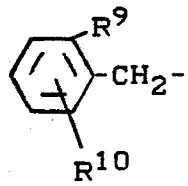

steht, worin

$R^9$ und $R^{10}$ gleich oder verschieden sind und für Wasserstoff, Halogen [wie insbesondere Fluor, Chlor oder Brom], Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkoxy-carbonyl, $C_1$-$C_4$-Alkylthio, $C_1$-$C_4$-Alkylsulfinyl,

$C_1$-$C_4$-Alkylsulfonyl oder Di-($C_1$-$C_4$-alkyl)-aminosulfonyl stehen;

in welcher weiter

$R^2$ für Wasserstoff, $C_1$-$C_4$-Alkyl oder einen Sulfonylrest $R^{11}$-$SO_2$- steht, worin

$R^{11}$ für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, Di-($C_1$-$C_4$-alkyl)-amino, $C_3$-$C_6$-Cycloalkyl-amino oder Di-($C_3$-$C_6$-cycloalkyl)-amino [welche gegebenenfalls durch Fluor, Chlor, Brom, Cyano, Nitro, Phenyl, Phenoxy,

$C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], für Benzyl [welches gegebenenfalls durch Fluor, Chlor, Brom, Cyano, Nitro, $C_1$-$C_4$-Alkyl, Trifluormethyl, $C_1$-$C_4$-Alkoxy-carbonyl, $C_1$-$C_4$-Alkoxy oder Di-($C_1$-$C_4$-alkyl)-amino-sulfonyl substituiert ist] oder für einen gegebenenfalls substituierten Phenylrest 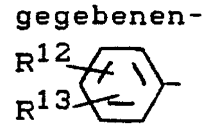

steht, worin

$R^{12}$ und $R^{13}$ gleich oder verschieden sind und die oben für $R^7$ und $R^8$ angegebenen Bedeutungen haben, jedoch nicht in jedem Einzelfall mit $R^7$ und $R^8$ identisch sind;

in welcher weiter

$R^3$ für den Rest $-O-R^{14}$ steht, worin

$R^{14}$ für $C_1$-$C_{12}$-Alkyl [welches gegebenenfalls durch Fluor, Chlor, Brom, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_6$-Cycloalkyl, $C_3$-$C_6$-Cycloalkyl-$C_1$-$C_2$-alkyl, $C_3$-$C_6$-Alkenyl, [welches gegebenenfalls durch Fluor, Chlor oder Brom substituiert ist], $C_3$-$C_6$-Alkinyl, $C_1$-$C_4$-Alkoxy-carbonyl-$C_1$-$C_2$-alkyl, Aminocarbonylmethyl, $C_1$-$C_4$-Alkylamino-carbonyl-methyl, Di-($C_1$-$C_4$-alkyl)-amino-carbonyl-methyl oder für Phenyl, Benzyl, Benzhydryl oder Phenylethyl [welche gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert sind] steht;

in welcher weiter

$R^3$ für den Rest 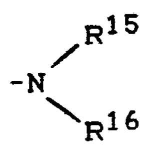 steht, worin

$R^{15}$ für Wasserstoff oder $C_1$-$C_4$-Alkyl steht und

$R^{16}$ für Wasserstoff, $C_1$-$C_4$-Alkyl [welches gegebenenfalls durch Fluor, Chlor, Brom, Cyano, $C_1$-$C_4$-Alkoxy oder $C_1$-$C_4$-Alkoxy-carbonyl substituiert ist], für $C_3$-$C_6$-Cycloalkyl, für

Phenyl, Benzyl oder Phenylethyl [welche gegebenenfalls durch Fluor, Chlor, Brom, Cyano, Nitro, $C_1$-$C_4$-Alkyl, $C_1$-$C_4$-Alkoxy oder $C_1$-$C_4$-Alkoxy-carbonyl substituiert sind], für $C_1$-$C_4$-Alkyl-carbonyl, $C_1$-$C_4$-Alkoxy-carbonyl, $C_1$-$C_4$-Alkyl-sulfonyl oder Phenylsulfonyl [welches gegebenenfalls durch Fluor, Chlor, Brom, Cyano, Nitro oder Methyl substituiert ist] steht;

in welcher weiter - für den Fall, daß $R^2$ für einen Sulfonylrest $R^{11}$-$SO_2$- steht, worin $R^{11}$ die oben angegebene Bedeutung hat - $R^3$ auch für Wasserstoff steht;

in welcher weiter

$R^4$ für Wasserstoff, Fluor, Chlor, Brom, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist] oder für $C_1$-$C_4$-Alkylthio [welches gegebenenfalls durch Fluor und/oder Chlor substituiert ist] steht;

$R^5$ für Wasserstoff, Fluor, Chlor, Brom, $C_1$-$C_4$-Alkyl [welches gegebenenfalls durch Fluor und/oder Chlor substituiert ist], für Cyano, Formyl, $C_1$-$C_4$-Alkyl-carbonyl oder $C_1$-$C_4$-Alkoxy-carbonyl steht und

$R^6$ für Wasserstoff, für $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für Amino, $C_1$-$C_4$-Alkylamino oder Di-($C_1$-$C_4$-alkyl)-amino steht

- mit der Maßgabe, daß $R^4$ und $R^6$ nicht gleichzeitig für Methyl stehen -

sowie 1:1-Addukte von Verbindungen der Formel (I) mit starken Säuren gefunden,

wobei folgende Verbindungen ausgenommen sind:

N'-(4,6-Dimethoxy-pyrimidin-2-yl)-N''-methoxy-N'''-(2-trifluormethoxy-benzolsulfonyl)-guanidin-Kaliumsalz,
N'-(4-Methoxy-6-methyl-pyrimidin-2-yl)-N''-dimethylamino-N'''-(2-methyl-benzolsulfonyl)-guanidin,
N'-(4-Methoxy-6-methyl-pyrimidin-2-yl)-N''-methoxy-N'''-(2-chlor-benzolsulfonyl)-guanidin und
N'-(4-Methoxy-6-methyl-pyrimidin-2-yl)-N''-dimethylamino-N'''-(2-chlor-benzolsulfonyl)-guanidin.

Die allgemeine Formel (I) steht - wenn M für Wasserstoff steht - für die einzelnen Tautomeren der Formeln (IA) und (IB)

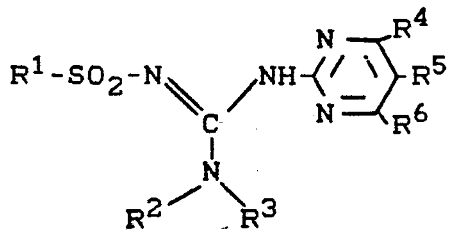

(IA)

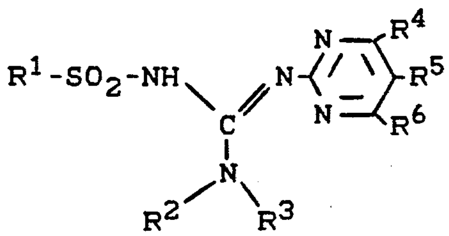

(IB)

in welchen

$R^1$, $R^2$, $R^3$, $R^4$, $R^5$ und $R^6$ die oben angegebenen Bedeutungen haben,

sowie für Gemische der Tautomeren (IA) und (IB).

Das Mischungsverhältnis (IA)/(IB) hängt von aggregationsbestimmenden Faktoren, wie z. B. Temperatur, Lösungsmittel und Konzentration ab.

Für den Fall, daß neben M auch $R^2$ für Wasserstoff steht, ist eine weitere tautomere Form (IC) möglich:

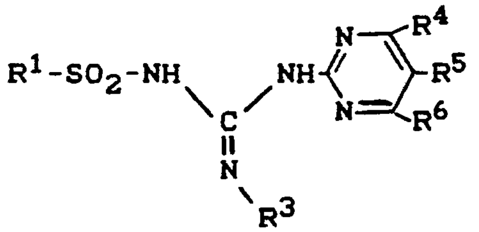

(IC)

Man erhält die neuen Sulfonylguanidinopyrimidin-Derivate der Formel (I)

(a) für den Fall, daß M für Wasserstoff steht,

wenn man Guanidinopyrimidin-Derivate der Formel (II)

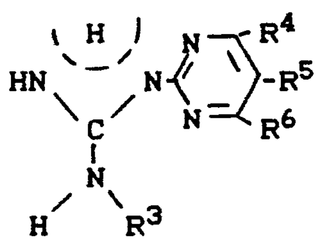

(II)

in welcher

$R^3$, $R^4$, $R^5$ und $R^6$ die oben angegebenen Bedeutungen haben,

mit Sulfonsäurechloriden der Formel (III)

$R^1-SO_2-Cl$ (III)

in welcher

$R^1$ die oben angegebene Bedeutung hat,

in Gegenwart von Säureakzeptoren und gegebenenfalls in Gegenwart von Verdünnungsmitteln umsetzt;

oder

(b) für den Fall, daß M für Wasserstoff steht und $R^2$ für Wasserstoff oder $C_1$-$C_4$-Alkyl steht,

wenn man die nach dem oben unter (a) angegebenen Verfahren erhältlichen Sulfonylguanidinopyrimidin-Derivate der Formel (ID)

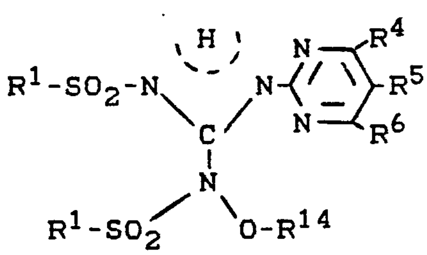

(ID)

in welcher

$R^1$, $R^4$, $R^5$, $R^6$ und $R^{14}$ die oben angegebenen Bedeutungen haben,

mit Aminoverbindungen der Formel (IV)

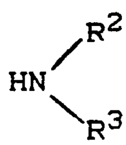

(IV)

in welcher

$R^2$ für Wasserstoff oder $C_1$-$C_4$-Alkyl steht und

$R^3$ die oben angegebene Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder

(c) für den Fall, daß M für Wasserstoff steht und

$R^2$ für einen Sulfonylrest der Formel $R^{11}-SO_2-$ steht, worin

$R^{11}$ die oben angegebene Bedeutung hat,

wenn man Sulfonylguanidinopyrimidin-Derivate der Formel (IE)

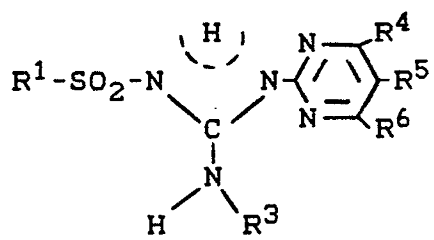

(IE)

in welcher

$R^1$, $R^3$, $R^4$, $R^5$ und $R^6$ die oben angegebenen Bedeutungen haben,

mit Sulfonsäurechloriden der Formel (V)

$R^{11}-SO_2-Cl$ (V)

in welcher

$R^{11}$ die oben angegebene Bedeutung hat,

in Gegenwart von Säureakzeptoren und gegebenenfalls in Gegenwart von Verdünnungsmitteln umsetzt;

oder

(d) ebenfalls für den Fall, daß M für Wasserstoff steht und

$R^2$ für einen Sulfonylrest der Formel $R^{11}-SO_2-$ steht, worin

$R^{11}$ die oben angegebene Bedeutung hat,

wenn man Sulfonylguanidinopyrimidin-Derivate der Formel (VI)

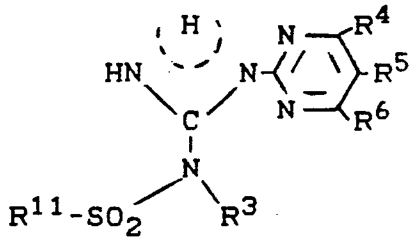

(VI)

in welcher

$R^3$, $R^4$, $R^5$, $R^6$ und $R^{11}$ die oben angegebenen Bedeutungen haben,

mit Sulfonsäurechloriden der Formel (III)

$$R^1-SO_2-Cl \qquad (III)$$

in welcher

$R^1$ die oben angegebene Bedeutung hat,

in Gegenwart von Säureakzeptoren und gegebenenfalls in Gegenwart von Verdünnungsmitteln umsetzt;

oder

(e) für den Fall, daß M für Wasserstoff steht und

$R^2$ für Wasserstoff oder $C_1$-$C_4$-Alkyl steht,

wenn man Sulfonylisothioureidopyrimidin-Derivate der Formel (VII)

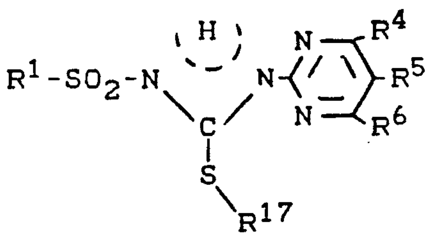

(VII)

in welcher

$R^1$, $R^4$, $R^5$ und $R^6$ die oben angegebenen Bedeutungen haben und

$R^{17}$ für $C_1$-$C_4$-Alkyl oder Benzyl steht,

mit Aminoverbindungen der Formel (IV)

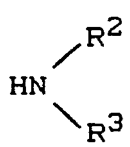

(IV)

in welcher

$R^2$ für Wasserstoff oder $C_1$-$C_4$-Alkyl steht und

$R^3$ die oben angegebene Bedeutung hat,

gegebenenfalls in Gegenwart von Verdünnungsmitteln umsetzt;

oder

(f) für den Fall, daß M für ein Metalläquivalent steht, wenn man die nach den oben unter (a), (b), (c), (d) und (e) angegebenen Verfahren erhältlichen Verbindungen der Formel (I), in welcher M für Wasserstoff steht und $R^1$, $R^2$, $R^3$, $R^4$, $R^5$ und $R^6$ die oben angegebenen Bedeutungen haben,

mit Metallhydroxiden, -hydriden oder -alkanolaten oder mit metallorganischen Verbindungen, gegebenenfalls in Gegenwart von Verdünnungsmitteln umsetzt;

oder

(g) für den Fall, daß 1:1-Addukte von Verbindungen der Formel (I) mit starken Säuren herzustellen sind,

wenn man Verbindungen der Formel (I), in welcher M für Wasserstoff steht und $R^1$, $R^2$, $R^3$, $R^4$, $R^5$ und $R^6$ die oben angegebenen Bedeutungen haben,

mit starken Säuren, gegebenenfalls in Gegenwart von Verdünnungsmitteln umsetzt.

Die neuen Sulfonylguanidinopyrimidin-Derivate der Formel (I) und ihre Addukte mit starken Säuren zeichnen sich durch eine starke herbizide Wirksamkeit aus.

Überraschenderweise zeigen die neuen Verbindungen der Formel (I) erheblich bessere herbizide Wirkung als vorbekannte Guanidine gleicher Wirkungsrichtung.

Gegenstand der Erfindung sind vorzugsweise Verbindungen der Formel (I), ein welcher

M für Wasserstoff oder eine Natrium-, Kalium- oder Calcium-äquivalent steht,

$R^1$ für einen gegebenenfalls substituierten Phenylrest

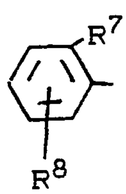

steht, worin

$R^7$ und $R^8$ gleich oder verschieden sind und für Wasserstoff, Fluor, Chlor, Brom, Cyano, Nitro, Methyl [welches gegebenenfalls durch Fluor, Chlor,

Cyano, $C_1$-$C_3$-Alkoxy-carbonyl, $C_1$-$C_3$-Alkoxy, $C_1$-$C_3$-Alkylthio, $C_1$-$C_3$-Alkylsulfinyl oder $C_1$-$C_3$-Alkylsulfonyl substituiert ist], für $C_1$-$C_2$-Alkoxy [welches gegebenenfalls durch Fluor, Chlor, Cyano, $C_1$-$C_3$-Alkoxy-carbonyl, $C_1$-$C_3$-Alkoxy, $C_1$-$C_3$-Alkylthio, $C_1$-$C_3$-Alkylsulfinyl oder $C_1$-$C_3$-Alkylsulfonyl substituiert ist], für $C_1$-$C_3$-Alkylthio, $C_1$-$C_3$-Alkylsulfinyl oder $C_1$-$C_3$-Alkylsulfonyl [welches gegebenenfalls durch Fluor, Chlor, Cyano oder $C_1$-$C_3$-Alkoxy-carbonyl substituiert sind], für Di-($C_1$-$C_3$-alkyl)-amino-sulfonyl, $C_1$-$C_3$-Alkoxy-methylamino-sulfonyl, Phenyl, Phenoxy, für $C_1$-$C_4$-Alkoxy-carbonyl, [welches gegebenenfalls durch Fluor, Chlor, Brom, Cyano oder $C_1$-$C_3$-Alkoxy substituiert ist], $C_3$-$C_6$-Cycloalkoxy-carbonyl, $C_1$-$C_4$- Alkylthio-carbonyl, Di-($C_1$-$C_3$-alkyl)-amino-carbonyl, $C_1$-$C_3$-Alkoxymethylamino-carbonyl, oder für $C_2$-$C_4$-Alkenyl [welches gegebenenfalls durch Fluor, Chlor, Cyano oder $C_1$-$C_3$-Alkoxy-carbonyl substituiert ist] stehen;

in welcher weiter

$R^1$ für einen gegebenenfalls substituierten Benzylrest

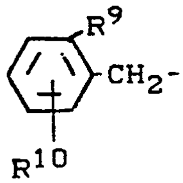 steht, worin

$R^9$ und $R^{10}$ gleich oder verschieden sind und für Wasserstoff, Fluor, Chlor, Cyano, Nitro, Methyl, Methoxy oder $C_1$-$C_3$-Alkoxy-carbonyl stehen;

in welcher weiter

$R^2$ für Wasserstoff, Methyl oder einen Sulfonylrest $R^{11}$-$SO_2$- steht, worin

$R^{11}$ für $C_1$-$C_8$-Alkyl [welches gegebenenfalls durch Fluor, Chlor oder Cyano substituiert ist], für $C_1$-$C_8$-Alkoxy [welches gegebenenfalls durch Fluor, Chlor oder Cyano substituiert ist], für $C_2$-$C_8$-Alkenyl [welches gegebenenfalls durch Fluor, Chlor, Cyano oder Phenyl substituiert ist], für $C_2$-$C_8$-Alkenyloxy [welches gegebenenfalls durch Fluor, Chlor, Cyano oder Phenyl substituiert ist], für $C_1$-$C_6$-Alkylamino, $C_3$-$C_6$-Cycloalkylamino oder Di-($C_1$-$C_3$-alkyl)-amino [welche gegebenenfalls durch Fluor, Chlor, Cyano, Phenyl, Phenoxy oder $C_1$-$C_2$-Alkoxy substituiert sind], für $C_2$-$C_6$-Alkenylamino [welches gegebenenfalls durch Fluor, Chlor, Cyano oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_3$-Alkoxy, $C_1$-$C_2$-Fluoralkoxy, $C_1$-$C_3$-Alkylthio, $C_1$-$C_3$-Alkylsulfinyl, $C_1$-$C_3$-Alkylsulfonyl, Cyano, Nitro und/oder $C_1$-$C_3$-Alkoxy-carbonyl substituiert sind], für Benzyl [welches gegebenenfalls durch Fluor, Chlor, Cyano, Nitro, Methyl, Methoxy und/oder $C_1$-$C_2$-Alkoxy-carbonyl substituiert ist] oder für einen gegebenenfalls substituierten Phenylrest 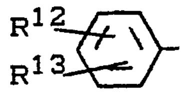 steht,

worin

$R^{12}$ und $R^{13}$ gleich oder verschieden sind und die vorausgehend für $R^7$ und $R^8$ vorzugsweise angegebenen Bedeutungen haben, jedoch nicht in jedem Einzelfall mit $R^7$ und $R^8$ identisch sind;

in welcher weiter

$R^3$ für den Rest -O-$R^{14}$ steht, worin

$R^{14}$ für $C_1$-$C_8$-Alkyl [welches gegebenenfalls durch Fluor oder Chlor substituiert ist], für $C_3$-$C_6$-Alkenyl [welches gegebenenfalls durch Chlor substituiert ist], für $C_1$-$C_3$-Alkoxy-carbonyl-$C_1$-$C_2$-alkyl, Phenyl, Phenylethyl oder

Benzyl [welches gegebenenfalls durch Fluor, Chlor, Nitro, Methyl, Methoxy oder $C_1$-$C_2$-Alkoxy-carbonyl substituiert ist] steht;

in welcher weiter

$R^3$ für den Rest 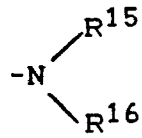 steht, worin

$R^{15}$ für Wasserstoff oder Methyl steht und

$R^{16}$ für Wasserstoff, $C_1$-$C_3$-Alkyl [welches gegebenenfalls durch Fluor, Chlor, Cyano, $C_1$-$C_2$-Alkoxy oder $C_1$-$C_2$-Alkoxy-carbonyl substituiert ist], für $C_3$-$C_6$-Cycloalkyl, Phenyl, Benzyl oder Phenylethyl [welche gegebenenfalls durch Fluor, Chlor, Brom, Nitro, Cyano, $C_1$-$C_2$-Alkyl, $C_1$-$C_2$-Alkoxy oder $C_1$-$C_2$-Alkoxy-carbonyl substituiert ist], für Acetyl, Methoxycarbonyl, Benzolsulfonyl oder Toluolsulfonyl steht;

in welcher weiter

$R^4$ für Wasserstoff, Chlor, Methyl [welches gegebenenfalls duch Fluor und/oder Chlor substituiert ist],

für $C_1$-$C_2$-Alkoxy [welches gegebenenfalls durch Fluor und/oder Chlor substituiert ist] oder für $C_1$-$C_2$-Alkylthio [welches gegebenenfalls durch Fluor und/oder Chlor substituiert ist] steht,

$R^5$ für Wasserstoff, Fluor, Chlor, Brom, Methyl, Acetyl oder $C_1$-$C_2$-Alkoxy-carbonyl steht und

$R^6$ für $C_1$-$C_3$-Alkyl [welches gegebenenfalls durch Fluor und/oder Chlor substituiert ist], für $C_1$-$C_3$-Alkoxy [welches gegebenenfalls durch Fluor und/oder Chlor substituiert ist], für Amino, $C_1$-$C_3$-Alkylamino oder Di-($C_1$-$C_3$-alkyl)-amino steht

- mit der Maßgabe, daß $R^4$ und $R^6$ nicht gleichzeitig für Methyl stehen.

Gegenstand der Erfindung sind vorzugsweise ferner 1:1-Addukte von Verbindungen der Formel (I) - wie vorausgehend definiert, wobei M für Wasserstoff steht - mit Halogenwasserstoffsäuren, wie Hydrogenchlorid, Hydrogenbromid und Hydrogeniodid, mit Schwefelsäure, Trifluoressigsäure, mit gegebenenfalls durch Fluor oder Chlor substituierten Alkansulfonsäuren mit bis zu 4 Kohlenstoffatomen oder auch mit Benzol- oder Naphthalin-sulfonsäuren, welche gegebenenfalls durch Fluor, Chlor, Brom oder Methyl substituiert sind.

Gegenstand der Erfindung sind insbesondere Verbindungen der Formel (I), in welcher

M für Wasserstoff oder ein Natrium-, Kalium- oder Calcium-äquivalent steht,

$R^1$ für einen substituierten Phenylrest 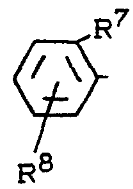

steht, worin

$R^7$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylamino-sulfonyl, Phenyl, $C_1$-$C_4$-Alkoxy-carbonyl, Difluormethylthio oder Trifluormethylthio steht und

$R^8$ für Wasserstoff steht;

in welcher weiter

$R^2$ für Wasserstoff, Methyl oder einen Sulfonylrest $R^{11}$-$SO_2$- steht, worin

$R^{11}$ für $C_1$-$C_4$-Alkyl [welches gegebenenfalls durch Fluor oder Chlor substituiert ist], für $C_1$-$C_4$-Alkylamino oder Di-($C_1$-$C_2$-alkyl)-amino [welche gegebenenfalls durch Fluor substituiert sind] oder für einen gegebenenfalls substituierten Phenylrest 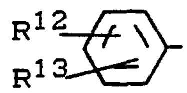 steht, worin

$R^{12}$ und $R^{13}$ gleich oder verschieden sind und für Wasserstoff, Fluor, Chlor, Brom, Cyano, Nitro, Methyl, Trifluormethyl, $C_1$-$C_2$-Alkoxy [welches gegebenenfalls durch Fluor, Chlor, Cyano oder $C_1$-$C_3$-Alkoxy-carbonyl substituiert ist], $C_1$-$C_3$-Alkylthio, $C_1$-$C_3$-Alkylsulfinyl oder $C_1$-$C_3$-Alkylsulfonyl [welche gegebenenfalls durch Fluor und/oder Chlor substituiert sind], Dimethylaminosulfonyl, Diethylaminosulfonyl, N-Methoxy-N-methylaminosulfonyl, Phenyl oder $C_1$-$C_4$-Alkoxy-carbonyl [welches gegebenenfalls durch Fluor, Chlor, Cyano oder $C_1$-$C_2$-Alkoxy substituiert ist] stehen;

in welcher weiter

$R^3$ für den Rest $-O-R^{14}$ steht, worin

$R^{14}$ für $C_1$-$C_4$-Alkyl [welches gegebenenfalls durch Chlor substituiert ist], für $C_3$-$C_4$-Alkenyl [welches gegebenenfalls durch Chlor substituiert ist], für $C_1$-$C_2$-Alkoxy-carbonyl-methyl, Phenyl, Phenylethyl oder Benzyl [welches gegebenenfalls durch Fluor, Chlor, Nitro, Cyano, Methyl, Methoxy oder $C_1$-$C_2$-Alkoxy-carbonyl substituiert ist] steht;

in welcher weiter

$R^3$ für den Rest 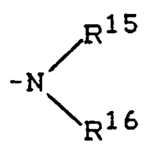 steht, worin

$R^{15}$ für Wasserstoff oder Methyl steht und

$R^{16}$ für Wasserstoff, $C_1$-$C_3$-Alkyl, $C_3$-$C_6$-Cycloalkyl, Phenyl, Acetyl, Methoxycarbonyl, Benzolsulfonyl oder Toluolsulfonyl steht;

in welcher weiter

$R^4$ für Wasserstoff, Chlor oder $C_1$-$C_2$-Alkoxy [welches gegebenenfalls durch Fluor und/oder Chlor substituiert ist] steht,

$R^5$ für Waserstoff, Methoxycarbonyl, Ethoxycarbonyl oder Acetyl steht und

$R^6$ für $C_1$-$C_2$-Alkyl [welches gegebenenfalls durch Fluor und/oder Chlor substituiert ist] oder $C_1$-$C_2$-Alkoxy [welches gegebenenfalls durch Fluor und/oder Chlor substituiert ist] steht,

sowie - für den Fall, daß M für Wasserstoff steht - die 1:1-Addukte der vorausgehend definierten Verbindungen mit Salzsäure, Schwefelsäure, Trifluoressigsäure, Methansulfonsäure, Benzolsulfonsäure und Toluolsulfonsäure.

Die oben einzeln genannten Verbindungen sind auch beim bevorzugten bzw. besonders bevorzugten Bereich ausgenommen.

Verwendet man beispielsweise für die Verfahrensvariante (a) 2-Fluor-benzolsulfonsäurechlorid und N'-(4-Methoxy-6-methyl-pyrimidin-2-yl)-N''-ethoxy-guanidin als Ausgangsstoffe, so kann der Reaktionsablauf durch folgendes Formelschema skizziert werden:

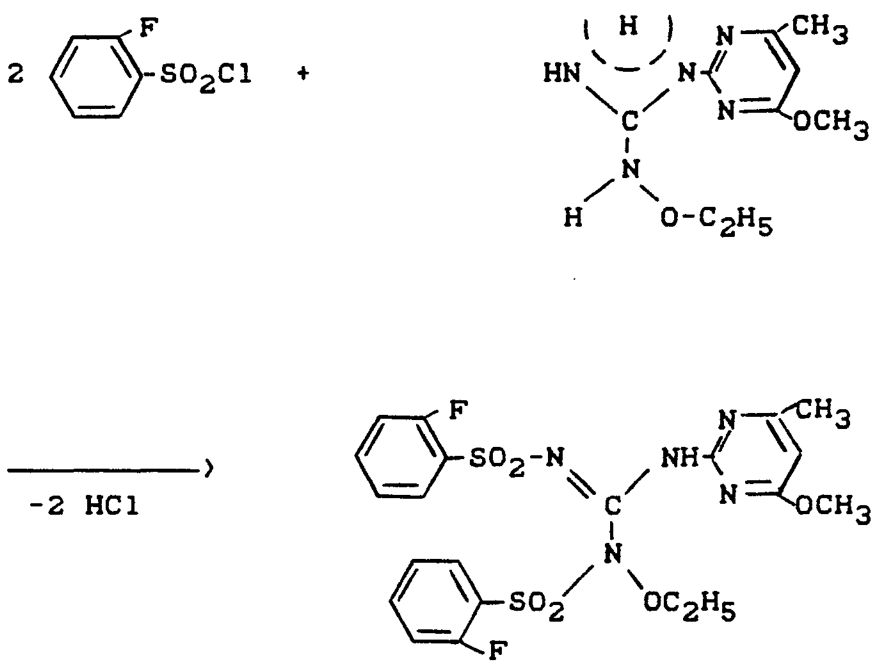


Verwendet man beispielsweise für die Verfahrensvariante (b) N'-(4-Difluormethoxy-6-methyl-pyrimidin-2-yl)-N''-methoxy-N'',N'''-bis-(2-trifluormethyl-benzolsulfonyl)-guanidin und Phenylhydrazin als Ausgangsstoffe, so kann der Reaktionsablauf durch das folgende Formelschema skizziert werden:

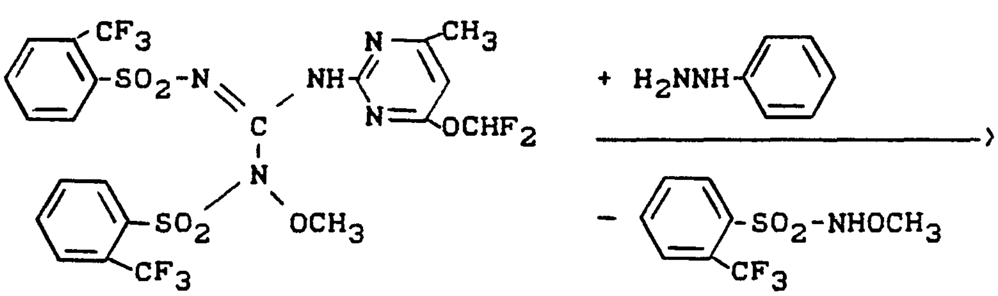

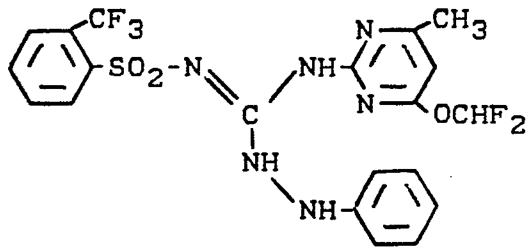

Verwendet man beispielsweise für die Verfahrensvariante (c) N'-(4,6-Dimethoxy-pyrimidin-2-yl)-N''-benzyloxy-N'''-(2-chlor-phenylmethylsulfonyl)-guanidin und Trifluormethan-sulfonsäurechlorid als Ausgangsstoffe, so kann der Reaktionsablauf durch das folgende Formelschema skizziert werden:

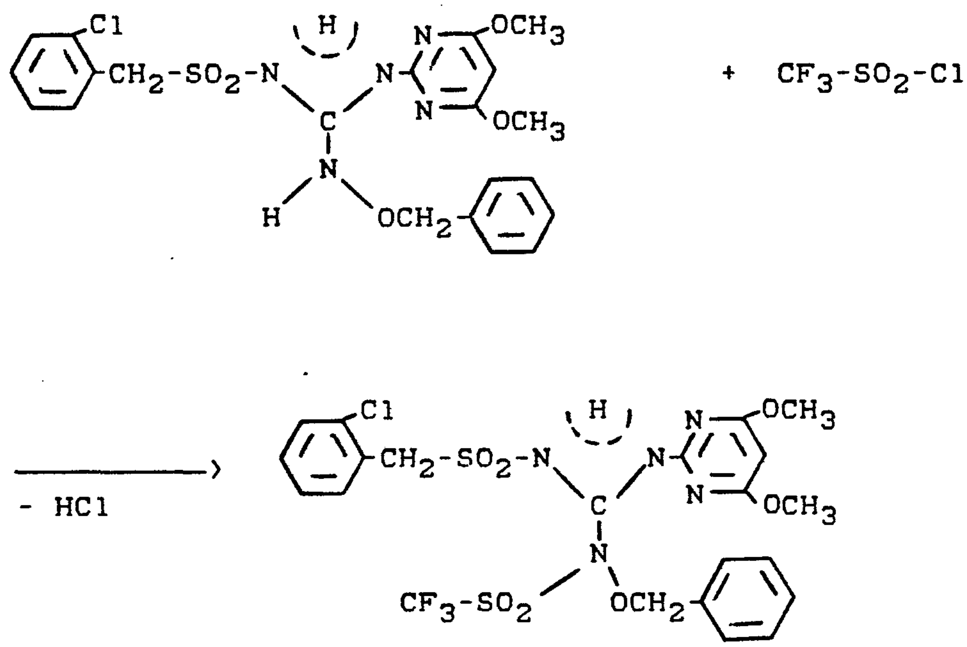


Verwendet man beispielsweise für die Verfahrensvariante

(d) N'-(4-Ethoxy-6-methyl-pyrimidin-2-yl)-N''-methoxy-N''-(2-ethoxycarbonyl-benzolsulfonyl)-guanidin und 2-Dimethylaminosulfonyl-benzolsulfonsäurechlorid als Ausgangsstoffe, so kann der Reaktionsablauf durch folgendes Formelschema skizziert werden:

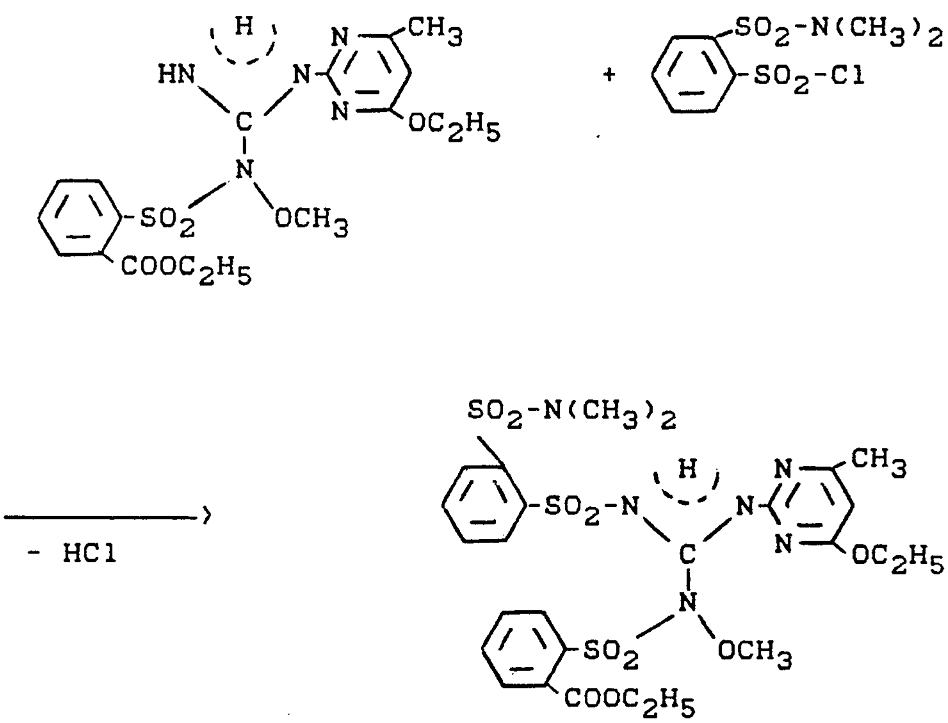


Verwendet man beispielsweise für die Verfahrensvariante (e) N'-(4-Methoxy-6-methyl-pyrimidin-2-yl)-N''-(2-methyl-benzolsulfonyl)-S-methyl-isothioharnstoff und N,N-Dimethylhydrazin als Ausgangsstoffe, so kann der Reaktionsablauf durch das folgende Formelschema skizziert werden:

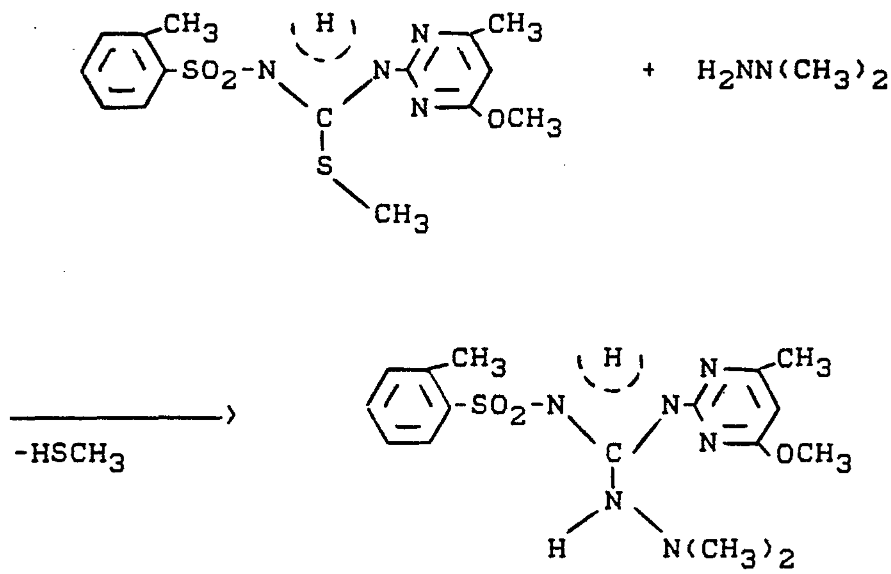


Verwendet man beispielsweise für die Verfahrensvariante (f) N'-(4,6-Dimethoxy-pyrimidin-2-yl)-N''-isopropoxy-N'''-(2-methoxycarbonyl-phenylmethylsulfonyl)-guanidin und Kaliumethanolat als Ausgangsstoffe, so kann der Reaktionsablauf durch das folgende Formelschema skizziert werden:

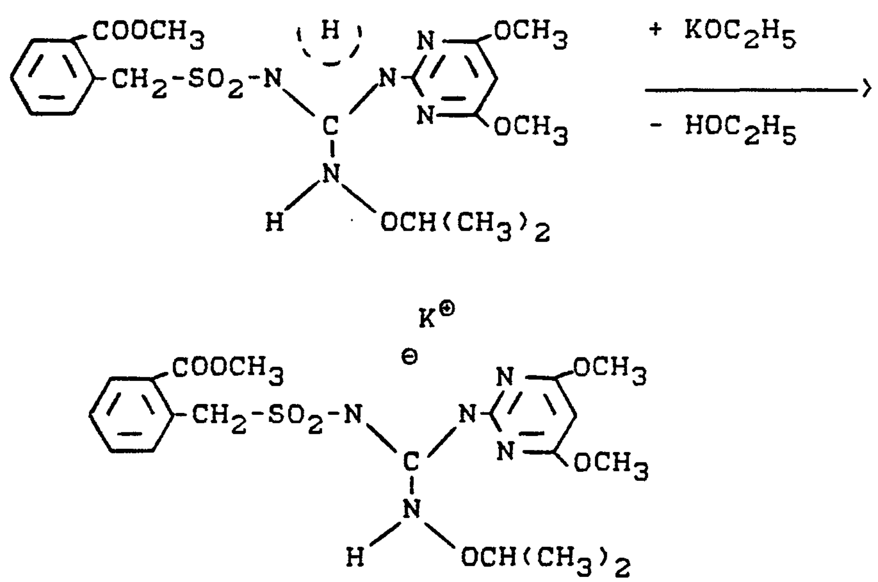

Verwendet man beispielsweise für die Verfahrensvariante (g) N'-(5-Acetyl-4-methyl-pyrimidin-2-yl)-N''-methoxy-N''-(2-chlor-benzolsulfonyl)-N'''-(2-difluormethoxy-benzolsulfonyl)-guanidin und Methansulfonsäure als Ausgangsstoffe, so kann der Reaktionsablauf durch folgendes Formelschema skizziert werden:

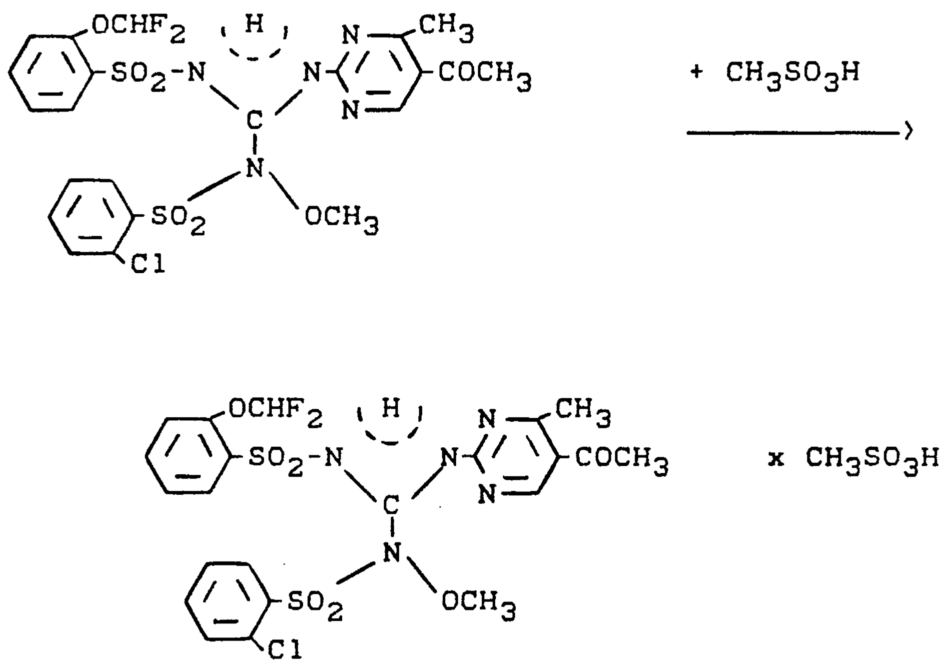


Die als Ausgangsstoffe für die Verfahrensvariante (a) zu verwendenden Guanidinopyrimidin-Derivate sind durch die Formel (II) allgemein definiert. In Formel (II) haben $R^3$, $R^4$, $R^5$ und $R^6$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (II) seien beispielsweise genannt:

N'-(4-Methyl-pyrimidin-2-yl)-, N'-(4-Ethyl-pyrimidin-2-yl)-, N'-(4-Methoxy-6-methyl-pyrimidin-2-yl)-, N'-(4-Ethoxy-6-methyl-pyrimidin-2-yl)-, N'-(4-Propoxy-6-methyl-2-yl)-, N'-(4-Isopropoxy-6-methyl-pyrimidin-2-yl)-, N'-(4-Chlor-6-methoxy-pyrimidin-2-yl)-, N'-(4-Chlor-6-ethoxy-pyrimidin-2-yl)-, N'-(4-Chlor-6-dimethylamino-pyrimidin-2-yl)-, N'-(4-Methyl-6-methylthio-pyrimidin-2-yl)-, N'-(4,6-Dimethoxy-pyrimidin-2-yl)-, N'-(4-Difluormethoxy-6-methyl-pyrimidin-2-yl)-, N'-(4-Dimethylamino-6-methyl-pyrimidin-2-yl)-N''-methoxy-guanidin, -N''-ethoxy-guanidin, -N''-propoxy-guanidin, -N''-isopropoxy-guanidin, -N''-butoxy-guanidin, -N''-isobutoxy-guanidin, -N''-sec.-butoxy-guanidin, -N''-pentoxy-guanidin, -N''-isopentoxy-guanidin, -N''-hexyloxy-guanidin, -N''-octyloxy-guanidin, -N''-allyloxy-guanidin, -N''-(2-chlor-ethoxy-)-guanidin, -N''-(2-fluor-ethoxy)-guanidin, -N''-(2-chlor-propoxy)-guanidin, -N''-(2-fluor-propoxy)-guanidin, -N''-(3-chlor-propoxy)-guanidin, -N''-(4-chlor-butoxy-)guanidin, -N''-methoxycarbonylmethoxy-guanidin, -N''-ethoxy-carbonyl-methoxy-guanidin, -N''-(1-methoxycarbonyl-ethoxy)-guanidin, -N''-(1-ethoxycarbonyl-ethoxy)-guanidin, -N''-dimethylaminocarbonyl-methoxy-guanidin, -N''-(2-phenyl-ethoxy)-guanidin, -N''-phenoxy-guanidin, -N''-(4-methyl-benzyloxy)-guanidin, -N''-(4-fluor-benzyloxy)-guanidin, -N''-(4-chlor-benzyloxy)-guanidin, -N''-(4-nitro-benzyloxy)-guanidin, -N''-(2,6-dichlor-benzyloxy)-guanidin, -N''-(4-methoxycarbonyl-benzyloxy)-guanidin und -N''-(4-ethoxycarbonyl-benzyloxy)-guanidin.

Die Ausgangsstoffe der Formel (II) sind Gegenstand älterer Schutzrechte (vergl. EP-OS 121 082); neu sind jedoch Verbindungen der Formel (II), in welcher

(a) $R^4$ für Chlor steht,

$R^5$ für Wasserstoff steht und

$R^6$ für $C_1$-$C_4$-Alkoxy [welches gegebenenfalls durch Fluor und/oder Chlor substituiert ist], für $C_1$-$C_4$-Alkylamino oder Di-($C_1$-$C_4$-alkyl)-amino steht; oder in welcher

(b) $R^4$ für $C_1$-$C_4$-Alkyl [welches gegebenenfalls durch Fluor und/oder Chlor substituiert ist] steht,

$R^5$ für Wasserstoff steht und

$R^6$ für durch Fluor und/oder Chlor substituiertes $C_1$-$C_4$-Alkoxy, für $C_1$-$C_4$-Alkylamino oder Di-($C_1$-$C_4$-alkyl)-amino steht; oder in welcher

(c) $R^4$ und $R^5$ für Wasserstoff stehen und

$R^6$ für $C_2$-$C_3$-Alkyl steht;

wobei jeweils $R^3$ die oben angegebene Bedeutung hat.

Man erhält die Guanidinopyrimidin-Derivate der Formel (II) - und damit auch die vorausgehend unter (a), (b) und (c) definierten neuen Verbindungen der Formeln (IIa), (IIb) und (IIc) - wenn man Cyanaminopyrimidin--Derivate der Formel (VIII)

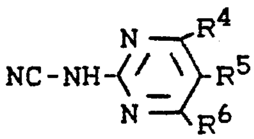

(VIII)

in welcher

$R^4$, $R^5$ und $R^6$ die oben angegebenen Bedeutungen haben,

mit Aminoverbindungen der Formel (IX)

$$H_2N\text{-}R^3 \quad (IX)$$

in welcher

$R^3$ die oben angegebene Bedeutung hat,

bzw. mit deren Hydrochloriden,

gegebenenfalls in Gegenwart von Verdünnungsmitteln, wie z. B. Ethanol, Isopropanol oder Butanol, bei Temperturen zwischen 20 °C und 150 °C, vorzugsweise zwischen 50 °C und 120 °C, umsetzt und gegebenenfalls die Umsetzungsprodukte mit Säureakzeptoren, wie z. B. Ammoniak, Natronlauge oder Soda behandelt.

Die Cyanaminopyrimidin-Derivate der Formel (VIII) sind Gegenstand älterer Schutzrechte (vergl. EP-OS 121 082); neu sind jedoch Verbindungen der Formel (VIII), in welcher

(a) $R^4$ für Chlor steht,

$R^5$ für Wasserstoff steht und

$R^6$ für $C_1$-$C_4$-Alkoxy [welches gegebenenfalls durch Fluor und/oder Chlor substituiert ist], für $C_1$-$C_4$-Alkylamino oder Di-($C_1$-$C_4$-alkyl)-amino steht; oder in welcher

(b) $R^4$ für $C_1$-$C_4$-Alkyl [welches gegebenenfalls durch Fluor und/oder Chlor substituiert ist] steht,

$R^5$ für Wasserstoff steht und

$R^6$ für durch Fluor und/oder Chlor substituiertes $C_1$-$C_4$-Alkoxy, für $C_1$-$C_4$-Alkylamino oder Di-($C_1$-$C_4$-alkyl)-amino steht; oder in welcher

(c) $R^4$ und $R^5$ für Wasserstoff stehen und

$R^6$ für $C_2$-$C_3$-Alkyl steht.

Man erhält die Verbindungen dr Formel (VIII) - und damit auch die vorausgehend unter (a), (b) und (c) definierten neuen Verbindungen der Formeln (VIIIa), (VIIIb) und (VIIIc) - im wesentlichen nach folgenden Synthesewegen:

($a^1$) Durch Umsetzung von Alkalimetall- oder Erdalkalimetall-Salzen von Cyanamid - wie z. B. Natriumcyanamid oder Calciumcyanamid - mit Chlorpyrimidinen der Formel (X)

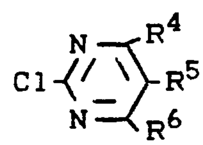

(X)

in welcher

$R^4$, $R^5$ und $R^6$ die oben angegebenen Bedeutungen haben,

gegebenenfalls in Gegenwart von inerten Verdünnungsmitteln, wie z. B. Ethanol, Aceton, Acetonitril oder Dimethylformamid, bei Temperaturen zwischen 0 °C und 100 °C. Nach Einengen und Auflösen des Rückstandes in Wasser können die Cyanaminopyrimidin-Derivate der Formel (VIII) durch Ansäuern, z. B. mit Salzsäure, ausgefällt und durch Absaugen isoliert werden.

Alternativ erhält man Cyanaminopyrimidin-Derivate der Formel (VIII)

($a^2$) für den Fall, daß $R^4$ und/oder $R^6$ für Hydroxy stehen, durch Umsetzung von Cyanoguanidin ('Dicyandiamid') mit geeigneten β-Dicarbonylverbindungen - oder deren Derivaten - wie z. B. 1,1-Dimethoxy-3-oxo-butan, Acetessigsäure-methylester oder -ethylester (vergl. J. Prakt. Chem. 77 (1908), 542 und J. Chem. Soc. 1948, 586) oder Malonsäure-dimethylester oder -diethylester (vergl. DE-PS 158 591).

Die aus Acetessigsäureestern bzw. Malonsäureestern erhaltenen 2-Cyanamino-4-hydroxy-6-methyl bzw. -4,6-dihydroxy-pyrimidine können auf bekannte Weise

durch Umsetzung mit Alkylierungsmitteln, wie z. B. Dimethyl- oder Diethylsulfat, gegebenenfalls in Gegenwart von Verdünnungsmitteln, wie z. B. Wasser, Methanol, Ethanol, n- und iso-Propanol, Aceton, Dioxan oder Dimethylformamid, und in Gegenwart von Säurebindemitteln, wie z. B. Natrium- oder Kalium-hydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidurch einer N-Alkylierung wird gegebenenfalls mit einem Acylierungsmittel, wie z. B. Acetanhydrid oder Acetylchlorid acyliert und nach der Alkylierung mit wässrigen Säuren oder Basen wieder entacyliert.

In einem weiteren Alternativverfahren erhält man Cyanamino-pyrimidin-Derivate der Formel (VIII), wenn man

($a^3$) Aminopyrimidine der Formel (XI)

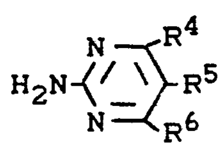

(XI)

in welcher

$R^4$, $R^5$ und $R^6$ die oben angegebenen Bedeutungen haben,

mit Carbonylisothiocyanaten der Formel (XII)

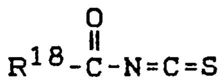

(XII)

in welcher

$R^{18}$ für Ethoxy oder Phenyl steht,

gegebenenfalls in Gegenwart eines inerten Verdünnungsmittels, wie z. B. Aceton, Acetonitril oder Toluol, bei Temperaturen zwischen 0 °C und 100 °C umsetzt, die hierbei gebildeten Carbonylthioharnstoffe der Formel (XIII)

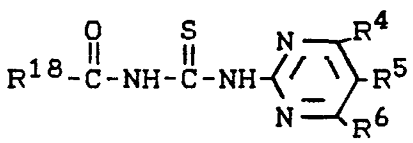

(XIII)

in welcher

$R^4$, $R^5$, $R^6$ und $R^{18}$ die oben angegebenen Bedeutungen haben,

gegebenenfalls nach Einengen durch Absaugen isoliert und mit wässrigen Alkalimetall- oder Erdalkalimetall-hydroxidlösungen, wie z. B. Natronlauge,

gegebenenfalls in Gegenwart eines organischen Lösungsmittels, wie z. B. Tetrahydrofuran oder Dioxan, bei Temperaturen zwischen 0 °C und 120 °C umsetzt und die nach Ansäuern, z. B. mit Salzsäure, kristallin erhaltenen Thioharnstoffe der Formel (XIV)

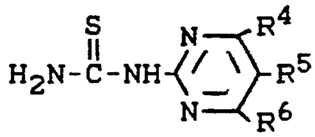

(XIV)

in welcher

$R^4$, $R^5$ und $R^6$ die oben angegebenen Bedeutungen haben,

durch Absaugen isoliert und Metallverbindungen, welche Schwefelwasserstoff binden können, wie z. B. mit Blei(II)-acetat, Kupfer(II)-acetat, Quecksilber(II)-acetat oder Eisen(II)-acetat, in Gegenwart von wässrigen Alkalimetall- oder Erdalkalimetall-hydroxidlösungen, wie z. B. Natronlauge, bei Temperaturen zwischen 20 °C und 100 °C umsetzt, nach Ende der Umsetzung filtriert und das Filtrat mit einer Säure, wie z. B. Essigsäure, ansäuert.

Die hierbei kristallin anfallenden Produkte der Formel (VIII) können durch Absaugen isoliert werden.

Die Ausgangsstoffe für die vorausgehend unter ($a^1$), ($a^2$) und ($a^3$) beschriebenen Herstellungsverfahren für die Cyanaminopyrimidin-Derivate der Formel (VIII) sind bekannt und/oder können nach an sich bekannten Verfahren hergestellt werden.

Hierzu gehören die Chlorpyrimidine der Formel (X) (vergl. J. Chem. Soc. (C) 1966, 2031; Chem. Pharm. Bull. 11 (1963), 1382 - 1388 und Arch. Pharm. 295 (1962), 649 - 657), die Aminopyrimidine der Formel (XI) (vergl. Chem. Pharm. Bull. 11 (1963), 1382 - 1388); J. Chem. Soc. 1946, 81 und US-PS 4 299 960 ) und die Carbonylisothiocyanate der Formel (XII) (vergl. J. Heterocycl. Chem. 5 (1968), 837 und US-PS 4 160 037).

Die weiter als Ausgangsstoffe zu verwendenden Aminoverbindungen der Formel (IX) sind ebenfalls bereits bekannt und/oder können nach an sich bekannten Verfahren hergestellt werden (vergl. Chem. Pharm. Bull. 15 (1967), 345; Bull. Soc. Chim. France 1958, 664; Synthesis 1976, 682; J. Chem. Soc. 1930, 228 und Helv. Chem. Acta 45 (1962), 1387).

Die weiter als Ausgangsstoffe für die Verfahrensvariante (a) zu verwendenden Sulfonsäurechloride sind durch die Formel (III) allgemein definiert. In Formel (III) hat $R^1$ vorzugsweise bzw. insbesondere die gleiche Bedeutung, wie sie oben im Rahmen der Substituentendefinition der Formel (I) vorzugsweise bzw. als insbesondere bevorzugt genannt ist.

Als Ausgangsstoffe der Formel (III) seien bespielsweise genannt:

2-Chlor-, 3-Chlor-, 4-Chlor-, 2,5-Dichlor-, 2-Fluor-, 4-Fluor-, 2-Brom-, 4-Brom-, 2-Nitro-, 4-Nitro-, 2-Cyano-, 2-Methyl-, 4-Methyl-, 2-Chlormethyl-, 2-Trifluormethyl-, 2-Methoxy-, 2-Difluormethoxy-, 2-Trifluormethoxy-, 2-Methylthio-, 2-Methylthiomethyl-, 2-Methylsulfinylmethyl-, 2-Methylsulfonylmethyl-, 2-Dimethylaminosulfonyl-, 2-Diethylaminosulfonyl-, 2-Phenyl-, 2-Methoxycarbonyl-, 2-Ethoxycarbonyl-, 2-Propoxycarbonyl-, 2-Isopropoxycarbonyl-, 2-Butoxycarbonyl-, 2-Dimethylaminocarbonyl-, 2-Diethylaminocarbonyl-, 2-Phenoxy-, 2-Methyl-5-chlor-, 2-Chlor-5-trifluormethyl-, 2-Methylsulfonyl-, 2-Ethylsulfonyl-, 2-Chlor-4-trifluormethoxy-, 3-Chlor-4-trifluormethoxy-, 2-Trifluormethoxy-5-chlor-, 3,5-Dichlor-, 2-Difluormethylthio- und 2-Trifluormethylthiobenzolsulfonsäurechlorid, ferner Methan-, Chlormethan-, Trifluormethan-, Ethan-, 2-Chlorethan-, Ethen-, Propan-, Butan-, Perfluorbutan- und Perfluor-octan-sulfonsäurechlorid; Methyl-, Ethyl-, Propyl-, Isopropyl-, Cyclohexyl-, Dimethyl- und Diethyl-sulfamidsäurechlorid sowie

(2-Chlor-phenyl)-, (2-Cyano-phenyl)- und (2-Methoxycarbonyl-phenyl)-methansulfonsäurechlorid.

Die Sulfonsäurechloride der Formel (III) sind bekannt und/oder können nach an sich bekannten Verfahren hergestellt werden( vergl. J. Org. Chem. 33 (1968), 2104; J.Org. Chem. 25 (1960), 1824; DE-AS 2 308 262; EP-OS 23 140, 23 141, 23 422, 35 893, 48 143, 51 466, 64 322, 70 041, 44 808, 44 809; US-PS 2 929 820, 4 282 242, 4 348 220 und 4 372 778 sowie Angew. Chem. 93 (1981), 151).

Man erhält diese Verbindungen im wesentlichen nach folgenden zwei Synthesewegen:

(1) durch Umsetzung der entsprechenden Sulfonsäuren $R^1SO_3H$ bzw. von deren Alkalimetall- oder Erdalkalimetall-Salzen mit Halogenierungsmitteln, wie z. B. Phosphor(V)chlorid (Phosphorpentachlorid), Phosphorylchlorid (Phosphoroxychlorid), Thionylchlorid, Phosgen oder Benzotrichlorid, gegebenenfalls in Gegenwart von Katalysatoren, wie z. B. Pyridin oder Dimethylformamid, und gegebenenfalls unter Verwendung von inerten Verdünnungsmitteln, wie z. B. Methylenchlorid, Chloroform, Acetonitril, Chlorbenzol und/oder Sulfolan bei Temperaturen zwischen -20 °C und +150 °C, vorzugsweise zwischen 0 °C und

+100 °C; nach Verdünnen mit Wasser können die Sulfonsäurechloride - soweit sie kristalllin anfallen - durch Absaugen isoliert werden oder durch Extrahieren mit einem mit Wasser nicht mischbaren Lösungsmittel, wie z. B. Methylenchlorid, Diethylether oder Hexan, Waschen und Trocknen der Extrakte, Einengen und Umkristallisieren bzw. Destillieren gereinigt werden; oder auch

(2) auf an sich bekannte Weise (vergl. J. Org. Chem. 25 (1960), 1824; DE-OS 2 308 262 und EP-OS 59 241) durch Umsetzung entspechender Aminoverbindungen $R^1-NH_2$ mit Natriumnitrit und Salzsäure, gegebenenfalls in Gegenwart von Essigsäure, bei Temperaturen zwischen -10 °C und +20 °C, vorzugsweise zwischen -5 °C und +10 °C, und anschließend (in situ) mit Schwefeldioxid oder einem Salz der schwefeligen Säure, wie z. B. Natriumsulfit oder Natriumhydrogensulfit in Gengenwart einer Kupferverbindung, wie z. B. Kupferchlorid oder Kupfersulfat, als Katalysator bei Temperaturen zwischen 0 °C und 80 °C, vorzugsweise zwischen 10 °C und 60 °C.

Die Aufarbeitung kann auf übliche Weise erfolgen: Bei Verdünnen mit Wasser fallen die Sulfonsäurechloride im allgemeinen kristallin an und können durch Absaugen isoliert werden. Sie können aber auch aus der wässrigen Dispersion mit einem mit Wasser praktisch nicht mischbaren Lösungsmittel,

wie z. B. Methylenchlorid oder Diethylether, extrahiert, getrocknet und durch Vakuumdestillation gereinigt werden.

Die als Ausgangsstoffe für die Verfahrensvariante (b) zu verwendenden Sulfonylguanidinopyrimidin-Derivate sind durch die Formel (ID) allgemein definiert. In Formel (ID) haben $R^1$, $R^4$, $R^5$, $R^6$ und $R^{14}$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (ID) seien genannt:

N'-(4-Chlor-6-methoxy-pyrimidin-2-yl)-N''-methoxy-
-N'',N'''-bis-(2-chlor-benzolsulfonyl)-,
-N'',N'''-bis-(2-brom-benzolsulfonyl)-,
-N'',N'''-bis-(2-fluor-benzolsulfonyl)-,
-N'',N'''-bis-(2-methyl-benzolsulfonyl)-,
-N'',N'''-bis-(2-trifluormethyl-benzolsulfonyl)-,
-N'',N'''-bis-(2-methoxy-benzolsulfonyl)-,
-N'',N'''-bis-(2-phenyl-benzolsulfonyl)- und
-N'',N'''-bis-(2-methoxycarbonyl-benzolsulfonyl)-
-guanidin,

N'-(4-Methyl-pyrimidin-2-yl)-N''-methoxy-
-N'',N'''-bis-(2-chlor-benzolsulfonyl)-,
-N'',N'''-bis-(2-methoxycarbonyl-benzolsulfonyl)-,
-N'',N'''-bis-(2-difluormethoxy-benzolsulfonyl)-,
-N'',N'''-bis-(2-diethylaminosulfonyl-benzolsulfonyl)-,
-N'',N'''-bis-(2-brom-benzolsulfonyl)-,
-N'',N'''-bis-(2-trifluormethylthio-benzolsulfonyl)-,
-N'',N'''-bis-(2-phenoxy-benzolsulfonyl)- und
-N'',N'''-bis-(2-methylsulfonyl-benzolsulfonyl)-
-guanidin,

N'-(4,6-Dimethoxy-pyrimidin-2-yl)-N''-methoxy-
-N'',N'''-bis-(2-chlor-benzolsulfonyl)-,
-N'',N'''-bis-(2-methoxycarbonyl-benzolsulfonyl)-,
-N'',N'''-bis-(2-difluormethoxy-benzolsulfonyl)-,
-N'',N'''-bis-(2-trifluormethoxy-benzolsulfonyl)-,
-N'',N'''-bis-(2-dimethylaminosulfonyl-benzolsulfonyl)-,
-N'',N'''-bis-(2-methylthio-benzolsulfonyl)-,
-N'',N'''-bis-(2-ethoxycarbonyl-benzolsulfonyl)-,
-N'',N'''-bis-(2-phenyl-benzolsulfonyl)-,
-N'',N'''-bis-(2-cyclopropyloxycarbonyl-benzolsulfonyl)-,
-N'',N'''-bis-(2-trifluormethylsulfonyl-benzolsulfonyl)-,
-N'',N'''-bis-(2-nitro-benzolsulfonyl)- und
-N'',N'''-bis-(2-cyano-benzolsulfonyl-
-guanidin,

N'-(4-Methoxy-6-methyl-pyrimidin-2-yl)-N''-methoxy-
-N'',N'''-bis-(2-chlor-benzolsulfonyl)-,
-N'',N'''-bis-(2-methoxycarbonyl-benzolsulfonyl)-,

-N'',N'''-bis-(2-methylsulfonyl-benzolsulfonyl)-,
-N'',N'''-bis-(2-methylthio-benzolsulfonyl)-,
-N'',N'''-bis-(2-trifluormethoxy-benzolsulfonyl)-,
-N'',N'''-bis-(2-ethoxycarbonyl-benzolsulfonyl)-,
-N'',N'''-bis-(2-trifluormethyl-benzolsulfonyl)-,
-N'',N'''-bis-(2-isopropylthio-benzolsulfonyl)-,
-N'',N'''-bis-(2-N-methoxy-N-methylaminosulfonyl-benzolsulfonyl)-,
-N'',N'''-bis-(2-trifluormethylthio-benzolsulfonyl)-,
-N'',N'''-bis-(2,5-dichlor-benzolsulfonyl)-,
-N'',N'''-bis-(2-phenoxy-benzolsulfonyl)- und
-N'',N'''-bis-(2-phenyl-benzolsulfonyl)-
-guanidin,

N'-(4-Difluormethoxy-6-methyl-pyrimidin-2-yl)-N''-methoxy-
-N'',N'''-bis-(2-ethoxycarbonyl-benzolsulfonyl)-,
-N'',N'''-bis-(2-brom-benzolsulfonyl)-,
-N'',N'''-bis-(2-methylsulfonylmethyl-benzolsulfonyl)-,
-N'',N'''-bis-(2-trifluormethylthio-benzolsulfonyl)-,
-N'',N'''-bis-(2-methoxy-benzolsulfonyl)-,
-N'',N'''-bis-(2-methoxycarbonyl-benzolsulfonyl)- und
-N'',N'''-bis-(2-chlor-benzolsulfonyl)-
-guanidin.

Die Verbindungen der Formel (ID) könnn nach dem oben unter (a) beschriebenen Verfahren hergetellt werden.

Die bei der Verfahrensvariante (b) weiter als Ausgangstoffe zu verwendenden Aminoverbindungen sind durch die Formel (IV) allgemein definiert. In Formel (IV) haben $R^2$ und $R^3$ vorzugsweise bzw. insbesondere die gleichen Bedeutungen, wie sie oben im Rahmen der Substituentendefinition der Formel (I) vorzugsweise bzw. als insbesndere bevorzugt angegeben worden sind.

Als Ausgangsstoffe der Formel (IV) seien beispielsweise genannt:

N,N-Dimethylhydrazin, Phenylhydrazin, O-Methyl-hydroxylamin, O-Ethyl-hydroxylamin, O,N-Dimethylhydroxylamin, O-Propyl-, O-Isopropyl-, O-Butyl-, O-sec.-Butyl-, O-Pentyl-, O-Isopentyl-, O-sec.-Pentyl-, O-Hexyl-, O-Isohexyl-, O-Heptyl-, O-Isoheptyl-, O-Octyl-, O-Isooctyl-, O-Allyl, O-Crotyl-, O-(2-Chlor-ethyl)-, O-(2-Fluor--ethyl)-, O-(2-Chlor-propyl)-, O-(3-Chlor-propyl)-, O-(4-Chlor-butyl)-, O-Methoxycarbonylmethyl-, O-Ethoxycarbonylmethyl-, O-(1-Methoxycarbonyl)-ethyl-, O-(1-Ethoxycarbonyl)-ethyl-, O-Aminocarbonylmethyl-, O-(2-Phenyl-ethyl)-, O-Phenyl-, O-(4-Methyl-benzyl)-, O-(4--Fluor-benzyl)-, O-(4-Chlor-benzyl)-, O-(4-Nitro-benzyl)-, O-(2,6-Dichlor-benzyl)-, O-(4-Methoxycarbonyl--benzyl)- und O-(4-Ethoxycarbonyl-benzyl)-hydroxylamin.

Aminoverbindungen der Formel (IV) sind bekannt und können nach an sich bekannten Verfahren hergestellt werden (vergl. Chem. Pharm. Bull. 15, (1967), 345; Bull. Soc. Chim. France 1958, 664; Synthesis 1976, 682; J. Chem. Soc. 1930, 228 und Helv. Chim. Acta 45 (1962), 1387).

Die bei der Verfahrensvariante (c) als Ausgangsstoffe zu verwendenden Sulfonylguanidinopyrimidin-Derivate sind durch die Formel (IE) allgemein definiert. In Formel (IE) haben $R^1$, $R^3$, $R^4$, $R^5$ und $R^6$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der formel (IE) seien genannt:

N'-(4,6-Dimethoxy-pyrimidin-2-yl)-N''-methoxy-N'''-(2-chlor-benzolsulfonyl-guanidin,
-N'''-(3-chlor-benzolsulfonyl)-,
-N'''-(4-chlor-benzolsulfonyl)-,
-N'''-(2-fluor-benzolsulfonyl)-,
-N'''-(4-fluor-benzolsulfonyl)-,
-N'''-(2-brom-benzolsulfonyl)-,
-N'''-(4-brom-benzolsulfonyl)-,
-N'''-(2-cyano-benzolsulfonyl)-,
-N'''-(2-nitro-benzolsulfonyl)-,

-N'''-(4-nitro-benzolsulfonyl)-,
-N'''-(2-methyl-benzolsulfonyl)-,
-N'''-(4-methyl-benzolsulfonyl)-,
-N'''-(2-chlormethyl-benzolsulfonyl)-,
-N'''-(2-trifluormethyl-benzolsulfonyl)-,
-N'''-(2-methoxy-benzolsulfonyl)-,
-N'''-(4-methoxy-benzolsulfonyl)-,
-N'''-(2-methylthio-benzolsulfonyl)-,
-N'''-(2-difluormethoxy-benzolsulfonyl)-,
-N'''-(2-trifluormethoxy-benzolsulfonyl)-,
-N'''-(2-methylthiomethyl-benzolsulfonyl)-,
-N'''-(2-dimethylamino-sulfonyl-benzolsulfonyl)-,
-N'''-(2-phenyl-benzolsulfonyl)-,
-N'''-(2-methoxysulfonyl-benzolsulfonyl)-,
-N'''-(2-methoxycarbonyl-benzolsulfonyl)-,
-N'''-(2-ethoxycarbonyl-benzolsulfonyl)-,
-N'''-(2-propoxycarbonyl-benzolsulfonyl)-,
-N'''-(2-methylaminocarbonyl-benzolsulfonyl)-,
-N'''-(2-ethylaminocarbonyl-benzolsulfonyl)-,
-N'''-(2-propylaminocarbonyl-benzolsulfonyl)-,
-N'''-(2-methoxyaminocarbonyl-benzolsulfonyl)-,
-N'''-(2-ethoxyaminocarbonyl-benzolsulfonyl)-,
-N'''-(2-propoxyaminocarbonyl-benzolsulfonyl)-,
-N'''-(2-dimethylaminocarbonyl-benzolsulfonyl)- und
-N'''-(2-diethylaminocarbonyl-benzolsulfonyl)-guanidin;

ferner

N'-(4,6-Dimethoxy-pyrimidin-2-yl)-N''-ethoxy-N'''-
-benzol-sulfonyl-,

-N'''-(2-chlor-benzolsulfonyl)-,
-N'''-(3-chlor-benzolsulfonyl)-,
-N'''-(4-chlor-benzolsulfonyl)-,
-N'''-(2,4-dichlor-benzolsulfonyl)-,
-N'''-(2,5-dichlor-benzolsulfonyl)-,
-N'''-(2-difluormethoxy-benzolsulfonyl)-,
-N'''-(2-trifluormethoxy-benzolsulfonyl)-,
-N'''-(2-methylthio-benzolsulfonyl)-,
-N'''-(2-methyl-benzolsulfonyl)-,
-N'''-(2-methylthiomethyl-benzolsulfonyl)-,
-N'''-(2-dimethylaminosulfonyl-benzolsulfonyl)-,
-N'''-(2-methoxysulfonyl-benzolsulfonyl)-,
-N'''-(2-methoxycarbonyl-benzolsulfonyl)-,
-N'''-(2-ethoxycarbonyl-benzolsulfonyl)-,
-N'''-(2-methylaminocarbonyl-benzolsulfonyl)-,
-N'''-(2-ethylaminocarbonyl-benzolsulfonyl)-,
-N'''-(2-methoxyaminocarbonyl-benzolsulfonyl)- und
-N'''-(2-dimethylaminocarbonyl-benzolsulfonyl)-guanidin;

ferner

N'-(4,6-Dimethoxy-pyrimidin-2-yl)-N''-propoxy-,
-N''-isopropoxy-,
-N''-butoxy
-N''-isobutoxy-,
-N''-sec.-butoxy,-
-N''-pentyloxy-,
-N''-hexyloxy-,
-N''-octyloxy-,
-N''-allyloxy-,

-N''-crotyloxy-,
-N''-(3-chlor-propoxy)-,
-N''-methyloxycarbonyl-methoxy-,
-N''-ethoxycarbonyl-methoxy-,
-N''-(1-methoxy-carbonyl-ethoxy)-,
-N''-(1-ethoxycarbonyl-ethoxy)-,
-N''-(2-phenyl-ethoxy)-,
-N''-phenoxy-,
-N''-benzyloxy-,
-N''-(4-methyl-benzyloxy)-,
-N''-(4-fluor-benzyloxy)-,
-N''-(4-chlor-benzyloxy)-,
-N''-(4-nitro-benzyloxy)-,
-N''-(2,6-dichlor-benzyloxy)-,
-N''-(4-methoxycarbonyl-benzyloxy)- und
-N''-(4-ethoxycarbonyl-benzyloxy)-,
-N'''-(2-chlor-benzolsulfonyl)-guanidin sowie
-N'''-(2-methoxycarbonyl-benzolsulfonyl)-guanidin;

ferner

N'-(4-Methoxy-6-methyl-pyrimidin-2-yl)-N''-methoxy-
-N'''-(2-chlor-benzolsulfonyl)-,
-N'''-(2-methyl-benzolsulfonyl)-,
-N'''-(2-methylthio-benzolsulfonyl)-,
-N'''-(2-difluormethoxy-benzolsulfonyl)- und
-N'''-(2-trifluormethoxy-benzolsulfonyl)-guanidin;

ferner

N'-(4-Ethoxy-6-methyl-pyrimidin-2-yl)- und N'-(4-Methyl-pyrimidin-2-yl)-N''-methoxy-N'''-(2-chlor-benzolsulfonyl)-guanidin;

ferner

N'-(4-Chlor-6-methoxy-pyrimidin-2-yl)-N''-methoxy-
-N'''-(2-chlor-benzolsulfonyl)-,
-N'''-(2-methoxycarbonyl-benzolsulfonyl)-,
-N'''-(2-difluormethylthio-benzolsulfonyl)- und
-N'''-(2-dimethylaminosulfonyl-benzolsulfonyl)-guanidin;

ferner

N'-(4-Chlor-6-ethoxy-pyrimidin-2-yl)-N''-ethoxy-
-N'''-(2-brom-benzolsulfonyl)-,
-N'''-(2-phenyl-benzolsulfonyl)-,
-N'''-(2-dimethylaminocarbonyl-benzolsulfonyl)-,
-N'''-(2-methylthiomethyl-benzolsulfonyl)-,
-N'''-(2-phenoxy-benzolsulfonyl)- und
-N'''-(isopropoxy-benzolsulfonyl)-guanidin;

ferner

N'-(4-Chlor-6-dimethylamino-pyrimidin-2-yl)-N''-methoxy-
-N'''-(2-fluor-benzolsulfonyl)-,
-N'''-(2-difluormethylthio-benzolsulfonyl)-,
-N'''-(2-methyl-benzolsulfonyl)-,
-N'''-(2-ethoxycarbonyl-benzolsulfonyl)- und
-N'''-(2-cyano-benzolsulfonyl)-guanidin;

ferner

N'-(4-Methyl-6-methylthio-pyrimidin-2-yl)-N''-allyloxy-
-N'''-(2-chlor-benzolsulfonyl)-,
-N'''-(2-isopropoxy-benzolsulfonyl)-,
-N'''-(2-phenoxy-benzolsulfonyl)-,
-N'''-(2-methylsulfonyl-benzolsulfonyl)- und
-N'''-(2-cyclopropyloxycarbonyl-benzolsulfonyl)-guanidin;

ferner

N'-(4-Dimethylamino-6-methyl-pyrimidin-2-yl)-N''-
-isopropoxy-N'''-(2-chlor-benzolsulfonyl)-,
-N'''-(2-chlordifluormethyl-benzolsulfonyl)-,
-N'''-(2-N-methoxy-N-methyl-aminosulfonyl-benzol-
sulfonyl)-,
-N'''-(2-phenyl-benzolsulfonyl)- und
-N'''-(2-isopropylthio-benzolsulfonyl)-guanidin;

ferner

N'-(4-Difluormethoxy-6-methyl-pyrimidin-2-yl)-N''-
-methoxycarbonyl-N'''-(2-methyl-benzolsulfonyl),-
-N'''-(2-diethylaminosulfonyl-benzolsulfonyl)-,
-N'''-(2-difluormethoxy-benzolsulfonyl)-,
-N'''-(2-nitro-benzolsulfonyl)-,
-N'''-(2-methylthio-benzolsulfonyl)- und
-N'''-(2-ethoxycarbonyl-benzolsulfonyl)-guanidin.

Die Verbindungen der Formel (IE) können nach den oben unter (a) und (b) beschriebenen Verfahren hergestellt werden.

Die bei der Verfahrensvariante (c) weiter als Ausgangsstoffe zu verwendenden Sulfonsäurechloride sind durch die Formel (V) allgemein definiert. In Formel (V) hat $R^{11}$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Beispiele für die Ausgangsstoffe der Formel (V) können die Sulfonsäurechloride gelten, die oben als Beispiele für Verbindungen der Formel (II) genannt wurden.

Die Sulfonsäurechloride der Formel (V) sind bekannt und/oder können nach an sich bekannten Verfahren hergestellt werden (vergl. Literatur und Synthesewege

für die Verbindungen der Formel (III) - oben).

Die als Ausgangsstoffe für die Verfahrensvariante (d) zu verwendenden Sulfonylguanidinopyrimidin-Derivate sind durch die Formel (VI) allgemein definiert. In Formel (VI) haben $R^3$, $R^4$, $R^5$, $R^6$ und $R^{11}$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (VI) seien genannt:

N'-(4-Methyl-pyrimidin-2-yl)-,
N'-(4-Methoxy-6-methyl-pyrimidin-2-yl)-,
N'-(4-Ethoxy-6-methyl-pyrimidin-2-yl)-,
N'-(4-Chlor-6-methoxy-pyrimidin-2-yl)-,
N'-(4-Chlor-6-ethoxy-pyrimidin-2-yl)-,
N'-(4-Chlor-6-dimethylamino-pyrimidin-2-yl)-,
N'-(4-Methyl-6-methylthio-pyrimidin-2-yl)-,
N'-(4-Dimethylamino-6-methyl-pyrimidin-2-yl)-,
N'-(4,6-Dimethoxy-pyrimidin-2-yl)- und
N'-(4-Difluormethoxy-6-methyl-pyrimidin-2-yl)-
-N''-methoxy-,
-N''-ethoxy-,
-N''-isopropoxy-,

-N''-propoxy-,
-N''-butoxy-,
-N''-isobutoxy-,
-N''-sec.-butoxy-,
-N''-pentyloxy-,
-N''-octyloxy-,
-N''-allyloxy-,
-N''-(3-chlor-propoxy)-,
-N''-methoxycarbonylmethoxy-,
-N''-ethoxycarbonylmethoxy-,
-N''-(2-phenyl-ethoxy)-,
-N''-benzyloxy-,
-N''-(4-methyl-benzyloxy)-,
-N''-(4-fluor-benzyloxy)-,
-N''-(4-chlor-benzyloxy)-,
-N''-(4-nitro-benzyloxy)-
-N''-(4-methoxycarbonyl-benzyloxy)-,
-N''-benzyolsulfonyl-,
-N''-(2-chlor-benzolsulfonyl)-,
-N''-(3-chlor-benzolsulfonyl)-,
-N''-(4-chlor-benzolsulfonyl)-,
-N''-(2-fluor-benzolsulfonyl)-,
-N''-(4-fluor-benzolsulfonyl)-,
-N''-(2-brom-benzolsulfonyl)-,
-N''-(2-cyano-benzolsulfonyl)-,
-N''-(2-nitro-benzolsulfonyl)-,
-N''-(4-nitro-benzolsulfonyl)-,
-N''-(2-methyl-benzolsulfonyl)-,
-N''-(4-methyl-benzolsulfonyl)-,
-N''-(2-trifluormethyl-benzolsulfonyl)-,
-N''-(2-methoxy-benzolsulfonyl)-,

-N''-(4-methoxy-benzolsulfonyl)-,
-N''-(2-methylthio-benzolsulfonyl)-,
-N''-(2-difluormethoxy-benzolsulfonyl)-,
-N''-(2-trifluormethoxy-benzolsulfonyl)-,
-N''-(2-methylthiomethyl-benzolsulfonyl)-,
-N''-(2-dimethylaminosulfonyl-benzolsulfonyl)-,
-N''-(2-phenyl-benzolsulfonyl)-,
-N''-(2-methoxysulfonyl-benzolsulfonyl)-,
-N''-(2-methoxycarbonyl-benzolsulfonyl)-,
-N''-(2-ethoxycarbonyl-benzolsulfonyl)-,
-N''-(2-propoxycarbonyl-benzolsulfonyl)-,
-N''-(2-methyl-methoxy-amino-carbonyl-benzolsulfonyl)-,
-N''-(2-dimethylamino-carbonyl-benzolsulfonyl)- und
-N''-(2-diethylamino-carbonyl-benzolsulfonyl)-guanidin.

Die Sulfonylguanidinopyrimidin-Derivate der Formel (VI) sind neu und können analog Verfahren (a) durch Umsetzung von Guanidinopyrimidin-Derivaten der Formel (II) mit etwa äquimolaren Mengen von Sulfonsäurechloriden der Formel (V) hergestellt werden.

Die bei der Verfahrensvariante (d) weiter als Ausgangsstoffe zu verwendenden Sulfonsäurechloride sind durch die Formel (III) allgemein definiert. In Formel (III) hat $R^1$ vorzugsweise bzw. insbesondere die gleiche Bedeutung, wie sie oben im Rahmen der Substituentendefinition der Formel (I) vorzugsweise bzw. als insbesondere bevorzugt genannt ist.

Beispiele für die Sulfonsäurechloride der Formel (III) und ihre Herstellungsverfahren hierfür wurden bereits weiter oben im Zusammenhang mit der Beschreibung der Ausgangsstoffe für Verfahren (a) angegeben.

Die bei der Verfahrensvariante (e) als Ausgangsstoffe zu verwendenden Sulfonylisothioureidopyrimidin-Derivate sind durch die Formel (VII) allgemein definiert. In Formel (VII) haben $R^1$, $R^4$, $R^5$ und $R^6$ vorzugsweise bzw. insbesondere die gleichen Bedeutungen, wie sie oben im Rahmen der Substituentendefinition der Formel (I) vorzugsweise bzw. als insbesondere bevorzugt genannt sind und $R^{17}$ steht vorzugsweise wie auch insbesondere für Methyl.

Als Beispiele für die Verbindungen der Formel (VII) seien genannt:

N'-(4-Chlor-6-methylthio-pyrimidin-2-yl)-,
N'-(4-Methyl-pyrimidin-2-yl)-,
N'-(4-Methoxy-6-methyl-pyrimidin-2-yl)-,
N'-(4-Ethoxy-6-methyl-pyrimidin-2-yl)-,
N'-(4-Chlor-6-methoxy-pyrimidin-2-yl)-,
N'-(4-Chlor-6-ethoxy-pyrimidin-2-yl)-,
N'-(4-Chlor-6-dimethylamino-pyrimidin-2-yl)-,
N'-(4-Methyl-6-methylthio-pyrimidin-2-yl)-,
N'-(4-Dimethylamino-6-methyl-pyrimidin-2-yl)-,
N'-(4,6-Dimethoxy-pyrimidin-2-yl)- und

N'-(4-Difluormethoxy-6-methyl-pyrimidin-2-yl)-,
-N''-(2-fluor-benzolsulfonyl)-,
-N''-(2-chlor-benzolsulfonyl)-,
-N''-(2-brom-benzolsulfonyl)-,
-N''-(2-methyl-benzolsulfonyl)-,
-N''-(2-methoxycarbonyl-benzolsulfonyl)-,
-N''-(2-ethoxycarbonyl-benzolsulfonyl)-,
-N''-(2-propoxycarbonyl-benzolsulfonyl)-,
-N''-(2-isopropoxycarbonyl-benzolsulfonyl)-,
-N''-(2-phenyl-benzolsulfonyl)-,
-N''-(2-trifluormethyl-benzolsulfonyl)-,
-N''-(2-difluormethoxy-benzolsulfonyl)- und
-N''-(2-trifluormethoxy-benzolsulfonyl)-S-methyl-isothioharnstoff.

Die Sulfonylisothioureidopyrimidin-Derivate der Formel (VII) sind bekannt und/oder können nach an sich bekannten Verfahren hergestellt werden (vergl. EP-OS 5986).- Verbindungen der Formel (VII), in welcher $R^1$ für einen ausschließlich in ortho-Position durch $C_1$-$C_4$-Alkylthio, $C_1$-$C_4$-Alkylsulfinyl, $C_1$-$C_4$-Alkylsulfonyl oder $C_1$-$C_4$-Fluoralkoxy substituierten Phenylrest steht und $R^4$, $R^5$, $R^6$ und $R^{17}$ die oben angegebene Bedeutung haben, sind noch nicht bekannt (diese neuen Verbindungen werden in Anspruch 8 durch Formel (VII A) bezeichnet).

Man erhält die Verbindungen der Formel (VII), wenn man N-Sulfonyl-imino-dithiokohlensäureester der Formel (XV)

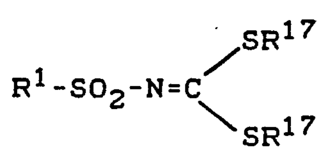

(XV)

in welcher

$R^1$ und $R^{17}$ die oben angegebenen Bedeutungen haben,

mit Aminopyrimidinen der Formel (XI)

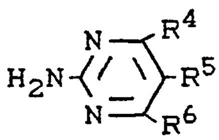

(XI)

in welcher

$R^4$, $R^5$ und $R^6$ die oben angegebenen Bedeutungen haben,

gegebenenfalls in Gegenwart von Basen, wie z. B. Natriumhydrid oder Kalium-tert.-butylat, und gegebenenfalls in Gegenwart von Verdünnungsmitteln, wie z. B. Tetrahydrofuran, Dioxan oder Dimethylformamid, bei Temperaturen zwischen 0 °C und 100 °C umsetzt, nach Reaktionsende mit Wasser verdünnt, mit einer starken Säure, wie z. B. Salzsäure, ansäuert und die kristallin anfallenden Produkte der Formel (VII) durch Absaugen isoliert.

Die hierbei als Ausgangsstoffe benötigten N-Sulfonyl--iminodithiokohlensäureester sind durch die Formel (XV) allgemein definiert. In Formel (XV) haben $R^1$ und $R^{17}$ vorzugsweise bzw. insbesondere die gleichen Bedeutungen, wie sie oben im Rahmen der Substituentendefinition für Formel (I) bzw. Formel (VII) vorzugsweise bzw. als insbesonder gekennzeichnet angegeben sind.

Als Beispiele für die Verbindungen der Formel (XV) seien genannt:

N-(2-Fluor-benzolsulfonyl-,
N-(2-Chlor-benzolsulfonyl-,
N-(2-Brom-benzolsulfonyl)-,
N-(2-Methyl-benzolsulfonyl)-,
N-(2-Methoxycarbonyl-benzolsulfonyl)-,
N-(2-Ethoxycarbonyl-benzolsulfonyl)-,
N-(2-Propoxycarbonyl-benzolsulfonyl)-,
N-(2-Isopropoxycarbonyl-benzolsulfonyl)-,
N-(2-Phenyl-benzolsulfonyl)-,
N-(2-Trifluormethyl-benzolsulfonyl)-,
N-(2-Difluormethoxy-benzolsulfonyl)-,
N-(2-Trifluormethoxy-benzolsulfonyl)-,
N-(2-Trifluormethylthio-benzolsulfonyl)-,
N-(2-Difluormethylthio-benzolsulfonyl)-,
N-(2-Phenoxy-benzolsulfonyl)-,
N-(2-Cyclopropyloxycarbonyl-benzolsulfonyl)-,
N-(2-Methylthio-benzolsulfonyl)-,
N-(2-Isopropylthio-benzolsulfonyl)-,
N-(2-Trifluormethylsulfonyl-benzolsulfonyl)-,
N-(2-Dimethylaminosulfonyl-benzolsulfonyl)-,
N-(2-N-Methoxy-N-methylaminosulfony-benzolsulfonyl)-,
N-(2-Cyano-benzolsulfonyl)-,
N-(2-Nitro-benzolsulfonyl)-,
N-(2-Dimethylaminocarbonyl-benzolsulfonyl)- und
N-(2-Methylsulfonylmethyl-benzolsulfonyl)-
-S,S-dimethyl-iminodithiokohlensäureester.

Die N-Sulfonyl-imino-dithiokohlensäureester der Formel (XV) sind bekannt und/oder können nach an sich bekannten Verfahren hergestellt werden (vergl. Chem. Ber. 99 (1966), 2855 und EP-OS 5 986).

Man erhält die Verbindungen der Formel (XV), wenn man Sulfonsäureamide der Formel (XVI)

$$R^1-SO_2-NH_2 \qquad (XVI)$$

in welcher

$R^1$ die oben angegebenen Bedeutungen hat,

mit Schwefelkohlenstoff in Gegenwart einer starken Base, wie z. B. Natriumhydroxid, und gegebenenfalls in Gegenwart von Verdünnungsmitteln, wie z. B. Wasser und Dimethylformamid, bei Temperaturen zwischen 0 °C und 100 °C umsetzt und anschließend (in situ) mit einem Alkylierungsmittel der Formel (XVII)

$$X-R^{17} \qquad (XVII)$$

in welcher

$R^{17}$ die oben angegebene Bedeutung hat und

X für Chlor, Brom oder Iod steht,

bei Temperaturen zwischen 0 °C und 100 °C umsetzt.

Die nach Verdünnen mit Wasser kristallin anfallenden Produkte der Formel (XV) können durch Absaugen isoliert werden.

Die als Ausgangsstoffe benötigten Sulfonsäureamide sind durch die Formel (XVI) allgemein definiert. In Formel (XVI) hat $R^1$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Beispiele fü die Verbindungen der Formel (XVI) seien genannt:

2-Trifluormethylthio-, 2-Difluormethylthio-, 2-Phenoxy-, 2-Cyclopropyloxycarbonyl-, 2-Methylthio-, 2-Isopropylthio-, 2-Trifluormethylsulfonyl-, 2-Dimethylaminocarbonyl-, 2-Dimethylaminosulfonyl-, 2-Cyano-, 2-Fluor-, 2-Chlor-, 2-Brom-, 2-Methyl-, 2-Methoxycarbonyl-,

2-Ethoxycarbony-, 2-Propoxycarbonyl-, 2-Isopropoxycarbonyl-, 2-Phenyl-, 2-Trifluormethyl-, 2-Difluormethoxy-, 2-Trifluormethoxy-, 2-N-Methoxy-N-methylaminosulfonyl-, 2-Nitro- und 2-Methylsulfonylmethyl-benzolsulfonamid.

Die Sulfonsäureamide der Formel (XVI) sind bekannt und/oder können nach an sich bekannten Verfahren hergestellt werden (vergl. EP-OS 23 422, 30 140, 35 893, 44 807, 44 808, 44 809, 51 466, 64 804, 70 041 und 70 802; US-PS 4 372 778 und Zh. Org. Khim. [J. Org. Chem. USSR] 8 (1972), 1023 - 1026 [engl. 1032 - 1034]).

Man erhält diese Verbindungen auf an sich bekannte Weise durch Umsetzung von Sulfonsäurechloriden der Formel (III) - oben - mit Ammoniak, gegebenenfalls in Gegenwart von Verdünnungsmitteln, wie z. B. Diethylether, Tetrahydrofuran oder auch Wasser, bei Temperaturen zwischen 0 °C und 100 °C. Die hierbei kristallin anfallenden Produkte der Formel (XVI) können durch Absaugen isoliert werden.

Beispiele für geeignete Ausgangsstoffe der Formel (III) und Herstellungsmethoden hierfür sind oben bei der Beschreibung der Ausgangsstoffe für Verfahren (a) aufgeführt.

Als Beispiele für die Ausgangsstoffe der Formel (XVII) seien genannt:

Methylchlorid, Methylbromid, Methyliodid, Ethylchlorid, Ethylbromid und Ethyliodid sowie Benzylchlorid und Benzylbromid.

Die Verbindungen der Formel (XVII) sind bekannt.

Die weiter als Ausgangsstoffe zu verwendenden Aminopyrimidine sind durch die Formel (XI) allgemein definiert. In Formel (XI) haben $R^4$, $R^5$ und $R^6$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (XI) seien genannt:

4-Chlor-6-methoxy-, 4-Chlor-6-ethoxy-, 4-Chlor-6-dimethylamino-, 4-Methyl-6-methylthio-, 4-Dimethylamino-6--methyl-, 4-Difluormethoxy-6-methyl-, 4,6-Dimethoxy-, 4-Methoxy-6-methyl-, 4-Ethoxy-6-methyl- und 4-Methyl--2-amino-pyrimidin.

Die Aminopyrimidine der Formel (XI) sind bekannt und/ oder können nach an sich bekannten Verfahren hergestellt werden (vergl. J. Chem. Soc. 1946, 81; Chem. Pharm. Bull. 11 (1963), 1382 - 1388, US-PS 4 299 960; EP-OS 19 811; AU-OS 8 316 181 und EP-OS 70 804).

Die bei der Verfahrensvariante (e) weiter als Ausgangsstoffe zu verwenden Aminoverbindungen sind durch die Formel (IV) allgemein definiert. In Formel (IV) steht $R^2$ vorzugsweise für Wasserstoff oder Methyl, insbesondere für Wasserstoff, und $R^3$ hat vorzugsweise bzw. insbesondere die gleiche Bedeutung, wie sie oben im Rahmen der Substituentendefinition für Formel (I) vorzugsweise bzw. als insbesondere bevorzugt angegeben ist.

Beispiele für geeignete Ausgangsstoffe der Formel (IV) und Literaturangaben hierzu sind oben bei der Beschreibung der Ausgangsstoffe für Verfahren (b) aufgeführt.

Die bei der Verfahrensvariante (f) als Ausgangsstoffe zu verwendenden Verbindungen sind durch die Formel (I) - mit der Maßgabe, daß M für Wasserstoff steht - allgemein definiert. In Formel (I) - soweit sie die als Ausgangsstoffe für Verfahren (f) zu verwendenden Verbindungen betrifft - steht M für Wasserstoff und $R^1$, $R^2$, $R^3$, $R^4$, $R^5$ und $R^6$ haben vorzugsweise bzw. insbesondere die gleichen Bedeutungen haben, wie sie oben im Rahmen

der Substituentendefinition für Formel (I) vorzugsweise bzw. als insbesondere bevorzugt angegeben sind.

Die als Ausgangsstoffe für Verfahren (f) zu verwendenden Verbindungen der Formel (I) können nach den unter (a), (b), (c), (d) und (e) beschriebenen Verfahren hergestellt werden.

Als Beispiele für die bei Verfahren (f) zu verwendenden Metallhydroxide, -hydride, -alkanolate bzw. metallorganischen Verbindungen seien genannt:

Lithium-, Natrium-, Kalium-, Magnesium- und Calcium-hydroxid, Lithium-, Natrium- und Calcium-hydrid, Natrium-methanolat und -ethanolat, Kalium-methanolat, -ethanolat und Kalium-ter.-butanolat sowie Butyllithium und Isopropylmagnesiumchlorid.

Die bei der Verfahrensvariante (g) als Ausgangsstoffe zu verwendenden Verbindungen sind durch die Formel (I) - mit der Maßgabe, daß M für Wasserstoff steht - allgemein definiert. In Formel (I) soweit sie die als Ausgangsstoffe für Verfahren (g) zu verwendenden Verbindungen betrifft - steht M für Wasserstoff und $R^1$, $R^2$, $R^3$, $R^4$, $R^5$ und $R^6$ haben vorzugsweise bzw. insbesondere die gleichen Bedeutungen, wie sie oben im Rahmen der Substituentendefinition für Formel (I) vorzugsweise bzw. als insbesondere bevorzugt angegeben sind.

Die als Ausgangsstoffe für Verfahren (g) zu verwendenden Verbindungen der Formel (I) können nach den unter (a), (b), (c), (d) und (e) beschriebenen Verfahren hergestellt werden. Bei Verfahren (g) werden starke Säuren als Ausgangsstoffe eingesetzt. Vorzugsweise sind dies Halogenwasserstoffsäuren, wie Hydrogenchlorid, Hydrogenbromid oder Hydrogeniodid, weiter Schwefelsäure oder gegebenenfalls durch Fluor oder Chlor substituierte Alkansulfonsäuren mit bis zu 4 Kohlenstoffatomen, wie z. B. Methansulfonsäure, Ethansulfonsäure, Chlormethansulfonsäure, 2-Chlorethansulfonsäure und Trifluormethansulfonsäure, Trifluoressigsäure, ferner Benzolsulfonsäure, p-Toluolsulfonsäure, Naphthalin-1-sulfonsäure, Naphthalin-2-sulfonsäure, Naphthalin-1,4-, -1,5-, -1,6-, 2,6- und -2,7-disulfonsäure.

Das erfindungsgemäße Verfahren (a) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls substituierte Kohlenwasserstoffe, wie z. B. Methylenchlorid, Chloroform, 1,2-Dichlorethan, Toluol, Xylol und Chlorbenzol, Nitrile, wie z. B. Acetonitril und Propionitril, Ether, wie z. B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei Verfahren (a) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylamino-pyridin.

Die Reaktionstemperaturen können bei Verfahren (a) innerhalb eines größeren Bereiches variiert werden. Im allgemeinen arbeitet man zwischen -80 °C und +100 °C, vorzugsweise zeischen -30 °C und +50 °C. Das erfindungsgemäße Verfahren (a) wird im allgemeinen bei Normaldruck durchgeführt.

Zur Durchführung von Verfahren (a) setzt man je Mol Guanidinopyrimidin-Derivat der Formel (II) im allgemeinen zwischen 1 und 5 Mol, vorzugsweise zwischen 2 und 3 Mol Sulfonsäurechlorid der Formel (III) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden: Gegebenenfalls nach Abdestillieren flüchtiger Komponenten wird mit Wasser und einem mit Wasser nicht mischbaren Lösungsmittel, wie z. B. Methylenchlorid, Chloroform oder Toluol, geschüttelt, die organische Phase mit Wasser gewaschen, getrocknet, filtriert und eingeengt. Die im Rückstand verbleibenden Produkte der Formel (I) werden durch Digerieren mit organischen Lösungsmitteln, wie z. B. Diethylether, Essigester, Ethanol oder Isopropanol zur Kristallisation gebracht und gegebenenfalls durch Umkristallisieren gereinigt.

Das erfindungsgemäße Verfahren (b) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n- und i-Propanol, Ether, wie Tetrahydrofuran, Dioxan und 1,2-Dimethoxyethan, Ester, wie Essigsäuremethylester und -ethylester, Nitrile, wie z. B. Acetonitril oder Propionitril, sowie Dimethylformamid und Wasser.

Als Säureakzeptoren können bei Verfahren (b) Säurebindemittel eingesetzt werden, welche in ihren nucleophilen Eigenschaften nicht nennenswert mit den Aminoverbindungen der Formel (IV) konkurrieren. Als solche seien Alkalimetall- und Erdalkalimetallcarbonate, wie z. B. Kaliumcarbonat und Calciumcarbonat, tertiäre Amine, wie z. B. Triethylamin, N,N-Dimethylanilin und

N,N-Dimethylbenzylamin, sowie Stickstoffheterocyclen, wie z. B. Pyridin, Diazabicyclooctan (DABCO) und Diazabicycloundecen (DBU) genannt.

Die Reaktionstemperatur kann bei Verfahren (b) innerhalb eines größeren Bereiches variiert werden. Im allgemeinen arbeitet man zwischen 0 °C und 150 °C, vorzugsweise zwischen 10 °C und 100 °C. Verfahren (b) wird im allgemeinen bei Normaldruck durchgeführt.

Zur Durchführung des erfindungsgemäßen Verfahrens (b) setzt man je Mol Verbindung der Formel (ID) im allgemeinen zwischen 1 und 10 Mol, vorzugsweise zwischen 2 und 5 Mol Aminoverbindung der Formel (IV) oder deren Hydrochlorid ein.

Im allgemeinen wird die Verbindung der Formel (ID) mit dem Verdünnungsmittel bei 20 °C oder unter leichtem Kühlen und die Aminoverbindung der Formel (IV) bzw. das Hydrochlorid hiervon und gegebenenfalls ein geeigneter Säureakzeptor dazugegeben. Das Reaktionsgemisch wird dann im allgemeinen bei Raumtemperatur oder erhöhter Temperatur bis zum Reaktionsende gerührt.

Die Aufarbeitung kann nach üblichen Methoden duchgeführt werden. Soweit die Produkte der Formel (I) aus dem Reaktionsgemisch kristallin anfallen, können sie durch Absaugen isoliert werden. Anderenfalls wird - gegebenen

falls nach Einengen - mit Wasser verdünnt und mit einem mit Wasser praktisch nicht mischbaren Lösungsmittel, wie z. B. Methylenchlorid, extrahiert. Durch Waschen der Extraktionslösung mit Wasser, Trocknen, Filtrieren, Einengen des Filtrates und Umkristallisieren des Rückstandes können die Produkte der Formel (I) in reiner Form erhalten werden.

Das erfindungsgemäße Verfahren (c) wird vorzugsweise unter Verwendung von Verdünnungsmitteln durchgeführt. Als solche kommen praktisch alle inerten oranischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (c) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (c) innerhalb eines größeren Bereichs variiert wrden. Im allgemeinen arbeitet man zwischen -80 °C und +100 °C, vorzugsweise zwischen -30 °C und +50 °C. Das Verfahren (c) wird im allgemeinen bei Normaldruck durchgeführt.

Zur Durchführung von Verfahren (c) setzt man je Mol Sulfonylguanidinopyrimidin-Derivat der Formel (IE) im allgemeinen zwischen 1 und 2 Mol, vorzugsweise zwischen 1,1 und 1,5 Mol Sulfonsäurechlorid der Formel (V) ein.

Die Durchführung und Aufarbeitung kann bei Verfahren (c) analog zu Verfahren (a) erfolgen.

Das erfindungsgemäße Verfahren (d) wird vorzugweise unter Verwendung von Verdünnungsmitteln durchgeführt. Als solche kommen praktisch alle inerten organischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (d) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (d) innerhalb eines größeren Bereichs variiert werden. Im allgemeinen arbeitet man zwischen -80 °C und +100 °C, vorzugsweise zwischen -30 °C und +50 °C. Das Verfahren (d) wird im allgemeinen bei Normaldruck durchgeführt.

Zur Durchführung von Verfahren (d) setzt man je Mol Sulfonylguanidinopyrimidin-Derivat der Formel (VI) im allgemeinen zwischen 1 und 2 Mol, vorzugsweise zwischen 1,1 und 1,5 Mol Sulfonsäurechlorid der Formel (III) ein.

Die Durchführung und Aufarbeitung kann bei Verfahren (d) analog zu Verfahren (a) erfolgen.

Das erfindungsgemäße Verfahren (e) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören insbesondere Tetrahydrofuran, Dioxan, 1,2-Dimethoxyethan, Acetonitril, Aceton, Methylethylketon, Methylisobutylketon, Dimethylformamid, Dimethylacetamid und Dimethylsulfoxid.

Die Reaktionstemperatur kann bei Verfahren (e) innerhalb eines größeren Bereichs variiert werden. Im allgemeinen arbeitet man zwischen 0 °C und 150 °C, vorzugsweise zwischen 10 °C und 100 °C. Das Verfahren (e) wird im allgemeinen bei Normaldruck oder geringfügig vermindertem Druck durchgeführt.

Zur Durchführung des erfindungsgemäßen Verfahrens (e) setzt man je Mol Sulfonylisothioureidopyrimidin-Derivat

der Formel (VII) im allgemeinen zwischen 1 und 10 Mol, vorzugsweise zwischen 1 und 5 Mol Aminoverbindung der Formel (IV) ein.

Im allgemeinen werden die Sulfonylisothioureidopyrimidin-Derivate der Formel (VII) und das Verdünnungsmittel vorgelegt und die Aminoverbindung der Formel (IV) wird zudosiert. Das Reaktionsgemisch wird dann bis zum Reaktionsende gerührt. Die Produkte der Formel (I) fallen gewöhnlich nach Abkühlen und gegebenenfalls nach Ansäuern, z. B. mit Salzsäure, kristallin an und können durch Absaugen isoliert werden.

Das erfindungsgemäße Verfahren (f) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z. B. Ethanol, n- und iso-Propanol, Ether, wie z. B. Tetrahydrofuran, Dioxan und 1,2-Dimethoxyethan, Ester, wie z. B. Essigsäureethylester und -methylester sowie Nitrile, wie z. B. Acetonitril.

Die Reaktionstemperatur kann bei Verfahren (f) innerhalb eines größeren Bereiches variiert werden. Im allgemeinen arbeitet man zwischen -20 °C und +50 °C, vorzugsweise zwischen 0 °C und 30 °C. Verfahren (f) wird im allgemeinen bei Normaldruck durchgeführt.

Zur Durchfühung des erfindungsgemäßen Verfahrens (f) setzt man je Mol Verbindung der Fomel (I) im allgemeinen zwischen 0,9 und 1,2 Mol, vorzugsweise zwischen 0,95 und 1,1 Mol Metallverbindung ein.

Im allgemeinen werden die Verbindungen der Formel (I) und das Verdünnungsmittel vorgelegt und - gegebenenfalls unter leichter Außenkühlung - die Metallverbindung - gegebenenfalls im Verdünnungsmittel gelöst - zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (g) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n- und iso-Propanol, Ether, wie Tetrahydrofuran, Dioxan und 1,2-Dimethoxyethan, Ester, wie Essigsäuremethylester und -ethylester sowie Ketone, wie Aceton, Methylethylketon und Methylisobutylketon.

Soweit die als Ausgangsstoffe verwendeten Säuren in wässriger Lösung eingesetzt werden, kann vorteilhaft auch Essigsäureanhydrid als Verdünnungsmittel verwendet werden.

Die Reaktionstemperatur kann bei Verfahren (g) innerhalb eines größeren Bereiches variiert werden. Im allgemeinen arbeitet man zwischen -20 °C und +50 °C, vorzugsweise zwischen 0 °C und 30 °C. Verfahren (g) wird im allgemeinen bei Normaldruck durchgeführt.

Zur Durchführung des erfindungsgemäßen Verfahrens (g) setzt man je Mol Verbindung der Formel (I) im allgemeinen zwischen 1 und 10 Mol, vorzugsweise 1 und 5 Mol einer starken Säure ein.

Im allgemeinen werden die Verbindungen der Formel (I) und das Verdünnungsmittel vorgelegt und - gegebenenfalls unter leichter Außenkühlung - die starke Säure zudosiert. Das Reaktionsgemisch wird bis zum Reaktionsende gerührt. Die 1:1-Addukte fallen im allgemeinen kristallin an und können durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur selektiven Bekämpfung mono- und dikotyler Unkräuter in mono- und dikotylen Kulturen wie z. B. Baumwolle, Reis, Getreide und Mais eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5-(4H)-on, 4-Amino-6-(1,1-dimethyl-ethyl)-3-ethylthio-1,2,4-triazin-5-(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5-(4H)-on, 2-Chlor-4-ethylamino-6-isopropylamino-1,3,5-triazin, das R-Enantiomere des 2-[4-(3,5-Dichlorpyridyl-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxyessigsäure, 2-(2-Methyl-4-chlor-phenoxy)-propionsäure, 3,5-Diiod-4-hydroxy-benzonitril, 3,5-Dibrom-4-hydroxybenzonitril sowie Diphenylether und Phenylpyridazine, wie z.B. Pyridate. Einige Mischungen zeigen überraschenderweise auch synergistische Wirkung. Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

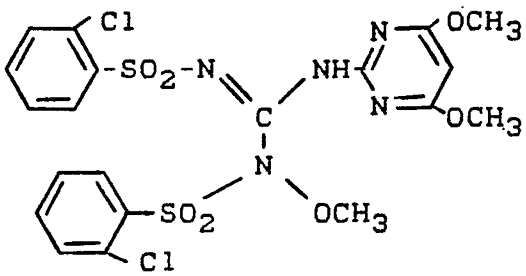

(Verfahren (a))

10,1 g (0,048 Mol) 2-Chlor-benzolsulfonsäurechlorid werden bei -10 °C zu einer Lösung von 4,5 g (0,02 Mol) N'-(4,6-Dimethoxy-pyrimidin-2-yl)-N''-methoxy-guanidin in 30 ml Pyridin gegeben. Das Reaktionsgemisch wird zwei Tage bei 20 °C gerührt. Nach Zugabe von weiteren 1,5 g 2-Chlor-benzolsulfonsäurechlorid wird das Gemisch weitere 24 Stunden bei 20 °C gerührt. Bei maximal 40 °C wird eingeengt, der Rückstand in Methylenchlorid aufgenommen und mit 5 %iger Salzsäure gewaschen. Die organische Phase wird getrocknet, filtriert und eingeengt. Der ölige Rückstand wird mit Isopropanol zur Kristallisation gebracht.

Man erhält 2,65 g (23 % der Theorie) N'-(4,6-Dimethoxy-pyrimidin-2-yl)-N''-methoxy-N'',N'''-bis-(2-chlor-benzolsulfonyl)-guanidin vom Schmelzpunkt 164 - 165 °C.

Beispiel 2

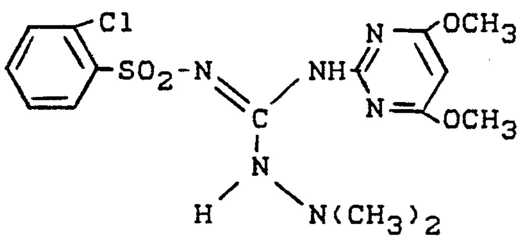

(Verfahren (b))

Eine Mischung aus 5,8 g (0,01 Mol) N'-(4,6-Dimethoxy-pyrimidin-2-yl)-N''-methoxy-N'',N'''-bis-(2-chlor-benzolsulfonyl)-guanidin, 1,5 g (0,025 Mol) N,N-Dimethylhydrazin, 20 ml Ethanol und 10 ml Wasser wird eine Stunde unter Rückfluß zum Sieden erhitzt. Das nach Erkalten kristallin angefallene Produkt wird durch Absaugen isoliert und aus Essigsäureethylester umkristallisiert.

Man erhält 1,3 g (31 % der Theorie) N'-(4,6-Dimethoxy-pyrimidin-2-yl)-N''-dimethylamino-N'''-(2-chlor-benzolsulfonyl)-guanidin vom Schmelzpunkt 190 °C.

Beispiel 3

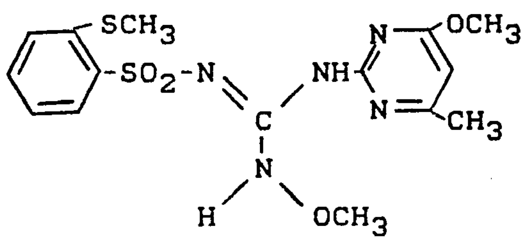

(Verfahren (e))

Eine Mischung aus 16,0 g (0,04 Mol) N'-(4-Methoxy-6-methyl-pyrimidin-2-yl)-N''-(2-methylthio-benzolsulfonyl)-S-methylisothioharnstoff, 4,0 g (0,085 Mol) O-Methyl-hydroxylamin und 80 ml Dioxan wird 15 Stunden bei 30 °C bis 33 °C gerührt. Ausschließend wird eingeengt, der Rückstand durch Verreiben mit Ethanol zur Kristallisation gebracht und das Produkt durch Absaugen isoliert.

Man erhält 8,4 g (53 % der Theorie) N'-(4-Methoxy-6-methyl-pyrimidin-2-yl)-N''-methoxy-N'''-(2-methylthio-benzolsulfonyl)-guanidin vom Schmelzpunkt 134 °C.

Beispiel 4

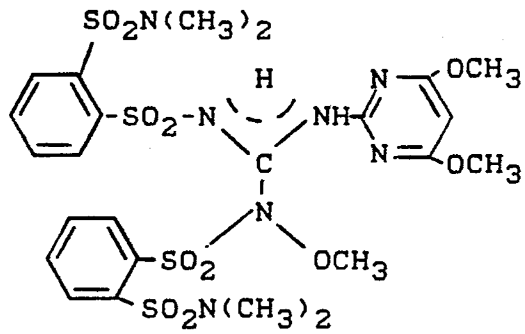

x $CH_3SO_3H$

(Verfahren (g))

Eine Mischung aus 5,4 g (0,0075 Mol) N'-(4,6-Dimethoxy--pyrimidin-2-yl)-N''-methoxy-N'',N'''-bis-(2-dimethylaminosulfonyl-benzolsulfonyl)-guanidin, 0,75 g (0,0075 Mol) Methansulfonsäure und 10 ml Aceton wird 24 Stunden bei 20 °C gerührt. Dann wird das kristallin angefallene Produkte durch Absaugen isoliert.

Man erhält 1,6 g (26 % der Theorie) des 1:1-Adduktes aus N'-(4,6-Dimethoxy-pyrimidin-2-yl)-N''-methoxy-N''-N'''--bis-(dimethylaminosulfonyl-benzolsulfonyl)-guanidin und Methansulfonsäure vom Schmelzpunkt 145 °C.

Analog erhält man:

Beispiel 5
----------

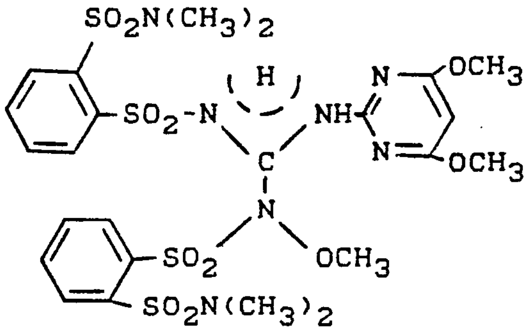

X $H_2SO_4$

Schmelzpunkt: 155 °C

Nach den in den vorausgehenden Beispielen exemplarisch beschriebenen Verfahren können die in der nachstehenden Tabelle 1 aufgeführten Verbindungen der Formel I hergestellt werden:

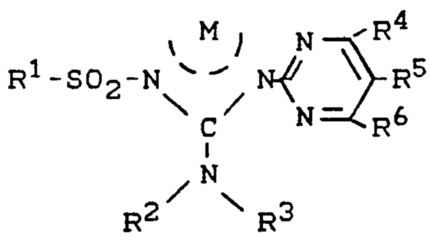

(I)

**Tabelle 1**

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 6 | 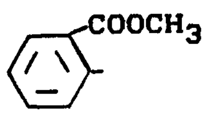 | 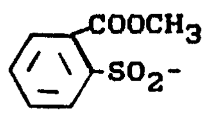 | $-OCH_3$ | $-OCH_3$ | H | $-OCH_3$ | H | 149 |
| 7 | 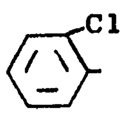 | 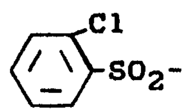 | $-OCH_3$ | $-CH_3$ | H | $-OCH_3$ | H | 172 |
| 8 | 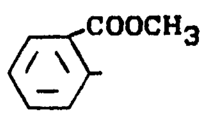 | 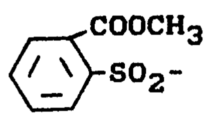 | $-OCH_3$ | $-CH_3$ | H | $-OCH_3$ | H | 152 |
| 9 | 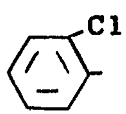 | 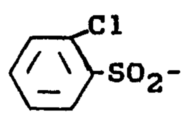 | H | $-CH_3$ | H | $-OCH_3$ | H | 155 |
| 10 | 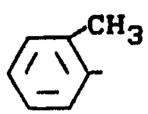 | H | $-OCH_3$ | $-CH_3$ | H | $-OCH_3$ | H | 108 |
| 11 | 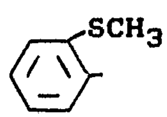 | H | $-N(CH_3)_2$ | $-CH_3$ | H | $-OCH_3$ | H | 174 |

<u>Tabelle 1</u>-Fortsetzung

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 12 | 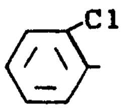 | H | $-OCH_3$ | $-OCH_3$ | H | $-OCH_3$ | H | 143 |
| 13 | 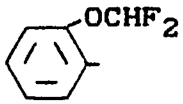 | 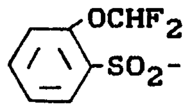 | $-OCH_3$ | $-OCH_3$ | H | $-OCH_3$ | H | 136 |
| 14 | 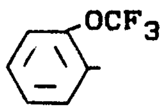 | 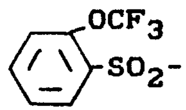 | $-OCH_3$ | $-OCH_3$ | H | $-OCH_3$ | H | 134 |
| 15 | 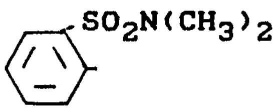 | 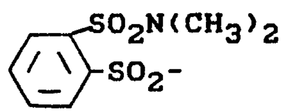 | $-OCH_3$ | $-OCH_3$ | H | $-OCH_3$ | H | 177 |
| 16 | 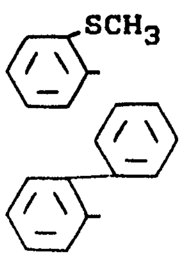 | 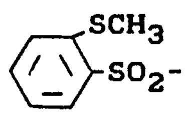 | $-OCH_3$ | $-OCH_3$ | H | $-OCH_3$ | H | |
| 17 | | 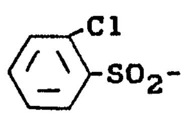 | $-OCH_3$ | $-CH_3$ | H | $-OCH_3$ | H | |

**Tabelle 1**-Fortsetzung

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 18 | 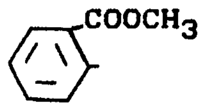 | H | $-OCH_3$ | $-OCH_3$ | H | $-OCH_3$ | H | |
| 19 | 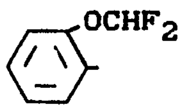 | H | $-OCH_3$ | $-OCH_3$ | H | $-OCH_3$ | H | 135 |
| 20 | 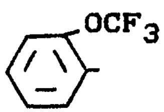 | H | $-OCH_3$ | $-OCH_3$ | H | $-OCH_3$ | H | 137 |
| 21 | 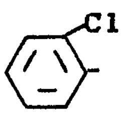 | 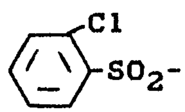 | $-OCH_3$ | $-CH_3$ | H | H | H | 140-141 |
| 22 | 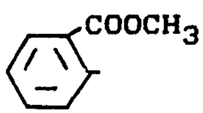 | 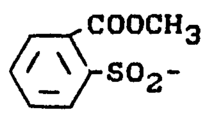 | $-OCH_3$ | $-CH_3$ | H | H | H | 154 |
| 23 | 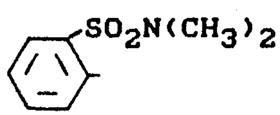 | 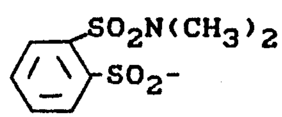 | $-OC_2H_5$ | $-CH_3$ | H | $-OCH_3$ | H | |

<u>Tabelle 1</u>-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 24 | 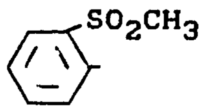 | 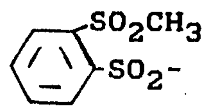 | $-OCH_3$ | $-CH_3$ | H | $-OCH_3$ | H | 112 |
| 25 | 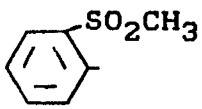 | H | $-OC_4H_9(-n)$ | $-CH_3$ | H | $-OCH_3$ | H | |
| 26 | 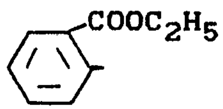 | 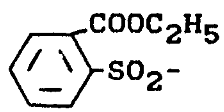 | $-OCH_3$ | $-OCH_3$ | H | $-OCH_3$ | H | |
| 27 | 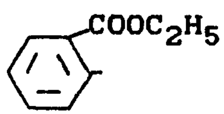 | H | $-OCH_2CH=CH_2$ | $-OCH_3$ | H | $-OCH_3$ | H | |
| 28 | 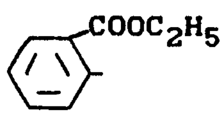 | H | $-OCH(CH_3)_2$ | $-OCH_3$ | H | $-OCH_3$ | H | |
| 29 | 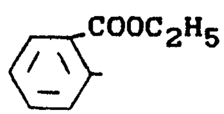 | H | $-O-CH_2-$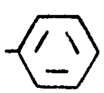 | $-OCH_3$ | H | $-OCH_3$ | H | |

**Tabelle 1**-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 30 | 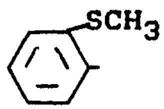 | 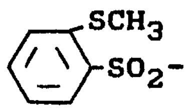 | $-OCH_3$ | $-CH_3$ | H | $-OCH_3$ | H | 178 (Zers.) |
| 31 | 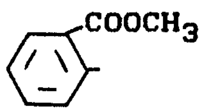 | H | $-OC_2H_5$ | $-OCH_3$ | H | $-OCH_3$ | H | |
| 32 | 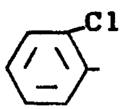 | H | 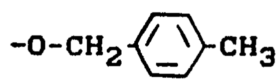 | $-OCH_3$ | H | $-OCH_3$ | H | |
| 33 | 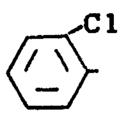 | H | $-OCH_2COOC_2H_5$ | $-CH_3$ | H | H | H | |
| 34 | 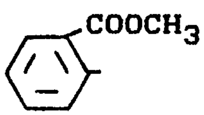 | H | $-OC_3H_7$(-n) | $-CH_3$ | H | H | H | |
| 35 | 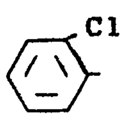 | H | $-OC_4H_9$(-n) | $-CH_3$ | H | $-OCH_3$ | H | |

Tabelle 1-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 36 | 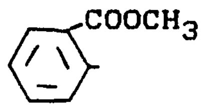 | H | $-OC_8H_{17}$(-n) | $-CH_3$ | H | $-OCH_3$ | H | |
| 37 | 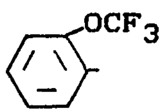 | 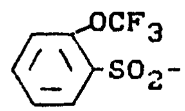 | $-OCH_3$ | $-CH_3$ | H | $-OCH_3$ | H | 194 |
| 38 | 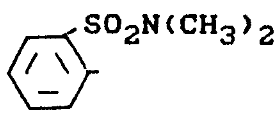 | 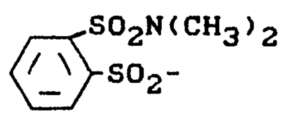 | $-OC_4H_9$(-i) | $-OCH_3$ | H | $-OCH_3$ | H | 196 |
| 39 | 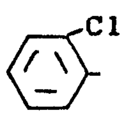 | H | $-OCH_3$ | $-CH_3$ | H | $-OCH_3$ | H | |
| 40 | 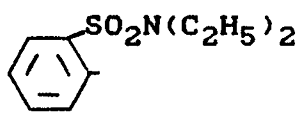 | 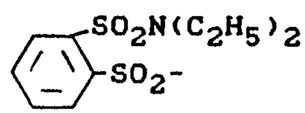 | $-OCH_3$ | $-CH_3$ | H | H | H | 137-138 |
| 41 | 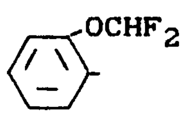 | 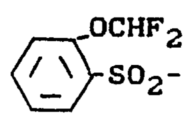 | $-OC_2H_5$ | $-CH_3$ | H | $-OC_2H_5$ | H | |

Tabelle 1-Fortsetzung

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 42 | 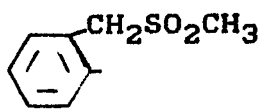 | 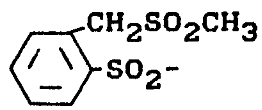 | $-OCH_3$ | -Cl | H | $-OCH_3$ | H | |
| 43 | 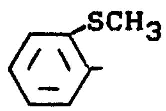 | H | $-OC_3H_7(-i)$ | -Cl | H | $-OCH_3$ | H | |
| 44 | 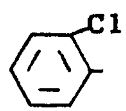 | H | $-N(CH_3)_2$ | -Cl | H | $-OCH_3$ | H | |
| 45 | 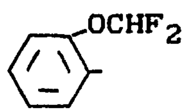 | 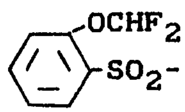 | $-OCH_3$ | $-CH_3$ | H | H | H | 163-167 |
| 46 | 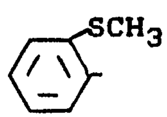 | 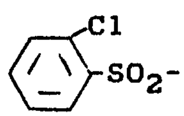 | $-OCH_3$ | -Cl | H | $-OC_2H_5$ | H | |
| 47 | 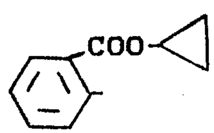 | 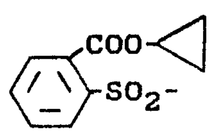 | $-OCH_2-$ 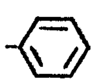 | $-CH_3$ | H | $-OCHF_2$ | H | |

Tabelle 1-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 48 | 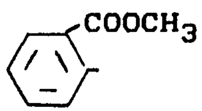 | 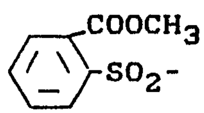 | $-OCH_3$ | $-CH_3$ | H | $-OCHF_2$ | H | |
| 49 | 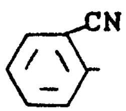 | 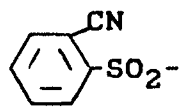 | $-OC_3H_7(-n)$ | $-CH_3$ | H | $-SCH_3$ | H | |
| 50 | 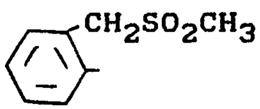 | 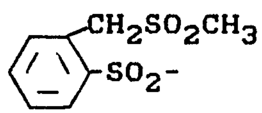 | $-OC_4H_9(-n)$ | $-CH_3$ | H | $-N(CH_3)_2$ | H | |
| 51 | 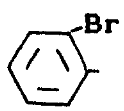 | 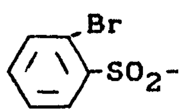 | $-OC_2H_5$ | $-OCH_3$ | H | $-OCH_3$ | H | |
| 52 | 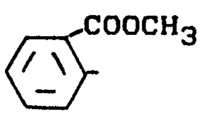 | H | $-N(CH_3)_2$ | $-CH_3$ | H | $-OCHF_2$ | H | |
| 53 | 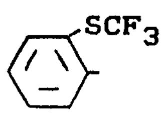 | 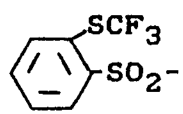 | $-OCH_3$ | $-CH_3$ | H | $-N(CH_3)_2$ | H | |

Tabelle 1-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 54 | 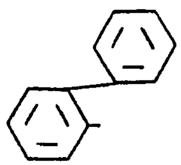 | 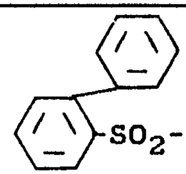 | $-OCH_2-CH=CH_2$ | $-CH_3$ | H | $-SCH_3$ | H | |
| 55 | 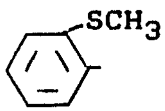 | H | $-OC_2H_5$ | $-CH_3$ | H | H | H | |
| 56 | 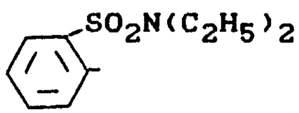 | 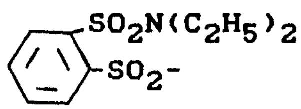 | $-OC_2H_5$ | $-CH_3$ | H | H | H | |
| 57 | 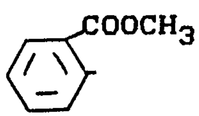 | 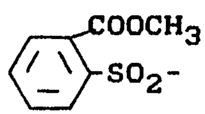 | $-OCH_2-COOC_2H_5$ | $-CH_3$ | H | H | H | 155 |
| 58 | 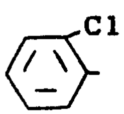 | 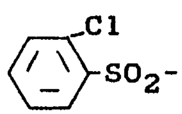 | $-OCH_2-COOC_2H_5$ | $-CH_3$ | H | H | H | 165-167 |
| 59 | 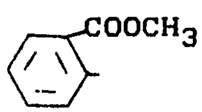 | 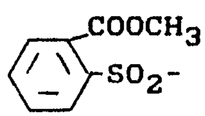 | 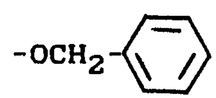 | $-CH_3$ | H | H | H | 128 |

Tabelle 1-Fortsetzung

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 60 | 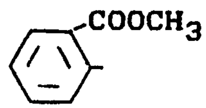 | 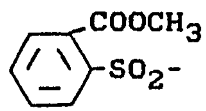 | $-OC_4H_9(-s)$ | $-OCH_3$ | H | $-OCH_3$ | H | 109 |
| 61 | 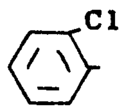 | 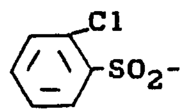 | $-OC_4H_9(-i)$ | $-OCH_3$ | H | $-OCH_3$ | H | 164 |
| 62 | 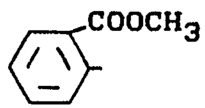 | 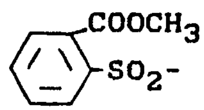 | $-OC_4H_9(-i)$ | $-OCH_3$ | H | $-OCH_3$ | H | 146 |
| 63 | 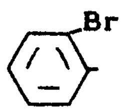 | 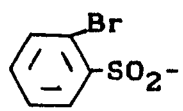 | $-OCH_3$ | $-CH_3$ | H | $-OCH_3$ | H | 174 |
| 64 | 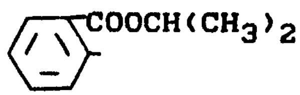 | 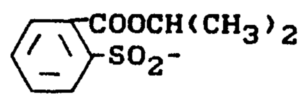 | $-OCH_3$ | $-CH_3$ | H | $-OCH_3$ | H | $n_D^{20}$ = 1.5391 |
| 65 | 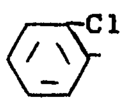 | 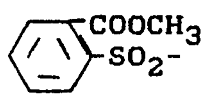 | $-OCH_3$ | $-OCH_3$ | H | $-OCH_3$ | H | |

Tabelle 1-Fortsetzung

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 66 | 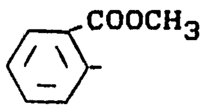 | H | $-OCH_2COOC_2H_5$ | $-CH_3$ | H | H | H | 91-93 |
| 67 | 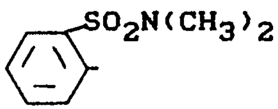 | H | 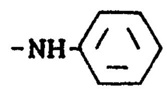 | $-OCH_3$ | H | $-OCH_3$ | H | 187 |
| 68 | 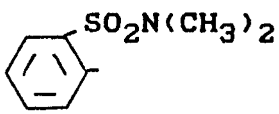 | H | $-NHCOCH_3$ | $-OCH_3$ | H | $-OCH_3$ | H | 181 |
| 69 | 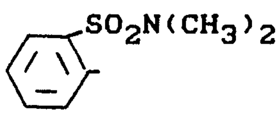 | H | $-N(CH_3)_2$ | $-OCH_3$ | H | $-OCH_3$ | H | 156 |
| 70 | 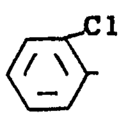 | 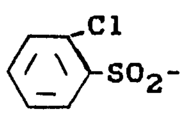 | $-OCH_3$ | H | H | H | H | 144 - 145 |
| 71 | 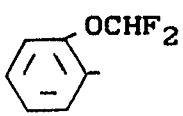 | H | $-N(CH_3)_2$ | $-OCH_3$ | H | $-OCH_3$ | H | 177 |

**Tabelle 1-Fortsetzung**

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 72 | 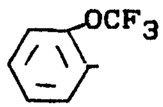 | H | $-N(CH_3)_2$ | $-OCH_3$ | H | $-OCH_3$ | H | 158 |
| 73 | 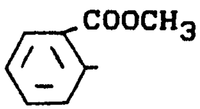 | H | $-N(CH_3)_2$ | $-OCH_3$ | H | $-OCH_3$ | H | 164-167 |
| 74 | 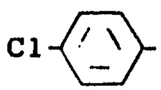 | 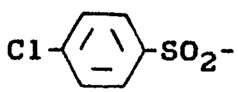 | $-OCH_3$ | H | H | $-CH_3$ | H | 148 |
| 75 | 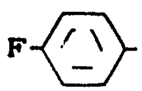 | 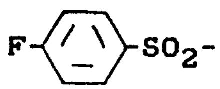 | $-OCH_3$ | H | H | $-CH_3$ | H | 135 |
| 76 | 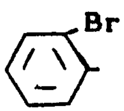 | 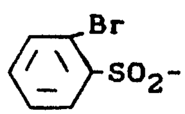 | $-OCH_3$ | H | H | $-CH_3$ | H | 163 |
| 77 | 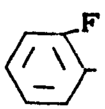 | 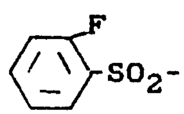 | $-OCH_3$ | H | H | $-CH_3$ | H | 160 |

Tabelle 1-Fortsetzung

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 78 | 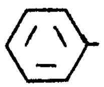 | 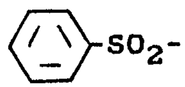 | $-OCH_3$ | H | H | $-CH_3$ | H | 118 |
| 79 | 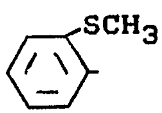 | 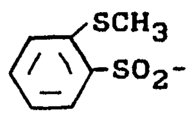 | $-OCH_3$ | H | H | $-CH_3$ | H | 159 |
| 80 | 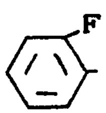 | 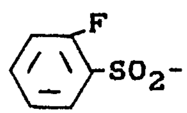 | $-OCH_3$ | $-OCH_3$ | H | $-CH_3$ | H | 174 |
| 81 | 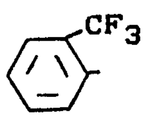 | 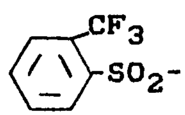 | $-OCH_3$ | $-OCH_3$ | H | $-CH_3$ | H | 165 |
| 82 | 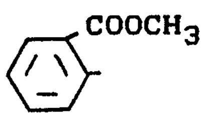 | 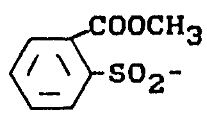 | $-OCH_2CH=CH_2$ | H | H | $-CH_3$ | H | 97 |
| 83 | 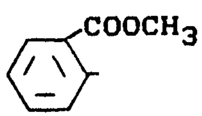 | 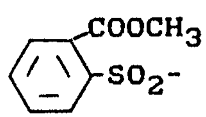 | $-OCH(CH_3)CH_2CH_3$ | H | H | $-CH_3$ | H | 130 |

Tabelle 1-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 84 | 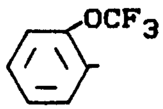 | 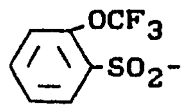 | $-OCH_3$ | H | H | $-CH_3$ | H | 170 |
| 85 | 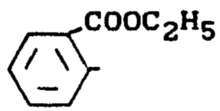 | 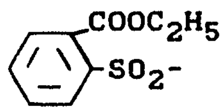 | $-OCH_3$ | H | H | $-CH_3$ | H | 168 |
| 86 | 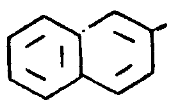 | 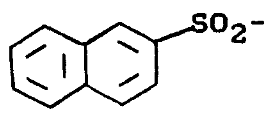 | $-OCH_3$ | H | H | $-CH_3$ | H | 107 |
| 87 | 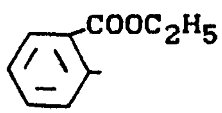 | 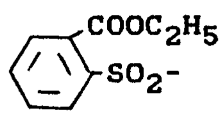 | $-OCH_3$ | H | H | $-C_2H_5$ | H | 141 |
| 88 | 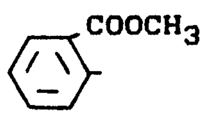 | 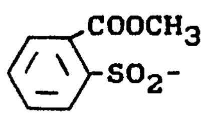 | $-OC_2H_5$ | H | H | $-CH_3$ | H | 135 |
| 89 | 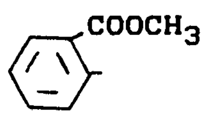 | 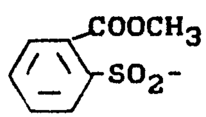 | 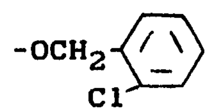 | H | H | $-CH_3$ | H | |

Tabelle 1-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 90 | 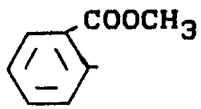 | 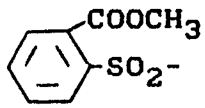 | $-OC_4H_9(-n)$ | H | H | $-CH_3$ | H | |
| 91 | 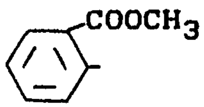 | H | 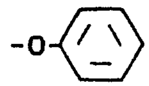 | H | H | $-CH_3$ | H | 137 |
| 92 | 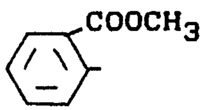 | H | $-OCH_2CH_2CH_2Cl$ | $-CH_3$ | Cl | $-CH_3$ | H | 129 |
| 93 | 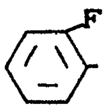 | 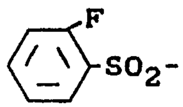 | 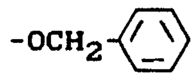 | $-CH_3$ | H | $-OCH_3$ | H | 180 |
| 94 | 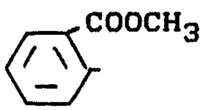 | H | 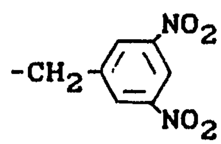 | $-CH_3$ | H | H | H | amorph |
| 95 | 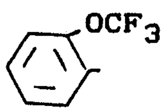 | $(n-)C_4H_9-SO_2-$ | $-OCH_3$ | H | H | $-CH_3$ | H | |

**Tabelle 1**-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 96 | 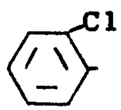 | $C_8F_{17}-SO_2-$ | $-OC_2H_5$ | H | H | $-CH_3$ | H | |
| 97 | 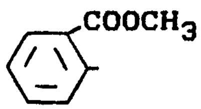 | $(CH_3)_2N-SO_2-$ | $-OCH_3$ | $-CH_3$ | H | $-OCH_3$ | H | |
| 98 | 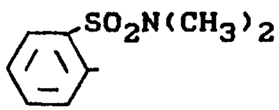 | $CH_3-SO_2-$ | $-OCH_3$ | $-OCH_3$ | H | $-OCH_3$ | H | |
| 99 | 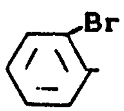 | $CF_3-SO_2-$ | $-OCH_2-$ 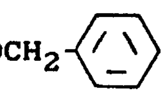 | $-CH_3$ | H | $-OCH_3$ | H | |
| 100 | 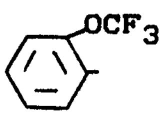 | $(CH_3)_2CHNHSO_2-$ | $-OCH_3$ | $-OCH_3$ | H | $-OCH_3$ | H | |

Tabelle 1-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 101 | 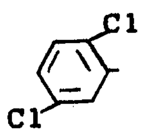 | 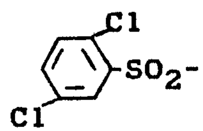 | 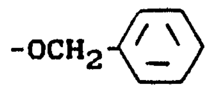 | $-CH_3$ | H | $-OCH_3$ | H | 163 |
| 102 | 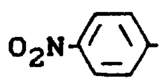 | 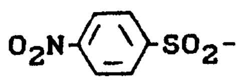 | 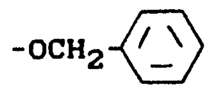 | $-CH_3$ | H | $-OCH_3$ | H | 151 |
| 103 | 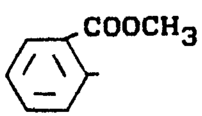 | 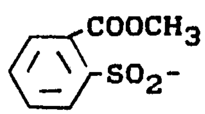 | 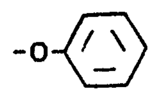 | $-CH_3$ | H | H | H | 130 |
| 104 | 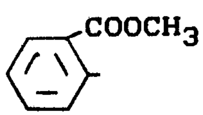 | 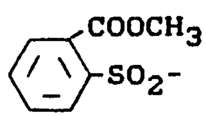 | $-OCH_3$ | Cl | H | $-OCH_3$ | H | 159 |
| 105 | 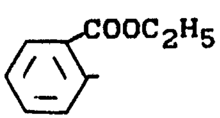 | 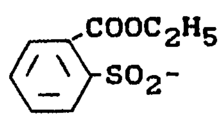 | $-OCH_3$ | Cl | H | $-OCH_3$ | H | |

Tabelle 1-Fortsetzung

| Bsp.-Nr. | $R^1$ | $R^2$ | $R^3$ | $R^4$ | $R^5$ | $R^6$ | M | Schmelzpunkt [°C] |
|---|---|---|---|---|---|---|---|---|
| 106 | 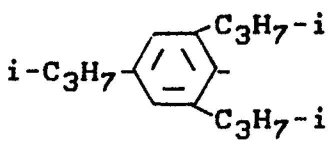 | 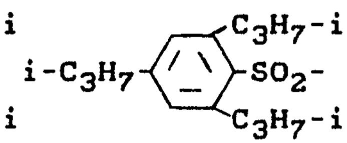 | $-OCH_3$ | $-OCH_3$ | H | $-OCH_3$ | H | 117 - 119 |
| 107 | 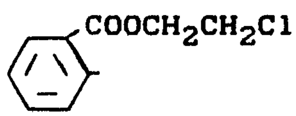 | 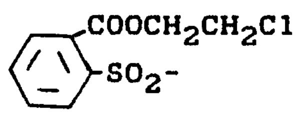 | $-OCH_3$ | $-CH_3$ | H | $-OCH_3$ | H | 138 |
| 108 | 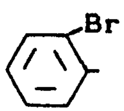 | 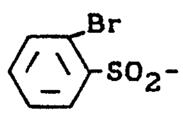 | $-OC_2H_5$ | $-CH_3$ | H | $-OCH_3$ | H | 164 |
| 109 | 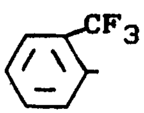 | 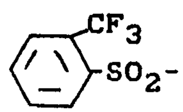 | $-OC_2H_5$ | $-CH_3$ | H | $-OCH_3$ | H | 154 |
| 110 | 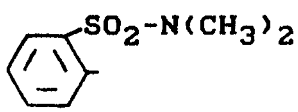 | 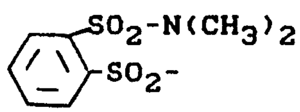 | $-OCH_3$ | $-CH_3$ | H | $-OCH_3$ | H | 141 |

**Tabelle 1-Fortsetzung**

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 111 | 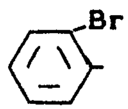 | 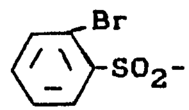 | $-OCH_3$ | $-OCH_3$ | H | $-OCH_3$ | H | 109 |
| 112 | 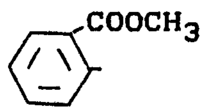 | 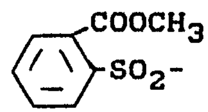 | $-OCH_3$ | H | H | H | H | 144-147 |
| 113 | 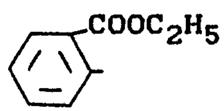 | 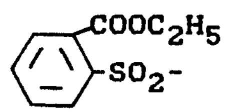 | $-OCH_2-$ 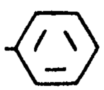 | $-CH_3$ | H | H | H | 150 - 151 |
| 114 | 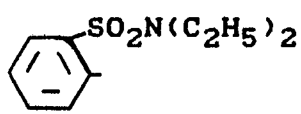 | 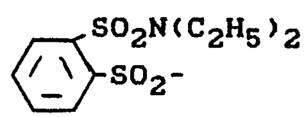 | $-OCH_2-$ 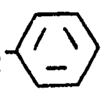 | $-CH_3$ | H | H | H | 151 - 152 |
| 115 | 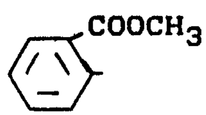 | 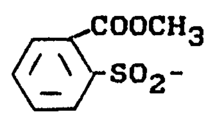 | $-OCH_2-$ 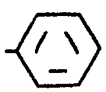 | $-C_2H_5$ | H | H | H | 148 |

Tabelle 1-Fortsetzung

| Bsp.-Nr. | $R^1$ | $R^2$ | $R^3$ | $R^4$ | $R^5$ | $R^6$ | M | Schmelz-punkt [°C] |
|---|---|---|---|---|---|---|---|---|
| 116 | 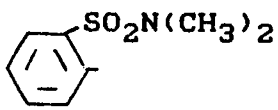 | 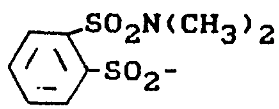 | $-OCH_3$ | $-CH_3$ | H | H | H | 114 - 116 |

7a) p-Toluolsulfonsäure-Salz von Beispiel 7, amorph.

8a) p-Toluolsulfonsäure-Salz von Beispiel 8, amorph.

8b) Schwefelsäure-Salz von Beispiel 8, amorph.

9a) p-Toluolsulfonsäure-Salz von Beispiel 9, vom Schmelzpunkt: 175 °C (Zers.)

9b) Schwefelsäure-Salz von Beispiel 9, vom Schmelzpunkt: 170 °C (Zers.)

22a) p-Toluolsulfonsäure-Salz von Beispiel 22, (Öl).

Herstellung von Ausgangsstoffen der Formel (II)

Beispiel (II-1)

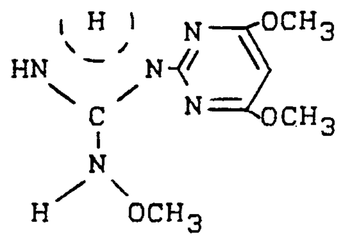

Eine Mischung aus 36,0 g (0,2 Mol) 2-Cyanamino-4,6-dimethoxy-pyrimidin, 33,4 g (0,4 Mol) O-Methyl-hydroxylamin-Hydrochlorid und 200 ml Ethanol wird 6 Stunden unter Rückfluß zum Sieden erhitzt. Nach Zugabe von weiteren 16,7 g (0,2 Mol) O-Methyl-hydroxylamin-Hydrochlorid wird das Gemisch weitere 6 Sunden unter Rückfluß erhitzt. Nach Abkühlen wird über Kieselgur filtriert, das Filtrat eingeengt und mit 200 ml Wasser verdünnt. Mit verdünnter Natronlauge wird auf pH 7 gebracht und das hierbei kristallin angefallene Produkt durch Absaugen isoliert.

Nach Umkristallisieren aus Isopropanol erhält man 16,4 g (36 % der Theorie) N'-(4,6-Dimethoxy-pyrimidin-2-yl)-N''-methoxy-guanidin vom Schmelzpunkt 122 °C.

Analog können die in der nachstehenden Tabelle 2 aufgeführten Verbindungen der Formel (II) hergestellt werden:

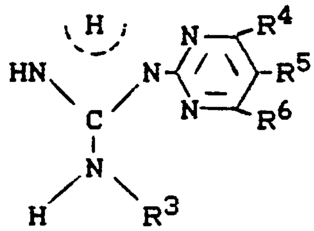

(II)

Tabelle 2

| Beisp.-Nr. | $R^3$ | $R^4$ | $R^5$ | $R^6$ | Schmelzpunkt[°C] |
|---|---|---|---|---|---|
| II-2 | $-OCH_3$ | $-CH_3$ | H | H | 152 |
| II-3 | $-OCH_3$ | $-CH_3$ | H | $-OCH_3$ | 126 |
| II-4 | $-OCH_2CH(CH_3)_2$ | $-OCH_3$ | H | $-OCH_3$ | 76 |
| II-5 | $-OCH_2CH=CH_2$ | $-OCH_3$ | H | $-OCH_3$ | |
| II-6 | $-OC_2H_5$ | $-OCH_3$ | H | $-CH_3$ | |
| II-7 | $-OCH(CH_3)_2$ | $-OCH_3$ | H | $-OCH_3$ | |
| II-8 | $-OCH_2CH_2-$ 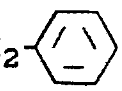 | $-OCH_3$ | H | $-OCH_3$ | |
| II-9 | $-OC_4H_9(-n)$ | $-OCH_3$ | H | $-OCH_3$ | |

<u>Tabelle 2</u>-Fortsetzung

| Beisp.-Nr. | $R^3$ | $R^4$ | $R^5$ | $R^6$ | Schmelzpunkt[°C] |
|---|---|---|---|---|---|
| II-10 | $-OC_4H_9(-n)$ | $-CH_3$ | H | $-OCH_3$ | |
| II-11 | $-OC_8H_{17}(-n)$ | $-OCH_3$ | H | $-CH_3$ | |
| II-12 | $-OCH_2CH_2CH_2Cl$ | $-OCH_3$ | H | $-OCH_3$ | |
| II-13 | $-OCH_2COOCH_3$ | $-OCH_3$ | H | $-OCH_3$ | |
| II-14 | $-OCH_2COOC_2H_5$ | $-OCH_3$ | H | $-OCH_3$ | |
| II-15 | $-OC_3H_7(-n)$ | $-OCH_3$ | H | $-OCH_3$ | |
| II-16 | $-OCH_2$- 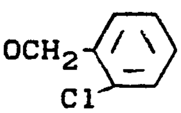 | $-OCH_3$ | H | $-OCH_3$ | |
| II-17 | -O- 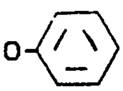 | $-OCH_3$ | H | $-OCH_3$ | |
| II-18 | $-OCH_2$- 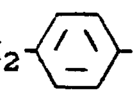 $-CH_3$ | $-OCH_3$ | H | $-CH_3$ | |
| II-19 | $-OCH_2$- 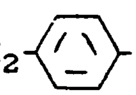 $-CH_3$ | $-OCH_3$ | H | $-OCH_3$ | |
| II-20 | $-OCH_2$- 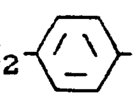 $-CH_3$ | $-CH_3$ | H | H | |
| II-21 | $-OCH_2$- 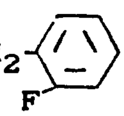 | $-OCH_3$ | H | $-OCH_3$ | |

Tabelle 2-Fortsetzung

| Beisp.-Nr. | $R^3$ | $R^4$ | $R^5$ | $R^6$ | Schmelzpunkt[°C] |
|---|---|---|---|---|---|
| II-22 | -O- 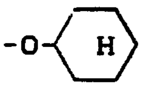 | $-OCH_3$ | H | $-OCH_3$ | |
| II-23 | $-O-CH_2-$ 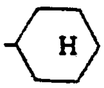 | $-OCH_3$ | H | $-OCH_3$ | |
| II-24 | $-O-CH_2CON(CH_3)_2$ | $-OCH_3$ | H | $-OCH_3$ | |
| II-25 | $-OCH_2OCH_3$ | $-OCH_3$ | H | $-OCH_3$ | |
| II-26 | $-OCH_2SCH_3$ | $-OCH_3$ | H | $-OCH_3$ | |
| II-27 | $-OCH_2-$ 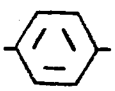 $-COOC_2H_5$ | $-OCH_3$ | H | $-OCH_3$ | |
| II-28 | $-OCH_2CF_3$ | $-OCH_3$ | H | $-OCH_3$ | |
| II-29 | $-OCH_2-$ 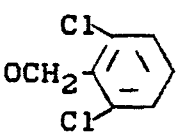 | $-OCH_3$ | H | $-OCH_3$ | |
| II-30 | $-OCH_2-$ 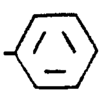 | $-CH_3$ | H | $-OCH_3$ | $n_D^{20}=1.5645$ |
| II-31 | $-OCH_2-$ 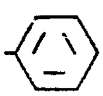 | $-OCH_3$ | H | $-OCH_3$ | 74 |
| II-32 | $-OCH_2-$ 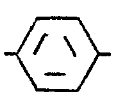 $-NO_2$ | $-OCH_3$ | H | $-CH_3$ | |

Tabelle 2-Fortsetzung

| Beisp.-Nr. | $R^3$ | $R^4$ | $R^5$ | $R^6$ | Schmelzpunkt [°C] |
|---|---|---|---|---|---|
| II-33 | $-OCH_2-$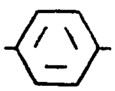$-NO_2$ | $-OCH_3$ | H | $-OCH_3$ | |
| II-34 | $-O-\underset{CH_3}{\underset{\vert}{C}}HCH_2CH_3$ | $-OCH_3$ | H | $-OCH_3$ | 68 |
| II-35 | $-OC_2H_5$ | $-CH_3$ | H | $-OC_2H_5$ | |
| II-36 | $-OCH_3$ | -Cl | H | $-OCH_3$ | 112 |
| II-37 | $-OC_3H_7$(-i) | -Cl | H | $-OCH_3$ | |
| II-38 | $-OCH_3$ | $-CH_3$ | H | $-OC_2H_5$ | |
| II-39 | $-OCH_3$ | $-CH_3$ | H | $-OCHF_2$ | |
| II-40 | $-OCH_2-$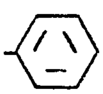 | $-CH_3$ | H | $-OCHF_2$ | |
| II-41 | $-OC_3H_7$(-n) | $-CH_3$ | H | $-SCH_3$ | |
| II-42 | $-OC_4H_9$(-n) | $-CH_3$ | H | $-N(CH_3)_2$ | |
| II-43 | $-OC_2H_5$ | $-OCH_3$ | H | $-OCH_3$ | |
| II-44 | $-OCH_3$ | $-OCH_3$ | H | $-N(CH_3)_2$ | |

<u>Tabelle 2</u>-Fortsetzung

| Beisp.-Nr. | $R^3$ | $R^4$ | $R^5$ | $R^6$ | Schmelz-punkt[°C] |
|---|---|---|---|---|---|
| II-45 | $-OCH_2-CH=CH_2$ | $-CH_3$ | H | $-SCH_3$ | |
| II-46 | $-OC_2H_5$ | $-CH_3$ | H | H | 95 |
| II-47 | $-OCH_2-COOC_2H_5$ | $-CH_3$ | H | H | |
| II-48 | $-OCH_2-$ 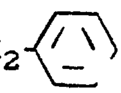 | $-CH_3$ | H | H | 150 |
| II-49 | $-OCH_2-$ 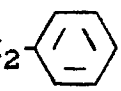 | -Cl | H | $-OC_2H_5$ | |
| II-50 | $-OC_3H_7(-n)$ | -Cl | H | $-N(CH_3)_2$ | |
| II-51 | $-OCH_3$ | -Cl | H | $-SCH_3$ | |
| II-52 | $-OCH_2CF_3$ | $-CH_3$ | H | $-SCH_3$ | |
| II-53 | $-OCH_2-CH=CH_2$ | $-CH_3$ | H | $-OCHF_2$ | |
| II-54 | $-OCH_2-$ 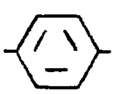 $-COOC_2H_5$ | $-CH_3$ | H | $-OC_2H_5$ | |
| II-55 | -O- 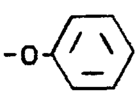 | $-CH_3$ | H | $-SCH_3$ | |
| II-56 | -O- 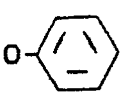 | $-CH_3$ | H | $-N(CH_3)_2$ | |

Tabelle 2-Fortsetzung

| Beisp.-Nr. | $R^3$ | $R^4$ | $R^5$ | $R^6$ | Schmelzpunkt[°C] |
|---|---|---|---|---|---|
| II-57 | $-OCH_2CH_2CH_2Cl$ | -Cl | H | $-OCH_3$ | |
| II-58 | $-OCH_2$- 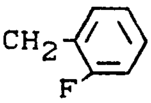 | $-CH_3$ | H | $-OC_2H_5$ | |
| II-59 | $-OCH_2$- 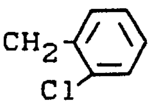 | H | H | $-CH_3$ | 140 |
| II-60 | $-OCH_2$- 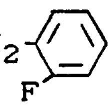 | H | H | $-CH_3$ | 205 |
| II-61 | $-OCH_3$ | H | H | $-C_2H_5$ | 98 |
| II-62 | $-OCH_2CH_2CH_2Cl$ | H | H | $-CH_3$ | 102 |
| II-63 | $-OCH_2$- 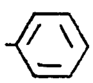 | $-C_2H_5$ | H | H | 112 |
| II-64 | $-OCH_2-C(CH_3)_3$ | H | H | $-CH_3$ | |
| II-65 | $-OCH_2CH_2-OCH_3$ | H | H | $-CH_3$ | 242 |
| II-66 | $-OCH_2CH_2-OC_2H_5$ | H | H | $-CH_3$ | |
| II-67 | $-OCH_2CH_2-SCH_3$ | H | H | $-CH_3$ | |

Tabelle 2-Fortsetzung

| Beisp.-Nr. | $R^3$ | $R^4$ | $R^5$ | $R^6$ | Schmelzpunkt[°C] |
|---|---|---|---|---|---|
| II-68 | $-OCH_2CH_2-SC_2H_5$ | H | H | $-CH_3$ | |
| II-69 | $-OCH_2-CONH_2$ | H | H | $-CH_3$ | |
| II-70 | $-OCH_2-CH=CHCl$ | H | H | $-CH_3$ | |
| II-71 | -OCH 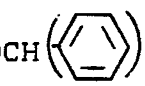 2 | H | H | $-CH_3$ | 165 |
| II-72 | -OC 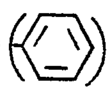 3 | H | H | $-CH_3$ | |
| II-73 | $-OCH_3$ | H | H | H | 107-109 |
| II-74 | $-OCH_3$ | $-OC_2H_5$ | H | $-OC_2H_5$ | |
| II-75 | $-OCH_2-CH=CH_2$ | H | H | $-C_2H_5$ | 83 |
| II-76 | $-OCH_2-CH=CH_2$ | $-CH_3$ | $-COOC_2H_5$ | H | 143 |

Herstellung von Ausgangsstoffen der Formel (III)

Beispiel (III-1)

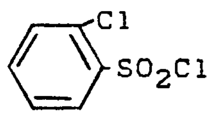

295 ml Phosphorylchlorid ('Phosphoroxychlorid') werden bei 20 °C bis 30 °C tropfenweise zu einer Mischung aus 172 g (0,8 Mol) 2-Chlor-benzolsulfonsäure-Natriumsalz, 300 ml Acetonitril und 300 ml Sulfolan gegeben. Das Reaktionsgemisch wird 4 Stunden bei 70 °C gerührt, anschließend auf 5 °C abgekühlt und mit Eiswasser verdünnt. Nach Extrahieren mit Petrolether, Waschen der Extraktionslösung mit Wasser, Trocknen, Filtrieren und Einengen wird das im Rückstand verbliebene Produkt durch Vakuumdestillation gereinigt.

Man erhält 117 g (70 % der Theorie) 2-Chlor-benzolsulfonsäurechlorid vom Siedepunkt 110 °C / 1,1 mbar.

Beispiel (III-2)

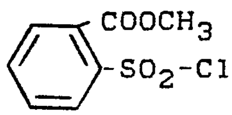

75,5 g (0,5 Mol) 2-Aminobenzoesäure-methylester werden in 176 ml konzentrierte Salzsäure und 100 ml Essigsäure gelöst. Hierzu wird bei 0 °C eine Lösung von 34,4 g Natriumnitrit in 70 ml Wasser getropft. Nach 15-minütigem Nachrühren wird das Reaktionsgemisch langsam zu einer auf 0 °C gekühlten gesättigten Lösung von Schwefeldioxid in 450 ml Essigsäure gegeben. Nach Entfernung des Kühlbades wird bis zum Ende der Gasentwicklung gerührt, wobei 10 g Kupfer(II)-chlorid portionsweise eingetragen werden. Nach Verdünnen mit Eiswasser, Extrahieren mit Methylenchlorid, Waschen der Extraktionslösung mit Wasser, Trocknen, Filtrieren und Einengen wird das im Rückstand verbliebene Produkt durch Vakuumdestillation gereinigt.

Man erhält 45 g (38 % der Theorie) 2-Methoxycarbonyl-benzolsulfonsäurechlorid vom Siedepunkt 150 °C/1,33 mbar.

Analog können die in der nachstehenden Tabelle 3 aufgeführten Verbindungen der Formel (III) hergestellt werden:

$$R^1\text{-}SO_2\text{-}Cl \qquad (III)$$

Tabelle 3

| Beisp.-Nr. | $R^1$ | Siedepunkt/Druck |
|---|---|---|
| III-3 | 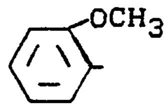 | (Öl, Zersetzung bei Destillation) |
| III-4 | 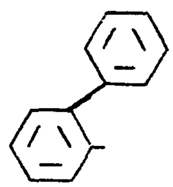 | [Schmelzpunkt:100°C] |
| III-5 | 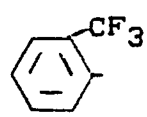 | (Öl) |
| III-6 | 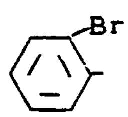 | 142 °C/4 mbar |
| III-7 | 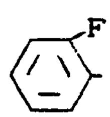 | 106 °C/4 mbar |

Tabelle 3-Fortsetzung

| Beisp.-Nr. | $R^1$ | Siedepunkt/Druck |
|---|---|---|
| III-8 | 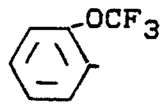 | [Schmelzpunkt: 32°C] |
| III-9 | 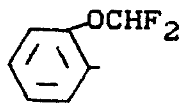 | (Öl, Zersetzung bei Destillation) |
| III-10 | 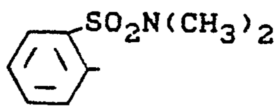 | [Schmelzpunkt:103°C] |
| III-11 | 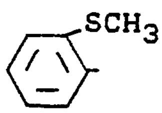 | (Öl, Zersetzung bei Destillation) |
| III-12 | 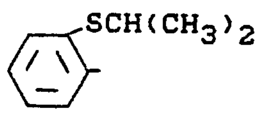 | 90 °C/1,33 mbar |
| III-13 | 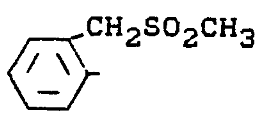 | [Schmelzpunkt:120°C] |
| III-14 | 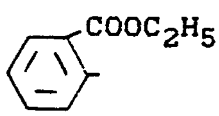 | 155 °C/5,32 mbar |
| III-15 | 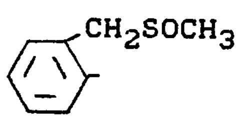 | |

Tabelle 3-Fortsetzung

| Beisp.-Nr. | $R^1$ | Siedepunkt/Druck |
|---|---|---|
| III-16 | 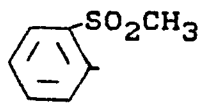 | [Schmelzpunkt:128°C] |
| III-17 | 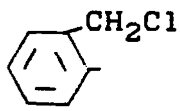 | |
| III-18 | 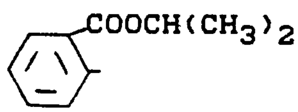 | |
| III-19 | 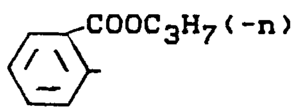 | |
| III-20 | 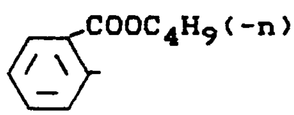 | |
| III-21 | 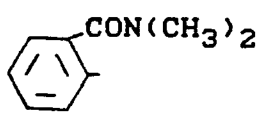 | |
| III-22 | 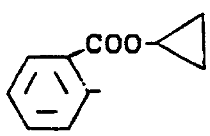 | |
| III-23 | 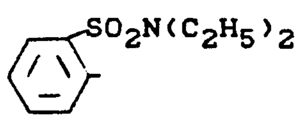 | [Schmelzpunkt: 73°C] |

Tabelle 3-Fortsetzung

| Beisp.-Nr. | $R^1$ | Siedepunkt/Druck |
|---|---|---|
| III-24 | 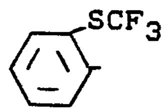 | [Schmelzpunkt:41-43°C] |
| III-25 | 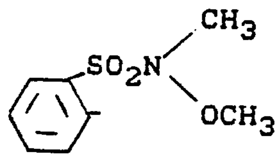 | |
| III-26 | 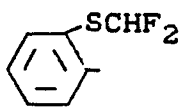 | |
| III-27 | 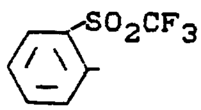 | |
| III-28 | 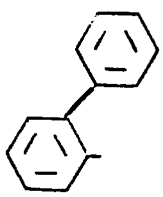 | |
| III-29 | 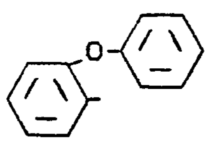 | |
| III-30 | 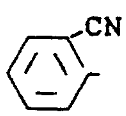 | |

Tabelle 3-Fortsetzung

| Beisp.-Nr. | $R^1$ | Siedepunkt/Druck |
| --- | --- | --- |
| III-31 | 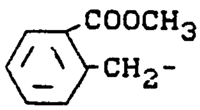 | [Schmelzpunkt: 84°C] |
| III-32 | 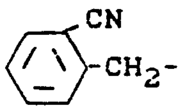 | |
| III-33 | 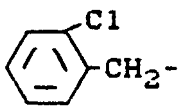 | wachsartig |

Herstellung von Ausgangsstoffen der Formel (VI)

Beispiel (VI-1)

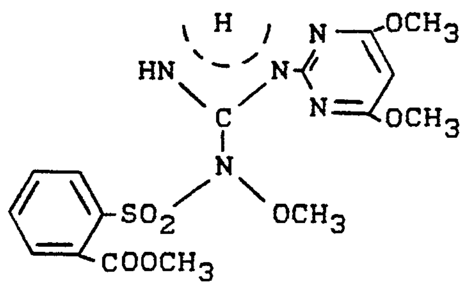

Eine Lösung aus 2,8 g (0,012 Mol) 2-Methoxycarbonyl-benzolsulfonsäurechlorid in 10 ml Tetrahydrofuran wird bei -5 °C unter Rühren zu einer Mischung aus 2,3 g (0,01 Mol) N'-(4,6-Dimethoxy-pyrimidin-2-yl)-N''-methoxy-guanidin und 30 ml Tetrahydrofuran gegeben. Dann wird eine Lösung von 1,3 g (0,012 Mol) Diazabicyclooctan in 10 ml Tetrahydrofuran bei -5 °C tropfenweise dazu gegeben und das Gemisch wird 2 Stunden bei 20 °C gerührt. Nach Zugabe von weiteren 0,5 g 2-Methoxycarbonyl-benzolsulfonsäurechlorid und 0,25 g Diazabicyclooctan wird weitere 2 Stunden bei 20 °C gerührt. Nach Filtration wird eingeengt, der Rückstand mit Toluol zur Kristallisation gebracht und durch Absaugen isoliert.

Man erhält 2,25 g (57 % der Theorie) N'-(4,6-Dimethoxy-pyrimidin-2-yl)-N''-methoxy-N''-(2-methoxycarbonyl-benzolsulfonyl)-guanidin vom Schmelzpunkt 142 - 143 °C.

Analog können die in der nachstehenden Tabelle 4 aufgeführten Verbindungen der Formel (VI) hergestellt werden:

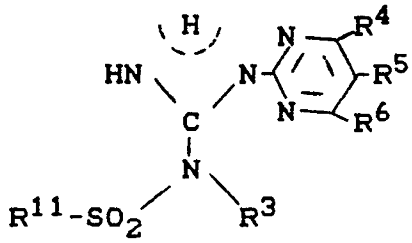

(VI)

Tabelle 4-Fortsetzung

| Beisp.-Nr. | $R^3$ | $R^4$ | $R^5$ | $R^6$ | $R^{11}$ | Schmelzpunkt [°C] |
|---|---|---|---|---|---|---|
| VI-2 | $-OCH_3$ | $-OCH_3$ | H | $-OCH_3$ | 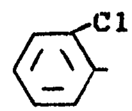 | (glasartig) |
| VI-3 | $-OCH_3$ | $-CH_3$ | H | $-OCH_3$ | 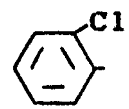 | |
| VI-4 | $-OCH_3$ | $-CH_3$ | H | $-OCH_3$ | 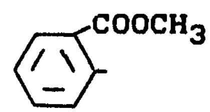 | |
| VI-5 | $-OCH_3$ | $-OCH_3$ | H | $-OCH_3$ | 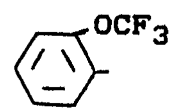 | (glasartig) |
| VI-6 | $-OCH_3$ | $-OCH_3$ | H | $-OCH_3$ | 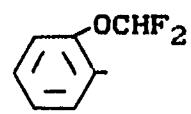 | (glasartig) |

Tabelle 4-Fortsetzung

| Beisp.-Nr. | $R^3$ | $R^4$ | $R^5$ | $R^6$ | $R^{11}$ | Schmelzpunkt [°C] |
|---|---|---|---|---|---|---|
| VI-7 | $-OCH_2CH(CH_3)_2$ | $-OCH_3$ | H | $-OCH_3$ | 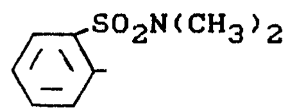 | |
| VI-8 | $-OCH_3$ | $-OCH_3$ | H | $-OCH_3$ | 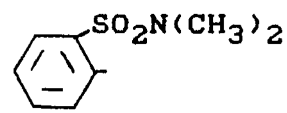 | |
| VI-9 | $-OCH_3$ | $-OCH_3$ | H | $-OCH_3$ | 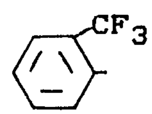 | |
| VI-10 | $-OCH_3$ | $-CH_3$ | H | H | 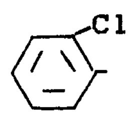 | 124 |
| VI-11 | $-OCH(CH_3)CH_2CH_3$ | $-OCH_3$ | H | $-OCH_3$ | 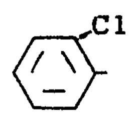 | 124 |

Tabelle 4-Fortsetzung

| Beisp.-Nr. | $R^3$ | $R^4$ | $R^5$ | $R^6$ | $R^{11}$ | Schmelzpunkt [°C] |
|---|---|---|---|---|---|---|
| VI-12 | $-OCH_3$ | $-CH_3$ | H | $-OCH_3$ | 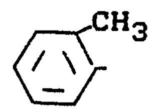 | |
| VI-13 | $-OCH_3$ | $-CH_3$ | H | $-OCH_3$ | 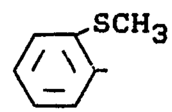 | |
| VI-14 | $-OCH_2-$ 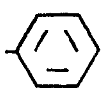 | $-CH_3$ | H | H | 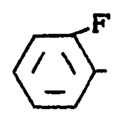 | |
| VI-15 | $-OC_2H_5$ | $-CH_3$ | H | $-OC_2H_5$ | 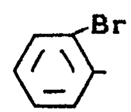 | |
| VI-16 | $-OCH_3$ | -Cl | H | $-OCH_3$ | 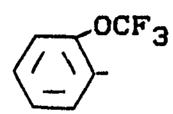 | |

Tabelle 4-Fortsetzung

| Beisp.-Nr. | $R^3$ | $R^4$ | $R^5$ | $R^6$ | $R^{11}$ | Schmelzpunkt [°C] |
|---|---|---|---|---|---|---|
| VI-17 | $-OC_3H_7$ | $-OCH_3$ | H | $-OCH_3$ | 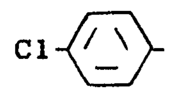 | |
| VI-18 | $-OCH_3$ | -Cl | H | $-OCH_3$ | 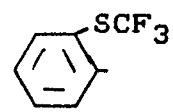 | |
| VI-19 | $-OCH_3$ | $-CH_3$ | H | H | 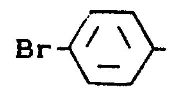 | |
| VI-20 | $-OC_2H_5$ | $-CH_3$ | H | $-OCH_3$ | 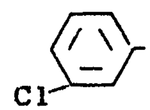 | |
| VI-21 | $-OCH_3$ | $-CH_3$ | H | $-OCHF_2$ | 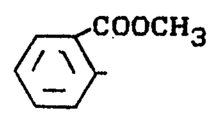 | |

Tabelle 4-Fortsetzung

| Beisp.-Nr. | $R^3$ | $R^4$ | $R^5$ | $R^6$ | $R^{11}$ | Schmelzpunkt [°C] |
|---|---|---|---|---|---|---|
| VI-22 | $-OCH_2CH=CH_2$ | $-CH_3$ | H | H | 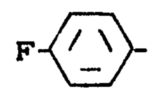 | |
| VI-23 | $-OC_4H_9(-n)$ | $-CH_3$ | H | H | 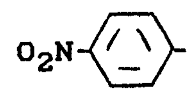 | |
| VI-24 | $-OCH_3$ | $-CH_3$ | H | $-OCH_3$ | $(n-)C_4H_9-$ | |
| VI-25 | $-OCH_3$ | $-OCH_3$ | H | $-OCH_3$ | $(CH_3)_2N-$ | |
| VI-26 | $-OCH_3$ | $-CH_3$ | H | H | $(n-)C_8F_{17}-$ | |

**Tabelle 4-Fortsetzung**

| Beisp.-Nr. | $R^3$ | $R^4$ | $R^5$ | $R^6$ | $R^{11}$ | Schmelzpunkt [°C] |
|---|---|---|---|---|---|---|
| VI-27 | $-OCH_3$ | H | H | H | 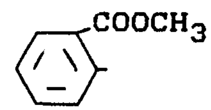 | 125 - 126 |
| VI-28 | $-OCH_3$ | H | H | H | 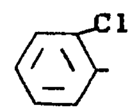 | 122 - 123 |

Herstellung von Ausgangsstoffen der Formel (VII)

Beispiel (VII -1)

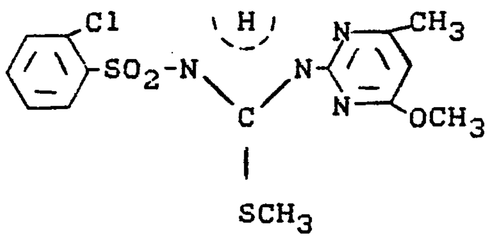

Eine Suspension von 8,4 g (0,35 Mol) Natriumhydrid (in Öl) wird unter Rühren zu einer Mischung aus 28,0 g (0,2 Mol) 2-Amino-4-methoxy-6-methyl-pyrimidin gegeben und das Gemisch wird 15 Stunden bei 20 °C gerührt. Dann werden 60 g (0,2 Mol) N-(2-Chlor-benzolsulfonyl)-S',S''-dimethyl-iminodithiokohlensäureester portionsweise so zum Reaktionsgemisch gegeben, daß die Reaktionstemperatur nicht über 40 °C steigt. Nach fünfstündigem Rühren bei 20 °C wird 1 l Eiswasser dazugegeben und filtriert. Das Filtrat wird mit konzentrierter Salzsäure angesäuert und das hierbei kristallin angefallene Produkt wird durch Absaugen isoliert.

Man erhält 67 g (87 % der Theorie N'-(4-Methoxy-6-methyl-pyrimidin-2-yl)-N''-(2-chlor-benzolsulfonyl)-S-methyl-isothioharnstoff vom Schmelzpunkt 148 °C.

Analog können die in der nachstehenden Tabelle 5 aufgeführten Verbindungen der Formel (VII) hergestellt werden:

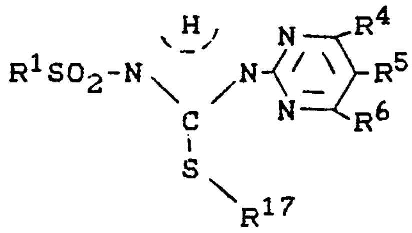

(VII)

Tabelle 5

| Bsp.-Nr. | $R^1$ | $R^4$ | $R^5$ | $R^6$ | $R^{17}$ | Schmelzpunkt[°C] |
|---|---|---|---|---|---|---|
| VII-2 | 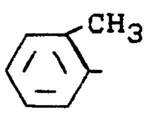 | $-CH_3$ | H | $-OCH_3$ | $-CH_3$ | 163 |
| VII-3 | 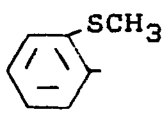 | $-CH_3$ | H | $-OCH_3$ | $-CH_3$ | 163 |
| VII-4 | 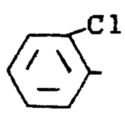 | $-OCH_3$ | H | $-OCH_3$ | $-CH_3$ | 168 |
| VII-5 | 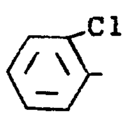 | $-CH_3$ | H | H | $-CH_3$ | 152-153 |
| VII-6 | 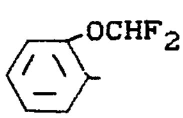 | $-OCH_3$ | H | $-OCH_3$ | $-CH_3$ | |

<u>Tabelle 5</u>-Fortsetzung

| Bsp.-Nr. | $R^1$ | $R^4$ | $R^5$ | $R^6$ | $R^{17}$ | Schmelzpunkt[°C] |
|---|---|---|---|---|---|---|
| VII-7 | 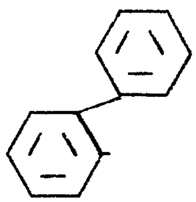 | $-OCH_3$ | H | $-OCH_3$ | $-CH_3$ | |
| VII-8 | 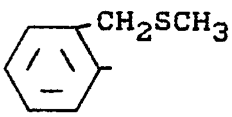 | $-CH_3$ | H | H | $-CH_3$ | |
| VII-9 | 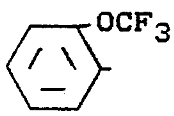 | $-OCH_3$ | H | $-OCH_3$ | $-CH_3$ | |
| VII-10 | 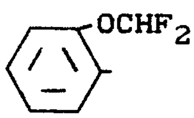 | $-CH_3$ | H | H | $-CH_3$ | |
| VII-11 | 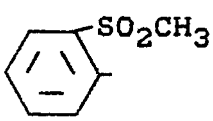 | $-CH_3$ | H | $-OCH_3$ | $-CH_3$ | |
| VII-12 | 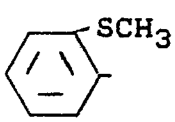 | $-OCH_3$ | H | $-OCH_3$ | $-CH_3$ | |
| VII-13 | 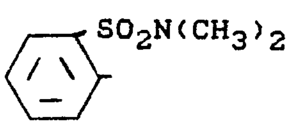 | $-OCH_3$ | H | $-OCH_3$ | $-CH_3$ | |
| VII-14 | 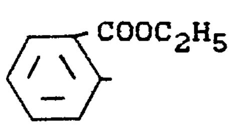 | $-OCH_3$ | H | $-CH_3$ | $-CH_3$ | |

Tabelle 5-Fortsetzung

| Bsp.-Nr. | $R^1$ | $R^4$ | $R^5$ | $R^6$ | $R^{17}$ | Schmelzpunkt [°C] |
|---|---|---|---|---|---|---|
| VII-15 | 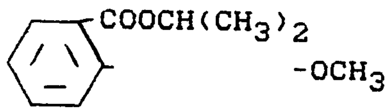 | $-OCH_3$ | H | $-OCH_3$ | $-CH_3$ | |
| VII-16 | 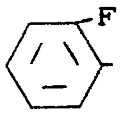 | $-OCH_3$ | H | $-OCH_3$ | $-CH_3$ | |
| VII-17 | 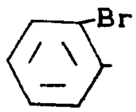 | $-OCH_3$ | H | $-OCH_3$ | $-CH_3$ | |
| VII-18 | 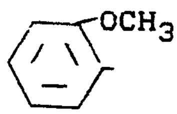 | $-OCH_3$ | H | $-OCH_3$ | $-CH_3$ | |
| VII-19 | 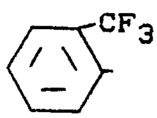 | $-OCH_3$ | H | $-OCH_3$ | $-CH_3$ | |
| VII-20 | 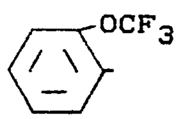 | $-CH_3$ | H | $-OC_2H_5$ | $-CH_3$ | |
| VII-21 | 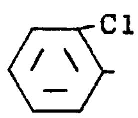 | $-CH_3$ | H | $-OC_2H_5$ | $-CH_3$ | |
| VII-22 | 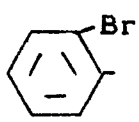 | -Cl | H | $-OCH_3$ | $-CH_3$ | |

Tabelle 5-Fortsetzung

| Bsp.-Nr. | $R^1$ | $R^4$ | $R^5$ | $R^6$ | $R^{17}$ | Schmelzpunkt[°C] |
|---|---|---|---|---|---|---|
| VII-23 | 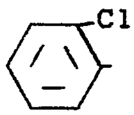 | -Cl | H | $-SCH_3$ | $-CH_3$ | 136 |
| VII-24 | 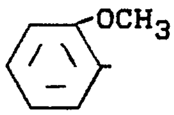 | -Cl | H | $-SCH_3$ | $-CH_3$ | |
| VII-25 | 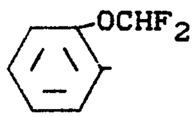 | -Cl | H | $-OC_2H_5$ | $-CH_3$ | |
| VII-26 | 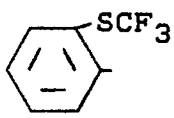 | $-OCH_3$ | H | $-OCH_3$ | $-CH_3$ | |
| VII-27 | 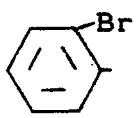 | -Cl | H | $-N(CH_3)_2$ | $-CH_3$ | |
| VII-28 | 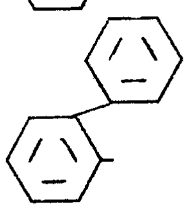 | -Cl | H | $-N(CH_3)_2$ | $-CH_3$ | |
| VII-29 | 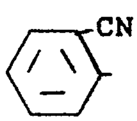 | $-CH_3$ | H | $-SCH_3$ | $-CH_3$ | |
| VII-30 | 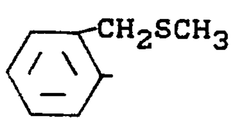 | $-CH_3$ | H | $-SCH_3$ | $-CH_3$ | |

Tabelle 5-Fortsetzung

| Bsp.-Nr. | $R^1$ | $R^4$ | $R^5$ | $R^6$ | $R^{17}$ | Schmelzpunkt[°C] |
|---|---|---|---|---|---|---|
| VII-31 | 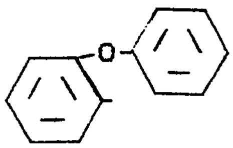 | $-CH_3$ | H | $-N(CH_3)_2$ | $-CH_3$ | |
| VII-32 | 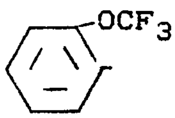 | $-CH_3$ | H | $-N(CH_3)_2$ | $-CH_3$ | |
| VII-33 | 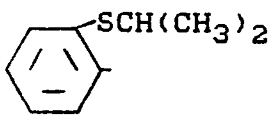 | $-CH_3$ | H | $-OCHF_2$ | $-CH_3$ | |
| VII-34 | 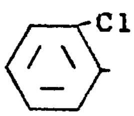 | $-CH_3$ | H | $-OCHF_2$ | $-CH_3$ | |
| VII-35 | 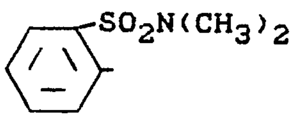 | $-CH_3$ | H | $-OCHF_2$ | $-CH_3$ | |
| VII-36 | 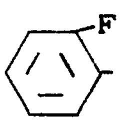 | $-CH_3$ | H | $-OC_2H_5$ | $-C_2H_5$ | |
| VII-37 | 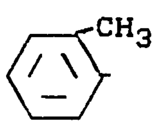 | $-CH_3$ | H | H | $-CH_3$ | |
| VII-38 | 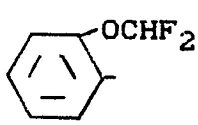 | $-CH_3$ | H | $-N(CH_3)_2$ | $-CH_3$ | |

Tabelle 5-Fortsetzung

| Bsp.-Nr. | $R^1$ | $R^4$ | $R^5$ | $R^6$ | $R^{17}$ | Schmelzpunkt [°C] |
|---|---|---|---|---|---|---|
| VII-39 | 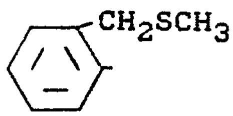 | -Cl | H | $-OCH_3$ | $-CH_3$ | |
| VII-40 | 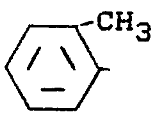 | $-OCH_3$ | H | $-OCH_3$ | $-CH_3$ | |
| VII-41 | 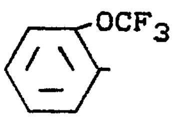 | $-CH_3$ | H | $-OCHF_2$ | $-CH_3$ | |
| VII-42 | 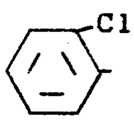 | $-CH_3$ | $-CH_3$ | H | $-CH_3$ | 133 |
| VII-43 | 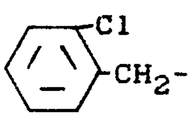 | $-CH_3$ | $-CH_3$ | H | $-CH_3$ | 150 |

0 173 321

Herstellung von Ausgangsstoffen der Formel (VIII)

Beispiel (VIII-1)

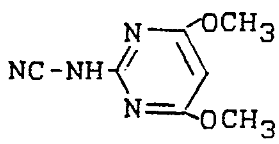

Eine auf 100 °C erhitzte Lösung von 24 g (0,427 Mol) Kaliumhydroxid in 100 ml Wasser wird bei 100 °C unter Rühren zu einer Mischung von 9,2 g (0,043 Mol) 4,6-Dimethoxy-pyrimidin-2-yl-thioharnstoff in 70 ml Wasser gegeben. Man rührt 2 Minuten bei 100 °C nach und gibt dann eine auf 100 °C erwärmte Lösung von 16,2 g (0,05 Mol) Blei-(II)-acetat in 30 ml Wasser hinzu. Man erhitzt noch 5 Minuten unter Rückfluß, kühlt dann auf 0 °C bis 5 °C ab und versetzt die wässrige Lösung mit 30 ml Eisessig. Das hierbei kristallin ausfallende Produkt wird durch Absaugen isoliert.

Man erhält 6,3 g (81,5 % der Theorie) 2-Cyanamino-4,6-dimethoxy-pyrimidin vom Schmelzpunkt 202 °C.

Beispiel (VIII-2)

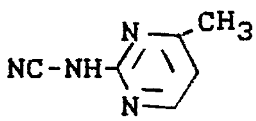

Zu einer Aufschlämmung von 84 g (1.0 Mol) Dicyandiamid in 500 ml Methanol werden 54 g Natriummethanolat (1.0 Mol) gegeben und 15 Minuten gerührt. Nach Zugabe von 132 g (1.0 Mol) Acetylacetaldehyddimethylacetal wird 3 Stunden unter Rückfluß gerührt. Die klare, dunkelrote Reaktionslösung wird im Vakuum bis zur Trockne eingeengt, der Rückstand wird in 1500 ml Wasser gelöst und mit konzentrierter Salzsäure unter Rühren auf pH 3 eingestellt. Das hierbei kristallin ausfallende Produkt wird durch Absaugen isoliert.

Man erhält 103 g (78 % der Theorie) 2-Cyanamino-4-methyl-pyrimidin vom Schmelzpunkt 203 °C (Zers.).

Analog können die in der nachstehenden Tabelle 6 aufgeführten Verbindungen der Formel (VIII) hergestellt werden:

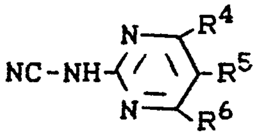 (VIII)

Tabelle 6

| Beisp.-Nr. | $R^4$ | $R^5$ | $R^6$ | Schmelzpunkt [°C] |
|---|---|---|---|---|
| VIII-3 | $-CH_3$ | H | $-OCH_3$ | 258 (Zers.) |
| VIII-4 | $-CH_3$ | H | $-OCHF_2$ | 174 |
| VIII-5 | -Cl | H | $-OCH_3$ | 200 |
| VIII-6 | -Cl | H | $-OC_2H_5$ | |
| VIII-7 | -Cl | H | $-N(CH_3)_2$ | |
| VIII-8 | $-CH_3$ | H | $-N(CH_3)_2$ | |
| VIII-9 | $-CH_3$ | H | $-OC_2H_5$ | |
| VIII-10 | $-CH_3$ | $-COOC_2H_5$ | H | 126 |

Tabelle 6-Fortsetzung

| Beisp.-Nr. | $R^4$ | $R^5$ | $R^6$ | Schmelzpunkt [°C] |
|---|---|---|---|---|
| VIII-11 | $-CH_3$ | $-COCH_3$ | H | 174 |
| VIII-12 | $-C_2H_5$ | H | H | 146 |
| VIII-13 | H | H | H | 186 |
| VIII-14 | $-OC_2H_5$ | H | $-OC_2H_5$ | 235 - 237 |
| VIII-15 | -OH | $-COOC_2H_5$ | H | 220 (Zers.) |
| VIII-16 | -OH | H | H | > 300 |
| VIII-17 | -Cl | H | $-CH_3$ | |

Herstellung der Ausgangsstoffe der Formel (XIII)

Beispiel (XIII-1)

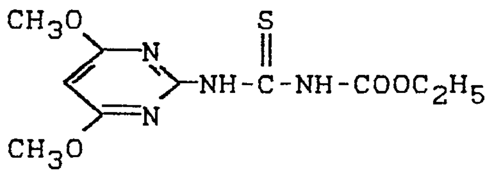

Eine Mischung aus 15,5 g (0,1 Mol) 2-Amino-4,6-dimethoxy-pyrimidin, 13,1 g (0,1 Mol) Ethoxycarbonylisothiocyanat und 200 ml Acetonitril wird 2 Stunden bei 60 °C gerührt. Dann wird auf 10 °C abgekühlt und das kristallin angefallene Produkt durch Absaugen isoliert.

Man erhält 22,5 g (75 % der Theorie) 1-(Ethoxycarbonyl)-3-(4,6-dimethoxy-pyrimidin-2-yl)-thioharnstoff vom Schmelzpunkt 194 °C (Zers.).

Analog können die in der nachstehenden Tabelle 7 aufgeführten Verbindungen der Formel (XIII) hergestellt werden:

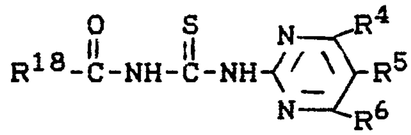 (XIII)

Tabelle 7

| Beisp.-Nr. | $R^4$ | $R^5$ | $R^6$ | $R^{18}$ | Schmelzpunkt[°C] |
|---|---|---|---|---|---|
| XIII-2 | $-OCH_3$ | H | $-OCH_3$ | 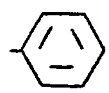 | 189 |
| XIII-3 | $-CH_3$ | H | H | 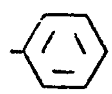 | 198-199 (Zers.) |
| XIII-4 | $-CH_3$ | H | $-OCH_3$ | $-OC_2H_5$ | 217 |
| XIII-5 | $-CH_3$ | H | $-OCH_3$ | 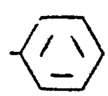 | 190 |
| XIII-6 | $-CH_3$ | H | $-OCHF_2$ | 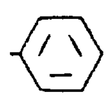 | 182 |
| XIII-7 | $-CH_3$ | H | $-OCHF_2$ | $-OC_2H_5$ | 184-185 |
| XIII-8 | $-OCHF_2$ | H | $-OCHF_2$ | $-OC_2H_5$ | 173 |

Tabelle 7-Fortsetzung

| Beisp.-Nr. | $R^4$ | $R^5$ | $R^6$ | $R^{18}$ | Schmelzpunkt[°C] |
|---|---|---|---|---|---|
| XIII-9 | -Cl | H | $-OCH_3$ | $-OC_2H_5$ | 160-162 |
| XIII-10 | -Cl | H | -Cl | $-OC_2H_5$ | 132-136 |
| XIII-11 | -Cl | H | $-N(CH_3)_2$ | $-OC_2H_5$ | 168 |
| XIII-12 | H | H | H | 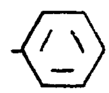 | 173 |
| XIII-13 | $-OC_2H_5$ | H | $-OC_2H_5$ | 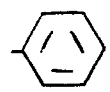 | 179 |
| XIII-14 | $-OC_2H_5$ | H | $-OC_2H_5$ | $-OC_2H_5$ | 159 |
| XIII-15 | $-CH_3$ | H | $-OC_2H_5$ | 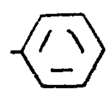 | 156 |
| XIII-16 | -Cl | H | $-CH_3$ | 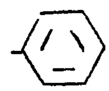 | 144 |

Herstellung der Ausgangsstoffe der Formel (XIV)

Beispiel (XIV-1)

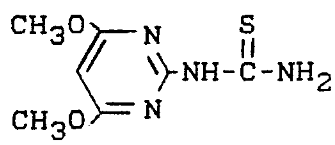

Eine Mischung von 5,0 g (0,0175 Mol) 1-(Ethoxycarbonyl)-3-(4,6-dimethoxy-pyrimidin-2-yl)-thioharnstoff, von 4,0 g (0,1 Mol) Natriumhydroxid und von 100 ml Wasser wird 2 Tage bei 20 °C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94 % der Theorie) 4,6-Dimethoxy-pyrimidin-2-yl-thioharnstoff vom Schmelzpunkt 245 - 248 °C (Zers.).

Analog können die in der nachstehenden Tabelle 8 aufgeführten Verbindungen der Formel (XIV) hergestellt werden:

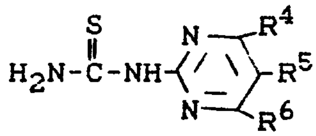

(XIV)

Tabelle 8

| Beisp.-Nr. | $R^4$ | $R^5$ | $R^6$ | Schmelzpunkt [°C] |
|---|---|---|---|---|
| XIV-2 | $-CH_3$ | H | H | 264-265 |
| XIV-3 | $-CH_3$ | H | $-OCH_3$ | 205-207 |
| XIV-4 | $-CH_3$ | H | $-OCHF_2$ | 192-194 |
| XIV-5 | -Cl | H | $-OCH_3$ | 225-227 (Zers.) |
| XIV-6 | -Cl | H | $-N(CH_3)_2$ | |
| XIV-7 | -Cl | H | $-OC_2H_5$ | |
| XIV-8 | $-CH_3$ | H | $-OC_2H_5$ | |
| XIV-9 | $-CH_3$ | H | $-SCH_3$ | |

Tabelle 8-Fortsetzung

| Beisp.-Nr. | $R^4$ | $R^5$ | $R^6$ | Schmelzpunkt [°C] |
|---|---|---|---|---|
| XIV-10 | $-CH_3$ | H | $-N(CH_3)_2$ | |
| XIV-11 | H | H | H | 263 |
| XIV-12 | $-OC_2H_5$ | H | $-OC_2H_5$ | 166 |
| XIV-13 | $-CH_3$ | H | -Cl | |

Herstellung von Ausgangsstoffen der Formel (XV)

Beispiel (XV-1)

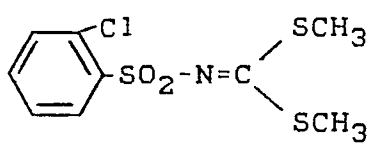

Zu einer Lösung von 20 g (0,1 Mol) 2-Chlor-benzolsulfonsäureamid in 80 ml Dimethylformamid werden bei 20 °C gleichzeitig (aus verschiedenen Tropftrichtern) 8 g (0,2 Mol) Natriumhydroxid - gelöst in 15 ml Wasser - und 6 ml (0,11 Mol) Schwefelkohlenstoff tropfenweise gegeben. Nach einstündigem Rühren werden 13 ml (0,22 Mol) Methyliodid zugetropft, und das Reaktionsgemisch wird eine weitere Stunde bei 20 °C gerührt. Durch Zugabe von 500 ml Wasser wird das Produkt ausgefällt und durch Absaugen isoliert.

Man erhält 22,1 g (75 % der Theorie) N-(2-Chlor-benzolsulfonyl)-S',S''-dimethyl-isodithiocarbamidsäureester vom Schmelzpunkt 112 °C.

Analog können die in der nachstehenden Tabelle 9 aufgeführten Verbindungen der Formel (XV) hergestellt werden:

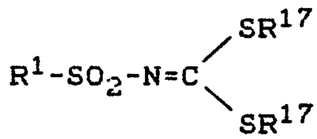 (XV)

Tabelle 9

| Beisp.-Nr. | $R^1$ | $R^{17}$ | Schmelzpunkt [°C] |
|---|---|---|---|
| XV-2 | 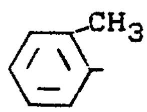 | $-CH_3$ | 103 |
| XV-3 | 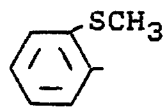 | $-CH_3$ | 143 |
| XV-4 | 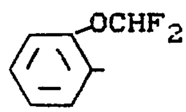 | $-CH_3$ | 112 |
| XV-5 | 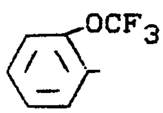 | $-CH_3$ | 88 |
| XV-6 | 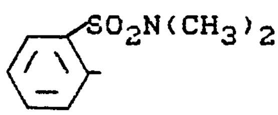 | $-CH_3$ | |

Tabelle 9-Fortsetzung

| Beisp.-Nr. | $R^1$ | $R^{17}$ | Schmelzpunkt [°C] |
|---|---|---|---|
| XV-7 | 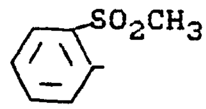 | $-CH_3$ | |
| XV-8 | 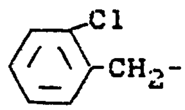 | $-CH_3$ | 96 |
| XV-9 | 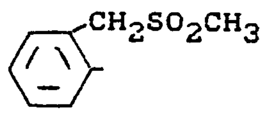 | $-CH_3$ | |
| XV-10 | 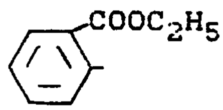 | $-CH_3$ | |
| XV-11 | 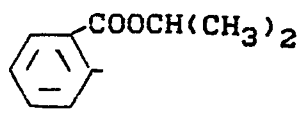 | $-CH_3$ | |
| XV-12 | 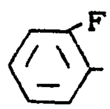 | $-CH_3$ | |
| XV-13 | 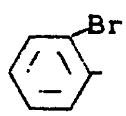 | $-CH_3$ | |
| XV-14 | 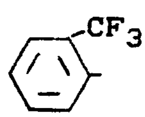 | $-CH_3$ | |

Tabelle 9-Fortsetzung

| Beisp.-Nr. | $R^1$ | $R^{17}$ | Schmelzpunkt [°C] |
|---|---|---|---|
| XV-15 | 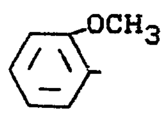 | $-CH_3$ | |
| XV-16 | 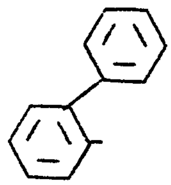 | $-CH_3$ | |
| XV-17 | 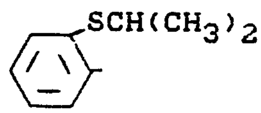 | $-CH_3$ | 98 |
| XV-18 | 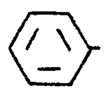 | $-CH_3$ | 100 |
| XV-19 | 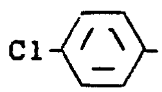 | $-CH_3$ | 94 |
| XV-20 | 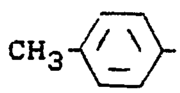 | $-CH_3$ | 107 |
| XV-21 | 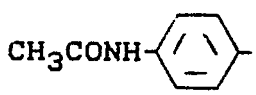 | $-CH_3$ | 170 |
| XV-22 | 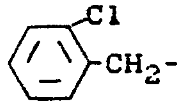 | $-C_2H_5$ | 64-66 |
| XV-23 | 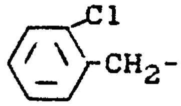 | $-CH(CH_3)_2$ | 65-67 |

Tabelle 9-Fortsetzung

| Beisp.-Nr. | $R^1$ | $R^{17}$ | Schmelzpunkt [°C] |
|---|---|---|---|
| XV-24 | 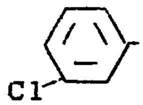 | $-CH_3$ | 84 |
| XV-25 | 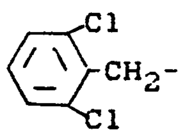 | $-CH_3$ | 91 |

Herstellung von Ausgangsstoffen der Formel (XVI)

Beispiel (XVI-1)

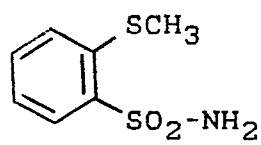

50 ml konzentrierte wässrige Ammoniaklösung werden bei 20 °C bis 30 °C unter Rühren zu einer Mischung aus 60 g (0,27 Mol) 2-Methylthiobenzolsulfonsäurechlorid und 60 ml Aceton getropft. Man rührt eine Stunde bei 20 °C nach und gießt das Reaktionsgemisch dann in 600 ml 10-prozentige Natronlauge. Diese Lösung wird filtriert und dann mit konzentrierter Salzsäure angesäuert. Das hierbei kristallin angefallene Produkt wird durch Absaugen isoliert.

Man erhält 19 g (30 % der Theorie) 2-Methylthio-benzolsulfonsäureamid vom Schmelzpunkt 149 °C.

Nach dem im vorausgehenden Beispiel exemplarisch beschriebenen Verfahren können die in der nachstehenden Tabelle 10 aufgeführten Verbindungen der Formel (XVI) hergestellt werden:

$$R^1\text{-}SO_2\text{-}NH_2 \qquad (XVI)$$

Tabelle 10

| Beisp.-Nr. | $R^1$ | Schmelzpunkt [°C] |
| --- | --- | --- |
| XVI-2 | 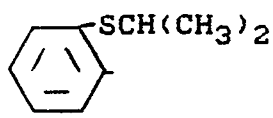 | 102 |
| XVI-3 | 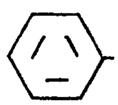 | 156 |
| XVI-4 | 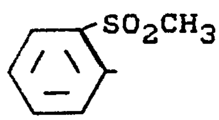 | 235 |
| XVI-5 | 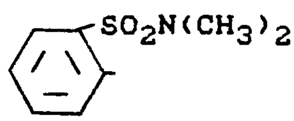 | |
| XVI-6 | 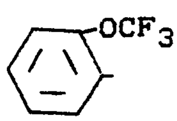 | 186 - 189 |

Tabelle 10-Fortsetzung

| Beisp.-Nr. | $R^1$ | Schmelzpunkt [°C] |
|---|---|---|
| XVI-7 | 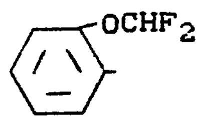 | 134 |
| XVI-8 | 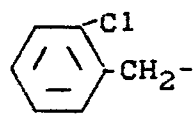 | |
| XVI-9 | 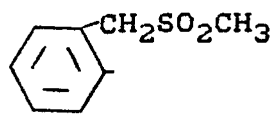 | 175 |
| XVI-10 | 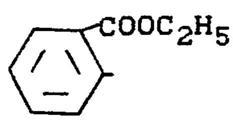 | 78 |
| XVI-11 | 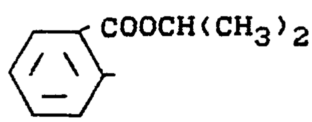 | |
| XVI-12 | 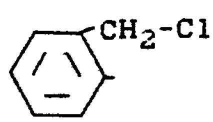 | |
| XVI-13 | 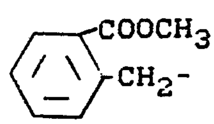 | |
| XVI-14 | 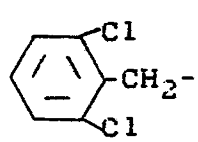 | 203 |

Beispiel A

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigten z. B. die folgenden Verbindungen der Herstellungsbeispiele eine ausgezeichnete Wirksamkeit: (3) und (11).

Beispiel B

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Bei diesem Test zeigten z. B. die folgenden Verbindungen der Herstellungsbeispiele eine ausgezeichnete Wirksamkeit: (3), (10), (11) und (39).

Patentansprüche

1. Sulfonylguanidinopyrimidin-Derivate der allgemeinen Formel (I)

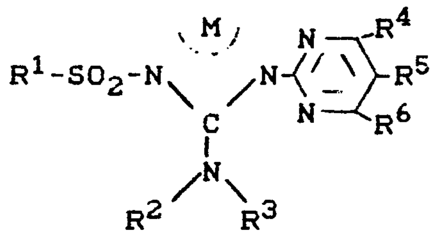

(I)

in welcher

M für Wasserstoff oder ein Metalläquivalent steht,

$R^1$ für einen gegebenenfalls substituierten Phenylrest 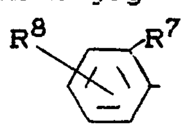 steht, worin

$R^7$ und $R^8$ gleich oder verschieden sind und für Wasserstoff, Halogen [insbesondere Fluor, Chlor und/oder Brom], Cyano, Nitro, $C_1$-$C_4$-Alkyl [welches gegebenenfalls durch Fluor, Chlor, Brom, Cyano, $C_1$-$C_4$-Alkoxycarbonyl, $C_1$-$C_4$-Alkyl-amino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkyl-sulfonyl substituiert ist], für $C_1$-$C_4$-Alkoxy [welches gegebenenfalls durch Fluor, Chlor, Brom, Cyano, $C_1$-$C_4$-Alkoxy-carbonyl, $C_1$-$C_4$--Alkoxy, $C_1$-$C_4$-Alkylthio, $C_1$-$C_4$-Alkylsulfinyl

oder $C_1$-$C_4$-Alkylsulfonyl substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], für $C_1$-$C_4$-Alkyl-amino-sulfonyl, Di-($C_1$-$C_4$-alkyl)-amino-sulfonyl, $C_1$-$C_4$-Alkoxyamino-sulfonyl, Benzyloxy-aminosulfonyl, $C_1$-$C_4$-Alkoxy-$C_1$-$C_4$-alkyl-aminosulfonyl, für Phenyl oder Phenoxy, für $C_1$-$C_4$-Alkoxysulfonyl, für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano oder $C_1$-$C_4$-Alkoxy-carbonyl substituiert ist], für $C_3$-$C_6$-Alkenoxy [welches gegebenenfalls durch Fluor, Chlor, Brom, Cyano oder $C_1$-$C_4$-Alkoxy-carbonyl substituiert ist], für $C_3$-$C_6$-Alkenylthio [welches gegebenenfalls durch Fluor, Chlor, Brom, Cyano oder $C_1$-$C_4$-Alkoxy-carbonyl substituiert ist], für $C_1$-$C_6$-Alkoxy-carbonyl [welches gegebenenfalls durch Fluor, Chlor, Brom, Cyano oder $C_1$-$C_4$-Alkoxy substituiert ist], für $C_3$-$C_6$-Cycloalkoxy-carbonyl, für $C_1$-$C_6$-Alkylthiocarbonyl [welches gegebenenfalls durch Fluor, Chlor, Brom, Cyano oder

$C_1$-$C_4$-Alkoxy-carbonyl substituiert ist], für Aminocarbonyl, $C_1$-$C_4$-Alkylamino-carbonyl, Di-($C_1$-$C_4$-alkyl)-amino-carbonyl, $C_1$-$C_4$-Alkoxy-amino-carbonyl, $C_3$-$C_4$-Alkenyloxy-amino-carbonyl, Benzyloxyaminocarbonyl, $C_1$-$C_4$--Alkoxy-$C_1$-$C_4$-alkyl-amino-carbonyl, Dimethylhydrazinocarbonyl, Dimethylhydrazinosulfonyl, für $C_3$-$C_6$-Alkinyloxy oder $C_3$-$C_6$-Alkinylthio stehen;

in welcher weiter

$R^1$ für eine gegebenenfalls substituierten Benzylrest

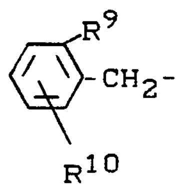

steht, worin

$R^9$ und $R^{10}$ gleich oder verschieden sind und für Wasserstoff, Halogen [wie insbesondere Fluor, Chlor oder Brom], Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkoxy--carbonyl, $C_1$-$C_4$- Alkylthio, $C_1$-$C_4$-Alkylsulfinyl,

$C_1$-$C_4$-Alkylsulfonyl oder Di-($C_1$-$C_4$-alkyl)-aminosulfonyl stehen;

in welcher weiter

$R^2$ für Wasserstoff, $C_1$-$C_4$-Alkyl oder einen Sulfonylrest $R^{11}$-$SO_2$- steht, worin

$R^{11}$ für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, Di-($C_1$-$C_4$-alkyl)-amino, $C_3$-$C_6$-Cycloalkyl-amino oder Di-($C_3$-$C_6$-cycloalkyl)-amino [welche gegebenenfalls durch Fluor, Chlor, Brom, Cyano, Nitro, Phenyl, Phenoxy,

$C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls durch Fluor, Chlor, Brom, $C_1$-$C_4$- Alkyl, Trifluormethyl, $C_1$-$C_4$-Alkoxy, $C_1$-$C_2$- Fluoralkoxy, $C_1$-$C_4$-Alkylthio, $C_1$-$C_4$--Alkylsulfinyl, $C_1$-$C_4$-Alkylsulfonyl, Cyano, Nitro und/oder $C_1$-$C_4$-Alkoxy-carbonyl substituiert sind], für Benzyl [welches gegebenenfalls durch Fluor, Chlor, Brom, Cyano, Nitro, $C_1$-$C_4$-Alkyl, Trifluormethyl, $C_1$-$C_4$--Alkoxy-carbonyl, $C_1$-$C_4$-Alkoxy oder Di-($C_1$--$C_4$-alkyl)-amino-sulfonyl substituiert ist] oder für einen gegebenenfalls substituierten Phenylrest 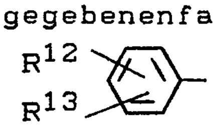 steht, worin

$R^{12}$ und $R^{13}$ gleich oder verschieden sind und die oben für $R^7$ und $R^8$ angegebenen Bedeutungen haben, jedoch nicht in jedem Einzelfall mit $R^7$ und $R^8$ identisch sind;

in welcher weiter

$R^3$ für den Rest $-O-R^{14}$ steht, worin

$R^{14}$ für $C_1$-$C_{12}$-Alkyl [welches gegebenenfalls durch Fluor, Chlor, Brom, $C_1$-$C_4$-Alkoxy, $C_1$--$C_4$-Alkylthio, $C_1$-$C_4$-Alkylsulfinyl oder $C_1$-$C_4$-Alkylsulfonyl substituiert ist], für $C_3$-$C_6$-Cycloalkyl, $C_3$-$C_6$-Cycloalkyl-$C_1$-$C_2$-alkyl, $C_3$-$C_6$-Alkenyl, [welches gegebenenfalls durch Fluor, Chlor oder Brom substituiert ist], $C_3$-$C_6$-Alkinyl, $C_1$-$C_4$-Alkoxy-carbonyl--$C_1$-$C_2$-alkyl, Aminocarbonylmethyl, $C_1$-$C_4$-Alkylamino-carbonyl-methyl, Di-($C_1$-$C_4$-alkyl)--amino-carbonyl-methyl oder für Phenyl, Benzyl, Benzhydryl oder Phenylethyl [welche gegebenenfalls durch Fluor, Chlor, Nitro, Cyano, $C_1$-$C_4$-Alkyl, $C_1$-$C_4$-Alkoxy oder $C_1$-$C_4$--Alkoxy-carbonyl substituiert sind] steht;

in welcher weiter

$R^3$ für den Rest 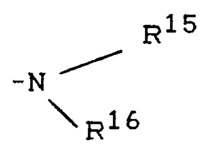 steht, worin

$R^{15}$ für Wasserstoff oder $C_1$-$C_4$-Alkyl steht und

$R^{16}$ für Wasserstoff, $C_1$-$C_4$-Alkyl [welches gegebenenfalls durch Fluor, Chlor, Brom, Cyano, $C_1$-$C_4$-Alkoxy oder $C_1$-$C_4$-Alkoxy-carbonyl substituiert ist], für $C_3$-$C_6$-Cycloalkyl, für

Phenyl, Benzyl oder Phenylethyl [welche gegebenenfalls durch Fluor, Chlor, Brom, Cyano, Nitro, $C_1$-$C_4$-Alkyl, $C_1$-$C_4$-Alkoxy oder $C_1$-$C_4$-Alkoxy-carbonyl substituiert sind], für $C_1$-$C_4$-Alkyl-carbonyl, $C_1$-$C_4$-Alkoxy-carbonyl, $C_1$-$C_4$-Alkyl-sulfonyl oder Phenylsulfonyl [welches gegebenenfalls durch Fluor, Chlor, Brom, Cyano, Nitro oder Methyl substituiert ist] steht;

in welcher weiter - für den Fall, daß $R^2$ für einen Sulfonylrest $R^{11}$-$SO_2$- steht, worin $R^{11}$ die oben angegebene Bedeutung hat - $R^3$ auch für Wasserstoff steht;

in welcher weiter

$R^4$ für Wasserstoff, Fluor, Chlor, Brom, $C_1$-$C_4$-Alkyl [welches gegebenenfalls durch Fluor und/ oder Chlor substituiert ist], für $C_1$-$C_4$-Alkoxy [welches gegebenenfalls durch Fluor und/oder Chlor substituiert ist] oder für $C_1$-$C_4$-Alkylthio [welches gegebenenfalls durch Fluor und/oder Chlor substituiert ist] steht;

$R^5$ für Wasserstoff, Fluor, Chlor, Brom, $C_1$-$C_4$-Alkyl [welches gegebenenfalls durch Fluor und/oder Chlor substituiert ist], für Cyano, Formyl, $C_1$-$C_4$-Alkylcarbonyl oder $C_1$-$C_4$-Alkoxy-carbonyl steht und

$R^6$ für Wasserstoff, für $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für Amino, $C_1$-$C_4$-Alkylamino oder Di-($C_1$-$C_4$-alkyl)-amino steht

- mit der Maßgabe, daß $R^4$ und $R^6$ nicht gleichzeitig für Methyl stehen -

sowie 1:1-Addukte von Verbindungen der Formel (I) mit starken Säuren gefunden,

wobei folgende Verbindungen ausgenommen sind:

N'-(4,6-Dimethoxy-pyrimidin-2-yl)-N''-methoxy-N'''-(2-trifluormethoxy-benzolsulfonyl)-guanidin-Kaliumsalz, N'-(4-Methoxy-6-methyl-pyrimidin-2-yl)-N''-dimethylamino-N'''-(2-methyl-benzolsulfonyl)-guanidin, N'-(4-Methoxy-6-methyl-pyrimidin-2-yl)-N''-methoxy-N'''-(2-chlor-benzolsulfonyl)-guanidin und N'-(4-Methoxy-6-methyl-pyrimidin-2-yl)-N''-dimethylamino-N'''-(2-chlor-benzolsulfonyl)-guanidin.

2. Verfahren zur Herstellung von Sulfonylguanidinopyrimidin-Derivaten der allgemeinen Formel (I) gemäß Anspruch 1, dadurch gekennzeichnet, daß man

(a) für den Fall, daß M für Wasserstoff steht

Guanidinopyrimidin-Derivate der Formel (II)

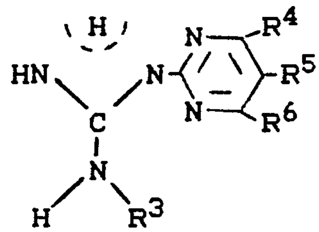

(II)

in welcher

$R^3$, $R^4$, $R^5$ und $R^6$ die oben angegebenen Bedeutungen haben,

mit Sulfonsäurechloriden der Formel (III)

$R^1-SO_2-Cl$ (III)

in welcher

$R^1$ die oben angegebene Bedeutung hat,

in Gegenwart von Säureakzeptoren und gegebenenfalls in Gegenwart von Verdünnungsmitteln umsetzt;

oder daß man

(b) für den Fall, daß M für Wasserstoff steht und $R^2$ für Wasserstoff oder $C_1$-$C_4$-Alkyl steht,

die nach dem oben unter (a) angegebenen Verfahren erhältlichen Sulfonylguanidinopyrimidin-Derivate der Formel (ID)

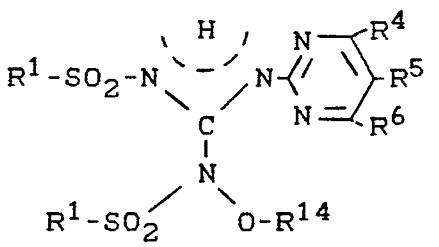

(ID)

in welcher

$R^1$, $R^4$, $R^5$, $R^6$ und $R^{14}$ die oben angegebenen Bedeutungen haben,

mit Aminoverbindungen der Formel (IV)

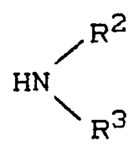

(IV)

in welcher

$R^2$ für Wasserstoff oder $C_1$-$C_4$-Alkyl steht und

$R^3$ die oben angegebenen Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder daß man

(c) für den Fall, daß M für Wasserstoff steht und

$R^2$ für einen Sulfonylrest der Formel $R^{11}-SO_2-$ steht, worin

$R^{11}$ die oben angegebene Bedeutung hat,

Sulfonylguanidinopyrimidin-Derivate der Formel (IE)

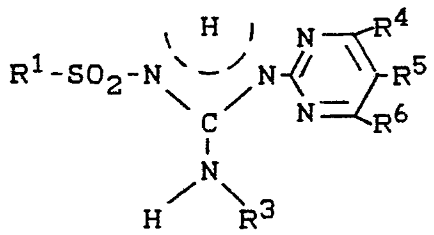

(IE)

in welcher

$R^1$, $R^3$, $R^4$, $R^5$ und $R^6$ die oben angegebenen Bedeutungen haben,

mit Sulfonsäurechloriden der Formel (V)

$$R^{11}-SO_2-Cl \quad (V)$$

in welcher

$R^{11}$ die oben angegebene Bedeutung hat,

in Gegenwart von Säureakzeptoren und gegebenenfalls in Gegenwart von Verdünnungsmitteln umsetzt;

oder daß man

(d) ebenfalls für den Fall, daß M für Wasserstoff steht und,

$R^2$ für einen Sulfonylrest der Formel $R^{11}$-$SO_2$- steht, worin

$R^{11}$ die oben angegebene Bedeutung hat,

Sulfonylguanidinopyrimidin-Derivate der Formel (VI)

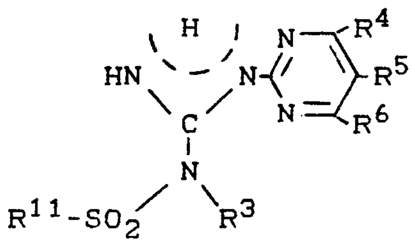

(VI)

in welcher

$R^3$, $R^4$, $R^5$, $R^6$ und $R^{11}$ die oben angegebene Bedeutungen haben,

mit Sulfonsäurechloriden der Formel (III)

$$R^1-SO_2-Cl \qquad (III)$$

in welcher

$R^1$ die oben angegebene Bedeutung hat,

in Gegenwart von Säureakzeptoren und gegebenenfalls in Gegenwart von Verdünnungsmitteln umsetzt;

oder daß man

(e) für den Fall, daß M für Wasserstoff steht und

$R^2$ für Wasserstoff oder $C_1$-$C_4$-Alkyl steht,

Sulfonylisothioureidopyrimidin-Derivate der Formel (VII)

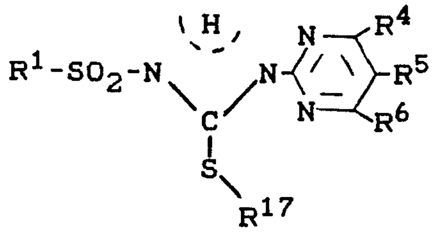 (VII)

in welcher

$R^1$, $R^4$, $R^5$ und $R^6$ die oben angegebenen Bedeutungen haben und

$R^{17}$ für $C_1$-$C_4$-Alkyl oder Benzyl steht,

mit Aminoverbindungen der Formel (IV)

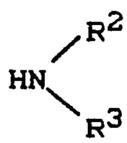 (IV)

in welcher

$R^2$ für Wasserstoff oder $C_1$-$C_4$-Alkyl steht und

$R^3$ die oben angegebene Bedeutung hat,

gegebenenfalls in Gegenwart von Verdünnungsmitteln umsetzt;

oder daß man

(f) für den Fall, daß M für ein Metalläquivalent steht, die nach den oben unter (a), (b), (c), (d) und (e) angegebenen Verfahren erhältlichen Verbindungen der Formel (I), in welcher M für Wasserstoff steht und $R^1$, $R^2$, $R^3$, $R^4$, $R^5$ und $R^6$ die oben angegebenen Bedeutungen haben,

mit Metallhydroxiden, -hydriden oder -alkanolaten oder mit metallorganischen Verbindungen, gegebenenfalls in Gegenwart von Verdünnungsmitteln umsetzt;

oder daß man

(g) für den Fall, daß 1:1-Addukte von Verbindungen der Formel (I) mit starken Säuren herzustellen sind,

Verbindungen der Formel (I), in welcher M für Wasserstoff steht und $R^1$, $R^2$, $R^3$, $R^4$, $R^5$ und $R^6$ die oben angegebenen Bedeutungen haben,

mit starken Säuren, gegebenenfalls in Gegenwart von Verdünnungsmitteln umsetzt.

3. Herbizide Mittel, gekennzeichnet durch einen Gehalt an mindestens einem Sulfonylguanidinopyrimidin--Derivat der allgemeinen Formel (I) gemäß Anspruch 1.

4. Verwendung von Sulfonylguanidinopyrimidin-Derivaten der allgemeinen Formel (I) gemäß Anspruch 1 zur Bekämpfung von unerwünschtem Pflanzenwachstum.

5. Verfahren zur Herstellung von herbiziden Mitteln, dadurch gekennzeichnet, daß man Sulfonylguanidinopyrmidin-Derivate der allgemeinen Formel (I) gemäß Anspruch 1 mit Streckmitteln und/oder oberflächenaktiven Mitteln vermischt.

6. Sulfonylguanidinopyrimidin-Derivate der allgemeinen Formel (VI)

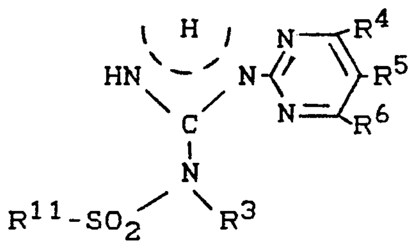

(VI)

in welcher

$R^3$ für den Rest $-O-R^{14}$ steht, worin

$R^{14}$ für $C_1$-$C_{12}$-Alkyl [welches gegebenenfalls durch Fluor, Chlor, Brom, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_6$-Cycloalkyl, $C_3$-$C_6$-Cycloalkyl-$C_1$-$C_2$-alkyl, $C_3$-$C_6$-Alkenyl [welches gegebenenfalls durch Fluor, Chlor oder Brom substituiert ist], $C_3$-$C_6$-Alkinyl, $C_1$-$C_4$-Alkoxy-carbonyl-$C_1$-$C_2$-alkyl, Aminocarbonylmethyl, $C_1$-$C_4$-Alkylamino-carbonyl-methyl, Di-($C_1$-$C_4$-alkyl)-amino-carbonyl-methyl oder für Phenyl, Benzyl, Benzhydryl oder Phenylethyl [welche gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert sind] steht;

in welcher weiter

$R^3$ für den Rest 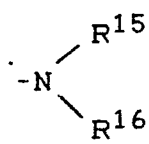 steht, worin

$R^{15}$ für Wasserstoff oder $C_1$-$C_4$-Alkyl steht und

$R^{16}$ für Wasserstoff, $C_1$-$C_4$-Alkyl [welches gegebenenfalls durch Fluor, Chlor, Brom, Cyano, $C_1$-$C_4$-Alkoxy oder $C_1$-$C_4$-Alkoxy-carbonyl substituiert ist], für $C_3$-$C_6$-Cycloalkyl, für

Phenyl, Benzyl oder Phenylethyl [welche gegebenenfalls durch Fluor, Chlor, Brom, Cyano, Nitro, $C_1$-$C_4$-Alkyl, $C_1$-$C_4$-Alkoxy oder $C_1$-$C_4$- Alkoxy-carbonyl substituiert sind], für $C_1$-$C_4$-Alkyl-carbonyl, $C_1$-$C_4$-Alkoxy-carbonyl, $C_1$-$C_4$-Alkyl-sulfonyl oder Phenylsulfonyl [welches gegebenenfalls durch Fluor, Chlor, Brom, Cyano, Nitro oder Methyl substituiert ist] steht;

$R^4$ für Wasserstoff, Fluor, Chlor, Brom, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist] oder für $C_1$-$C_4$-Alkylthio [welches gegebenenfalls durch Fluor und/oder Chlor substituiert ist] steht;

$R^5$ für Wasserstoff, Fluor, Chlor, Brom, $C_1$-$C_4$-Alkyl [welches gegebenenfalls durch Fluor und/oder Chlor substituiert ist], für Cyano, Formyl, $C_1$-$C_4$-Alkylcarbonyl oder $C_1$-$C_4$-Alkoxy-carbonyl steht,

$R^6$ für Wasserstoff, für $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für Amino, $C_1$-$C_4$-Alkylamino oder Di-($C_1$-$C_4$-alkyl)-amino steht

- mit der Maßgabe, daß $R^4$ und $R^6$ nicht gleichzeitig für Methyl stehen -

und in welcher ferner

$R^{11}$ für $C_1$-$C_{12}$-Alkyl [welches gegebenenfalls durch Fluor, Chlor, Brom, Cyano, Nitro, $C_1$-$C_4$-Alkoxy--carbonyl, $C_1$-$C_4$-Alkylamino-carbonyl, Di-($C_1$-$C_4$--alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$--Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, Di-($C_1$-$C_4$--alkyl)-amino, $C_3$-$C_6$-Cycloalkyl-amino oder Di--($C_3$-$C_6$-cycloalkyl)-amino [welche gegebenenfalls durch Fluor, Chlor, Brom, Cyano, Nitro, Phenyl, Phenoxy, $C_1$-$C_4$-Alkoxy, $C_1$-$C_4$-Alkylthio, $C_1$-$C_4$--Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls

durch Fluor, Chlor, Brom, $C_1$-$C_4$- Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/ oder $C_1$-$C_4$-Alkoxy-carbonyl substituiert sind], für Benzyl [welches gegebenenfalls durch Fluor, Chlor, Brom, Cyano, Nitro, $C_1$-$C_4$-Alkyl, Trifluormethyl, $C_1$-$C_4$-Alkoxy-carbonyl, $C_1$-$C_4$- Alkoxy oder Di-$(C_1$-$C_4$-alkyl)-amino-sulfonyl substituiert ist] oder für einen gegebenenfalls substituierten Phenylrest 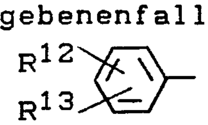

steht, worin

$R^{12}$ und $R^{13}$ gleich oder verschieden sind und für Wasserstoff, Halogen [insbesondere Fluor, Chlor, und/ oder Brom], Cyano, Nitro, $C_1$-$C_4$-Alkyl [welches gegebenenfalls durch Fluor, Chlor, Brom, Cyano, $C_1$-$C_4$-Alkoxycarbonyl, $C_1$-$C_4$-Alkylamino-carbonyl, Di-$(C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkyl-sulfonyl substituiert ist], für $C_1$-$C_4$-Alkoxy [welches gegebenenfalls durch Fluor, Chlor, Brom, Cyano, $C_1$-$C_4$-Alkoxy-carbonyl, $C_1$-$C_4$-Alkoxy, $C_1$-$C_4$-Alkylthio,

$C_1$-$C_4$-Alkylsulfinyl oder $C_1$-$C_4$-Alkylsulfonyl substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], für $C_1$-$C_4$-Alkylamino-sulfonyl, Di-($C_1$-$C_4$-alkyl)-amino-sulfonyl , $C_1$-$C_4$-Alkoxyamino-sulfonyl, Benzyloxy-aminosulfonyl, $C_1$-$C_4$-Alkoxy-$C_1$-$C_4$-alkyl-amino-sulfonyl, für Phenyl oder Phenoxy, für $C_1$-$C_4$-Alkoxysulfonyl, für $C_2$--$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano oder $C_1$-$C_4$-Alkoxy-carbonyl substituiert ist], für $C_3$-$C_6$-Alkenoxy [welches gegebenenfalls durch Fluor, Chlor, Brom, Cyano oder $C_1$-$C_4$-Alkoxy-carbonyl substituiert ist], für $C_3$-$C_6$-Alkenylthio [welches gegebenenfalls durch Fluor, Chlor, Brom, Cyano oder $C_1$-$C_4$-Alkoxy-carbonyl

substituiert ist], für $C_1$-$C_6$-Alkoxy-carbonyl [welches gegebenenfalls durch Fluor, Chlor, Brom, Cyano oder $C_1$-$C_4$-Alkoxy substituiert ist], für $C_3$-$C_6$-Cycloalkoxy-carbonyl, für $C_1$-$C_6$-Alkylthiocarbonyl [welches gegebenenfalls durch Fluor, Chlor, Brom, Cyano oder $C_1$-$C_4$-Alkoxy-carbonyl substituiert ist], für Aminocarbonyl, $C_1$-$C_4$-Alkylamino-carbonyl, Di-($C_1$-$C_4$-alkyl)-amino-carbonyl, $C_1$-$C_4$-Alkoxy-aminocarbonyl, $C_3$-$C_4$-Alkenyloxy-amino-carbonyl, Benzyloxyaminocarbonyl, $C_1$-$C_4$-Alkoxy-$C_1$-$C_4$-alkyl-amino-carbonyl, Dimethylhydrazinocarbonyl, Dimethylhydrazinosulfonyl, für $C_3$-$C_6$-Alkinyloxy oder $C_3$-$C_6$-Alkinylthio stehen.

7. Verfahren zur Herstellung von Sulfonylguanidinopyrimidin-Derivaten der allgemeinen Formel (VI) gemäß Anspruch 6, dadurch gekennzeichnet, daß man Guanidinopyrimidin-Derivate der Formel (II) gemäß Anspruch 2 mit Sulfonsäurechloriden der Formel (V) gemäß Anspruch 2 in Gegenwart von Säureakzeptoren und gegebenenfalls in Gegenwart von Verdünnungsmitteln umsetzt.

8. Sulfonylisothioureidopyrimidin-Derivate der allgemeinen Formel (VII A)

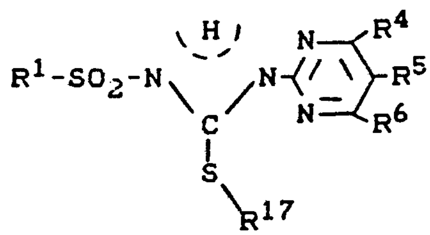

(VII A)

in welcher

$R^1$ für einen substituierten Phenylrest 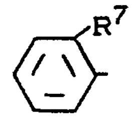 steht, worin

$R^7$ für $C_1$-$C_4$-Alkylthio, $C_1$-$C_4$-Alkylsulfinyl, $C_1$-$C_4$-Alkylsulfonyl oder $C_1$-$C_4$-Fluoralkoxy steht,

$R^4$ für Wasserstoff, Fluor, Chlor, Brom, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist] oder für $C_1$-$C_4$-Alkylthio [welches gegebenenfalls durch Fluor und/oder Chlor substituiert ist] steht;

$R^5$ für Wasserstoff, Fluor, Chlor, Brom, $C_1$-$C_4$-Alkyl [welches gegebenenfalls durch Fluor und/oder Chlor substituiert ist], für Cyano, Formyl, $C_1$-$C_4$-Alkylcarbonyl oder $C_1$-$C_4$-Alkoxy-carbonyl

steht,

$R^6$ für Wasserstoff, für $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für Amino, $C_1$-$C_4$-Alkylamino oder Di-($C_1$-$C_4$-alkyl)-amino steht

- mit der Maßgabe, daß $R^4$ und $R^6$ nicht gleichzeitig für Methyl stehen - und

$R^{17}$ für $C_1$-$C_4$-Alkyl oder Benzyl steht.

9. Verfahren zur Herstellung von Sulfonylisothioureidopyrimidin-Derivaten der Formel (VII A) gemäß Anspruch 8, dadurch gekennzeichnet, daß man N-sulfonyl-imino-dithiokohlensaäureester der Formel (XV A)

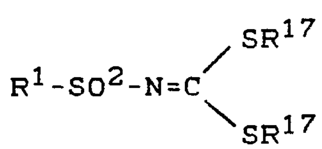

(XV A)

in welcher

$R^1$ und $R^{17}$ die unter Anspruch 8 angegebene Bedeutung haben,

mit Aminopyrimidinen der Formel (XI)

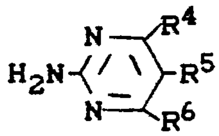

(XI)

in welcher

$R^4$, $R^5$ und $R^6$ die unter Anspruch 8 angegebenen Bedeutungen haben,

gegebenenfalls in Gegenwart von Basen und gegebenenfalls in Gegenwart von Verdünnungsmitteln umsetzt, nach Reaktionsende mit Wasser verdünnt und mit einer starken Säure ansäuert.

10. Guanidinopyrimidin-Derivate der Formel (II)

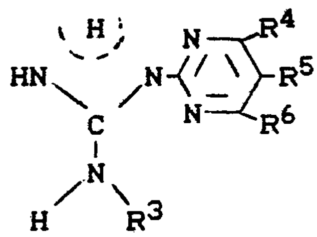

(II)

in welcher

$R^3$ für den Rest $-O-R^{14}$ steht, worin

$R^{14}$ für $C_1$-$C_{12}$-Alkyl [welches gegebenenfalls durch Fluor, Chlor, Brom, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_6$-Cycloalkyl, $C_3$-$C_6$-Cycloalkyl-$C_1$-$C_2$-alkyl, $C_3$-$C_6$-Alkenyl, [welches gegebenenfalls durch Fluor, Chlor oder Brom substituiert ist], $C_3$-$C_6$-Alkinyl, $C_1$-$C_4$-Alkoxy-carbonyl-$C_1$-$C_2$-alkyl, Aminocarbonylmethyl, $C_1$-$C_4$-Alkylamino-carbonyl-methyl, Di-($C_1$-$C_4$-alkyl)-amino-carbonyl-methyl oder für Phenyl, Benzyl, Benzhydryl oder Phenylethyl [welche gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert sind] steht;

in welcher weiter

$R^3$ für den Rest 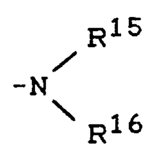 steht, worin

$R^{15}$ für Wasserstoff oder $C_1$-$C_4$-Alkyl steht und

$R^{16}$ für Wasserstoff, $C_1$-$C_4$-Alkyl [welches gegebenenfalls durch Fluor, Chlor, Brom, Cyano, $C_1$-$C_4$-Alkoxy oder $C_1$-$C_4$-Alkoxy-carbonyl substituiert ist], für $C_3$-$C_6$-Cycloalkyl, für Phenyl, Benzyl oder Phenylethyl

[welche gegebenenfalls durch Fluor, Chlor, Brom, Cyano, Nitro, $C_1$-$C_4$-Alkyl, $C_1$-$C_4$-Alkoxy oder $C_1$-$C_4$- Alkoxy-carbonyl substituiert sind], für $C_1$-$C_4$-Alkyl-carbonyl, $C_1$-$C_4$-Alkoxy-carbonyl, $C_1$-$C_4$-Alkyl-sulfonyl oder Phenylsulfonyl [welches gegebenenfalls durch Fluor, Chlor, Brom, Cyano, Nitro oder Methyl substituiert ist] steht;

in welcher weiter

(a) $R^4$ für Chlor steht,

$R^5$ für Wasserstoff steht und

$R^6$ für $C_1$-$C_4$-Alkoxy [welches gegebenenfalls durch Fluor und/oder Chlor substituiert ist], für $C_1$-$C_4$-Alkylamino oder Di-($C_1$-$C_4$-alkyl)-amino steht; oder in welcher weiter

(b) $R^4$ für $C_1$-$C_4$-Alkyl [welches gegebenenfalls durch Fluor und/oder Chlor substituiert ist] steht,

$R^5$ für Wasserstoff steht und

$R^6$ für durch Fluor und/oder Chlor substituiertes $C_1$-$C_4$-Alkoxy, für $C_1$-$C_4$-Alkylamino oder Di-($C_1$-$C_4$-alkyl)-amino steht; oder in welcher weiter

(c) $R^4$ und $R^5$ für Wasserstoff stehen und

$R^6$ für $C_2$-$C_3$-Alkyl steht.

11. Verfahren zur Herstellung von Guanidinopyrimidin-Derivaten der Formel (II) gemäß Anspruch 10, dadurch gekennzeichnet, daß man Cyanaminopyrimidin-Derivate der Formel (VIII)

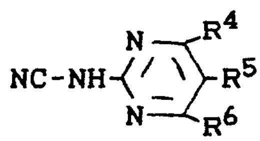

(VIII)

in welcher

$R^4$, $R^5$ und $R^6$ die oben angegebenen Bedeutungen haben,

mit Aminoverbindungen der Formel (IX)

$H_2N-R^3$ (IX)

in welcher

$R^3$ die oben angegebene Bedeutung hat,

bzw. mit deren Hydrochloriden, gegebenenfalls in Gegenwart von Verdünnungsmitteln umsetzt und gegebenenfalls die Umsetzungsprodukte mit Säureakzeptoren behandelt.

12. Cyanaminopyrimidin-Derivate der Formel (VIII)

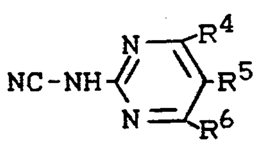 (VIII)

in welcher

(a) $R^4$ für Chlor steht,

$R^5$ für Wasserstoff steht und

$R^6$ für $C_1$-$C_4$-Alkoxy [welches gegebenenfalls durch Fluor und/oder Chlor substituiert ist], für $C_1$-$C_4$-Alkylamino oder Di-($C_1$-$C_4$-alkyl)-amino steht; oder in welcher

(b) $R^4$ für $C_1$-$C_4$-Alkyl [welches gegebenenfalls durch Fluor und/oder Chlor substituiert ist] steht,

$R^5$ für Wasserstoff steht und

$R^6$ für durch Fluor und/oder Chlor substituiertes $C_1$-$C_4$-Alkoxy, für $C_1$-$C_4$-Alkylamino oder Di-($C_1$-$C_4$-alkyl)-amino steht; oder in welcher

(c) $R^4$ und $R^5$ für Wasserstoff stehen und

$R^6$ für $C_2$-$C_3$-Alkyl steht;

13. Verfahren zur Herstellung von Cyanaminopyrimidin-Derivaten der Formel (VIII) gemäß Anspruch 12, dadurch gekennzeichnet, daß man

(a) Alkalimetall- oder Erdalkalimetall-Salze von Cyanamid mit Chlorpyrimidinen der Formel (X)

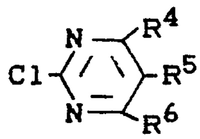

(X)

in welcher

$R^4$, $R^5$, $R^6$ die oben angegebenen Bedeutungen haben,

gegebenenfalls in Gegenwart von inerten Verdünungsmitteln umsetzt, oder

(b) Cyanoguanidin mit β-Dicarbonylverbindungen oder deren Derivaten umsetzt, oder

(c) Aminopyrimidine der Formel (XI)

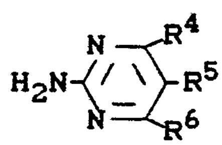

(XI)

in welcher

$R^4$, $R^5$ und $R^6$ die oben angegebenen Bedeutungen haben,

mit Carbonylisothiocyanaten der Formel (XII)

$$R^{18}-\overset{O}{\overset{\parallel}{C}}-N=C=S \qquad (XII)$$

in welcher

$R^{18}$ für Ethoxy oder Phenyl steht,

gegebenenfalls in Gegenwart von Verdünnungsmittels umsetzt und die hierbei gebildeten Carbonylthioharnstoffe der Formel (XIII)

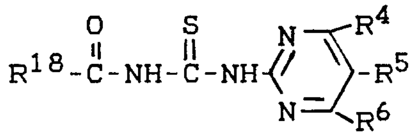 (XIII)

in welcher

$R^4$, $R^5$, $R^6$ und $R^{18}$ die oben angegebenen Bedeutungen haben,

gegebenenfalls nach Einengen isoliert und mit wässrigen Alkalimetall- oder Erdalkalimetallhydroxidlösungen gegebenenfalls in Gegenwart eines organischen Lösungsmittels umsetzt und die nach Ansäuern kristallin erhaltenen Thioharnstoffe der Formel (XIV)

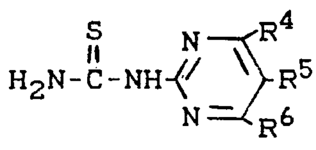 (XIV)

in welcher

$R^4$, $R^5$ und $R^6$ die oben angegebenen Bedeutungen haben,

durch Absaugen isoliert und mit Metallverbindungen, welche Schwefelwasserstoff binden können, in Gegenwart von wässrigen Alkalimetall- oder Erdalkalimetall-hydroxidlösungen umsetzt, nach Ende der Umsetzung filtriert und das Filtrat ansäuert.

Europäisches Patentamt

**EUROPÄISCHER RECHERCHENBERICHT**

**0 173 321**

EP 85110834.0

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| P,D,A | EP - A1 - 0 121 082 (BAYER)<br>* Formel I, Ansprüche 1-7; Seiten 37,38; Seite 47, Zeilen 1-23 *<br>-- | 1-13 | C 07 D 239/42<br>C 07 D 239/46<br>A 01 N 47/44 |
| A | EP - A1 - 0 117 014 (DU PONT)<br>* Zusammenfassung *<br>-- | 1-5,8 | |
| A | DE - A1 - 3 243 533 (SANDOZ)<br>* Ansprüche 1,7,8 *<br>-- | 1,3,4 | |
| A | DD - A - 71 015 (KOCHMANN)<br>* Anspruch *<br>---- | 3 | RECHERCHIERTE SACHGEBIETE (Int. Cl.4)<br>C 07 D 239/00 |

Der vorliegende Recherchenbericht wurde für alle Patentansprüche erstellt.

| Recherchenort | Abschlußdatum der Recherche | Prüfer |
|---|---|---|
| WIEN | 18-10-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82